(19) **Europäisches Patentamt / European Patent Office / Office européen des brevets**

(11) **EP 1 144 011 B1**

(12) # EUROPEAN PATENT SPECIFICATION

(45) Date of publication and mention of the grant of the patent:
**10.03.2010 Bulletin 2010/10**

(51) Int Cl.:
*A61K 47/48* (2006.01)   *A61P 35/00* (2006.01)

(21) Application number: **99967462.5**

(22) Date of filing: **10.12.1999**

(86) International application number:
**PCT/US1999/030393**

(87) International publication number:
**WO 2000/033888 (15.06.2000 Gazette 2000/24)**

(54) **PRODRUG COMPOUNDS AND PROCESS FOR PREPARATION THEREOF**

PRODRUGS UND VERFAHREN ZU DEREN HERSTELLUNG

COMPOSES DE PROMEDICAMENTS ET PROCEDE DE PREPARATION

(84) Designated Contracting States:
**AT BE CH CY DE DK ES FI FR GB GR IE IT LI LU MC NL PT SE**
Designated Extension States:
**AL LT LV MK RO SI**

(30) Priority: **11.12.1998 US 111793 P**
**08.02.1999 US 119312 P**

(43) Date of publication of application:
**17.10.2001 Bulletin 2001/42**

(73) Proprietor: **Coulter Pharmaceutical, Inc.**
**South San Francisco, CA 94080-7014 (US)**

(72) Inventors:
• **LOBL, Thomas, J.**
  **Foster City, CA 94404 (US)**
• **DUBOIS, Vincent**
  **Fleurus (BE)**
• **FERNANDEZ, Anne-Marie**
  **B-1050 Brussels (BE)**
• **GANGWAR, Sanjeev**
  **Alameda, CA 94501 (US)**
• **LEWIS, Evan**
  **Daly City, CA 94015 (US)**
• **NIEDER, Matthew, H.**
  **Burlingame, CA 94010 (US)**
• **TROUET, André**
  **B-3009 Herent (BE)**
• **VISKI, Peter**
  **Belmont, CA 94002 (US)**
• **YARRANTON, Geoffrey, T.**
  **Burlingame, CA 94010 (US)**

(74) Representative: **Gowshall, Jonathan Vallance**
**Forrester & Boehmert**
**Pettenkoferstrasse 20-22**
**80336 München (DE)**

(56) References cited:
EP-A- 0 037 388    EP-A- 0 475 230
EP-A- 0 640 622    WO-A-96/05863
WO-A-98/10651    WO-A-98/18493
WO-A-98/52966    WO-A-99/02175
WO-A-99/28345

• MASQUELIER, M. ET AL: "Incorporation and binding of anthracycline derivatives to low density lipoprotein: in vitro and in vivo studies on drug-LDL conjugates" RECENT ADV. CHEMOTHER., PROC. INT. CONGR. CHEMOTHER., 14TH, VOLUME ANTICANCER SECT. 1, PAGES 311-12. EDITOR(S): ISHIGAMI, JOJI. PUBLISHER: UNIV. TOKYO PRESS, TOKYO, JAPAN., XP000914544
• KING, H. DALTON ET AL: "Synthesis and proteolytic cleavage of 3'-N- peptidyl -adriamycin prodrugs" PEPT.: CHEM., STRUCT. BIOL., PROC. AM. PEPT. SYMP., 11TH, MEETING DATE 1989, PAGES 137-9. EDITOR(S): RIVIER, JEAN E.; MARSHALL, GARLAND R. PUBLISHER: ESCOM SCI. PUB., LEIDEN, NETH., XP000914543

- TROUET A ET AL: "A COVALENT LINKAGE BETWEEN DAUNORUBICIN AND PROTEINS THAT IS STABLE IN SERUM AND REVERSIBLE BY LYSOSOMAL HYDROLASES AS REQUIRED FOR A LYSOSOMOTROPIC DRUG CARRIER CONJUGATE IN-VITRO AND IN-VIVO STUDIES" PROCEEDINGS OF THE NATIONAL ACADEMY OF SCIENCES OF THE UNITED STATES OF AMERICA 1982, vol. 79, no. 2, 1982, pages 626-629, XP002029566 ISSN: 0027-8424
- UMEMOTO N ET AL: "PREPARATION AND IN-VITRO CYTOTOXICITY OF A METHOTREXATE-ANTI-MM46 MONOCLONAL ANTIBODY CONJUGATE VIA AN OLIGOPEPTIDE SPACER" INTERNATIONAL JOURNAL OF CANCER 1989, vol. 43, no. 4, 1989, pages 677-684, XP000120606 ISSN: 0020-7136
- DE MARRE ANNE ET AL: "Evaluation of the hydrolytic and enzymatic stability of macromolecular Mitomycin C derivatives." JOURNAL OF CONTROLLED RELEASE 1994, vol. 31, no. 1, 1994, pages 89-97, XP000456583 ISSN: 0168-3659
- DATABASE CHEMABS [Online] CHEMICAL ABSTRACTS SERVICE, COLUMBUS, OHIO, US MASQUELIER, M. ET AL: "Antitumor activity of daunorubicin linked to proteins: biological and antitumor properties of peptidic derivatives of daunorubicin used as intermediates" retrieved from STN Database accession no. 97:150635 HCA XP002139800 & CURR. CHEMOTHER. IMMUNOTHER., PROC. INT. CONGR. CHEMOTHER., 12TH, VOL. MEETING DATE 1981, VOLUME 2, 1428-30. EDITOR(S): PERITI, PIERO; GIALDRONI GRASSI, GIULIANA. PUBLISHER: AM. SOC. MICROBIOL., WASHINGTON, D. C.,
- MASQUELIER, MICHELE ET AL: "Amino acid and dipeptide derivatives of daunorubicin. 1. Synthesis, physicochemical properties, and lysosomal digestion" J. MED. CHEM., 1980, VOL. 23, NO. 11, PAGE(S) 1166-70, XP000914522
- WALDMANN H ET AL: "ENZYMATIC PROTECTING GROUP TECHNIQUES" CHEMICAL REVIEWS, US, AMERICAN CHEMICAL SOCIETY. EASTON, vol. 94, no. 4, 1 June 1994 (1994-06-01), pages 911-937, XP000450393 ISSN: 0009-2665
- ABOUD-PIRAK E ET AL: "CYTOTOXIC ACTIVITY OF DAUNORUBICIN OR VINDESINE CONJUGATED TO A MONOCLONAL ANTIBODY ON CULTURED MCF-7 BREAST CARCINOMA CELLS" BIOCHEMICAL PHARMACOLOGY 1989, vol. 38, no. 4, 1989, pages 641-648, XP000914578 ISSN: 0006-2952
- FLACK S S ET AL: "Synthesis and Binding Properties of a Peptide Receptor" TETRAHEDRON LETTERS, vol. 36, no. 19, 8 May 1995 (1995-05-08), pages 3409-3412, XP000498971 ISSN: 0040-4039

Remarks:
    WIPO A3 publication data is not currently available.

**Description**

INTRODUCTION

Technical Field

The present invention is directed to new compounds, and methods for making them. The compounds generally work as prodrugs and in most cases are modified versions of existing compounds, especially cytotoxic agents. These prodrugs have higher specificity for the intended targets and reduced specificity to unintended targets.

Background

Many therapeutic agents, such as anthracyclines and vinca alkaloids, are especially effective for the treatment of cancers. However these molecules are often characterized *in vivo* by an acute toxicity, especially a bone marrow and mucosal toxicity, as well as a chronic cardiac toxicity in the case of the anthracyclines and chronic neurological toxicity in the case of the vinca alkaloids. Similarly, methotrexate may be used for the treatment of inflammatory reactions, such as rheumatic diseases, but its high toxicity limits its applications. Development of more specific antitumor agents is desirable for greater effectiveness against tumor cells and a decrease in the number and severity of the side effects of these products (toxicity, destruction of non-tumor cells, etc.). Development of more specific anti-inflammatory agents is also desirable.

In order to minimize toxicity problems, therapeutic agents are advantageously presented to patients in the form of prodrugs. Prodrugs are molecules capable of being converted to drugs (active therapeutic compounds) in vivo by certain chemical or enzymatic modifications of their structure. For purposes of reducing toxicity, this conversion should be confined to the site of action or target tissue rather than the circulatory system or non-target tissue. Prodrugs are often characterized by a low stability in blood and serum, however, since blood and serum contain enzymes which degrade the prodrugs.

A desirable class of prodrugs that overcomes such problems have been disclosed in Patent Cooperation Treaty International Publication No. WO 96/05863 and in U.S. Patent No. 5,962,216. Further useful prodrug compounds and methods of making such prodrugs are desirable, however, as are methods of making the prodrugs.

A particular object of the invention is a prodrug that displays a high specificity of action, a reduced toxicity, and an improved stability in blood relative to produgs of similar structure (especially the closest structure) that have existed in the public domain.

SUMMARY OF THE INVENTION.

The compound of the invention as defined in the claims is a prodrug form of a therapeutic agent linked directly or indirectly to an oligopeptide, which in turn, is linked to a stabilizing group

More generally, the present invention may be described as new prodrug compounds of a therapeutic agent, especially prodrugs comprising an antitumor therapeutic agent, displaying improved therapeutic properties relative to the products of the prior art, especially improved therapeutic properties in the treatment of cancerous tumors and/or in the treatment of inflammatory reactions such as rheumatic diseases. Improved therapeutic properties include decreased toxicity and increased efficacy. Particularly desired are prodrugs which display a high specificity of action, a reduced toxicity, an improved stability in the serum and blood, and which do not move into target cells until activated by a target cell associated enzyme. Prodrug compounds of a marker enabling tumors to be characterized (diagnosis, progression of the tumor, assay of the factors secreted by tumor cells, etc.) are also contemplated.

The present invention also relates to the pharmaceutical composition comprising the compound according to the invention and optionally a pharmaceutically acceptable adjuvant or vehicle.

Further, a method of decreasing toxicity by modifying a therapeutic agent to create a prodrug is disclosed.

Several processes for creating a prodrug of the invention are described.

BRIEF DESCRIPTION OF THE DRAWINGS

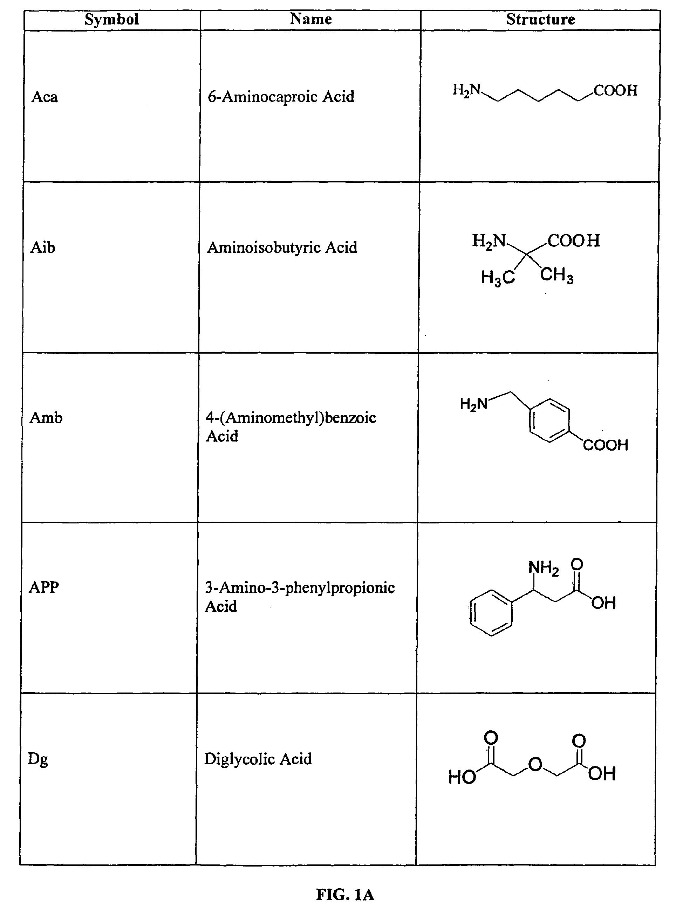

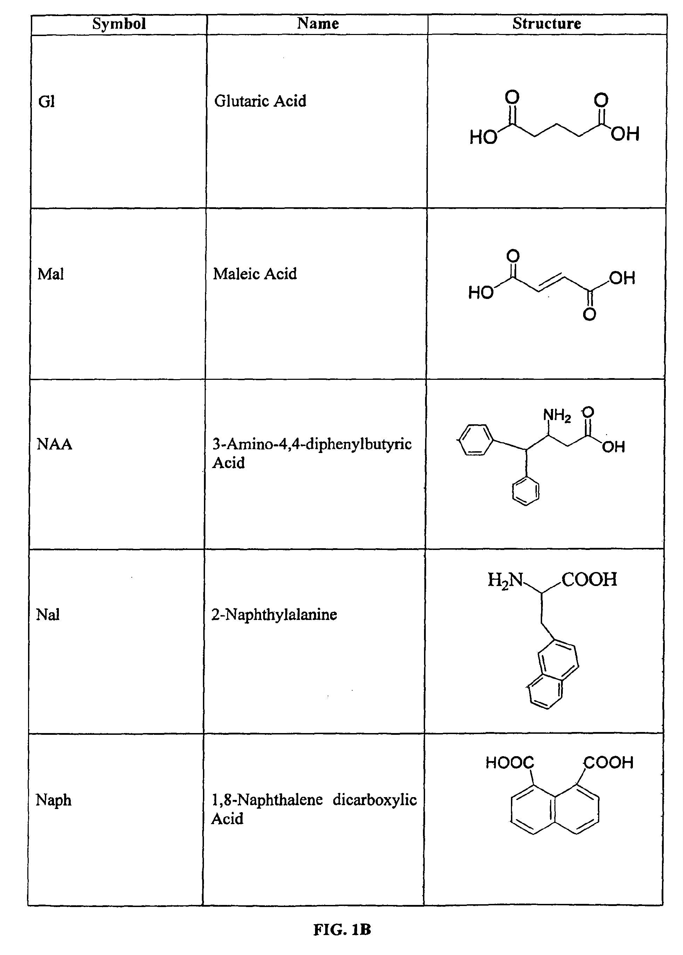

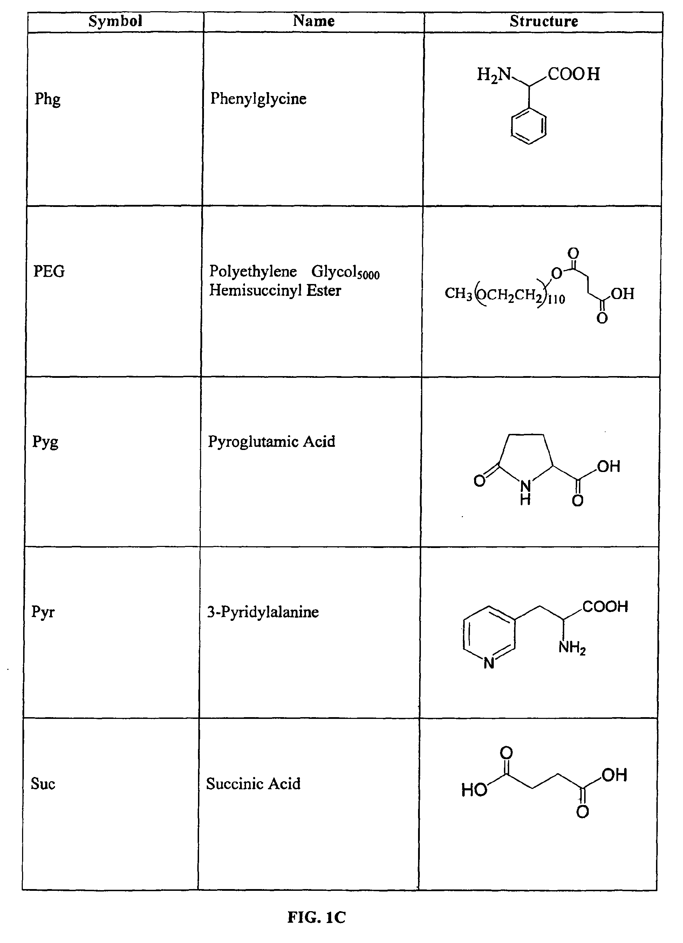

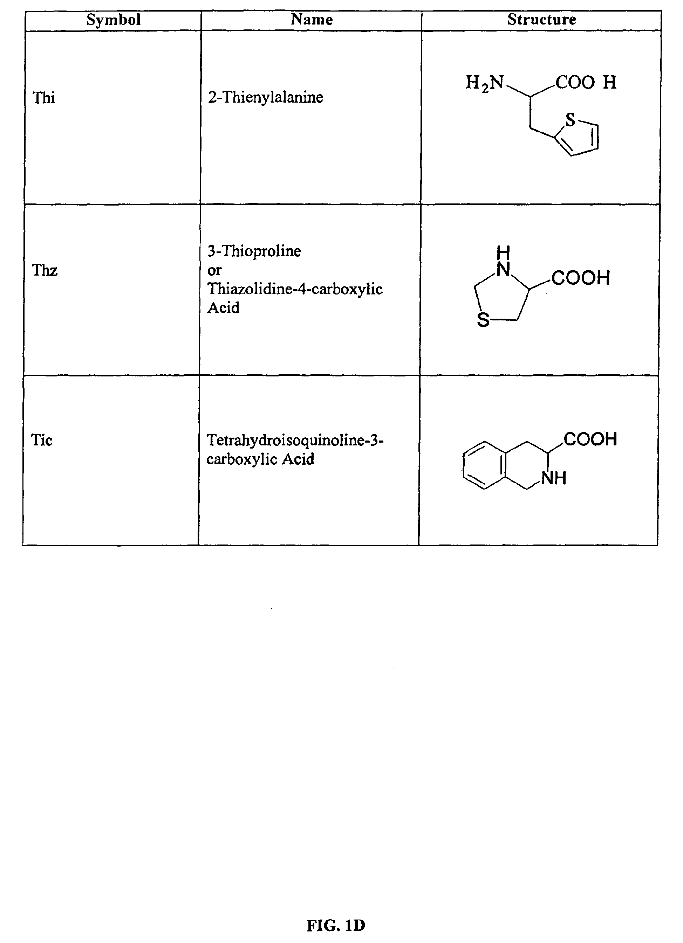

**Figs. 1A-1D** are a table of abbreviations, names, and structures.
**Fig. 2** is an exemplary scheme of cleavage of a prodrug of the invention in the extracellular vicinity of the target cell.
**Fig. 3** illustrates a synthesis of Fmoc-βAla-Leu-Ala-Leu, a typical intermediate of the invention.
**Fig. 4** illustrates an "Fmoc-route" synthesis of Methyl-succinyl-βAla-Leu-Ala-Leu, a typical intermediate of the invention.

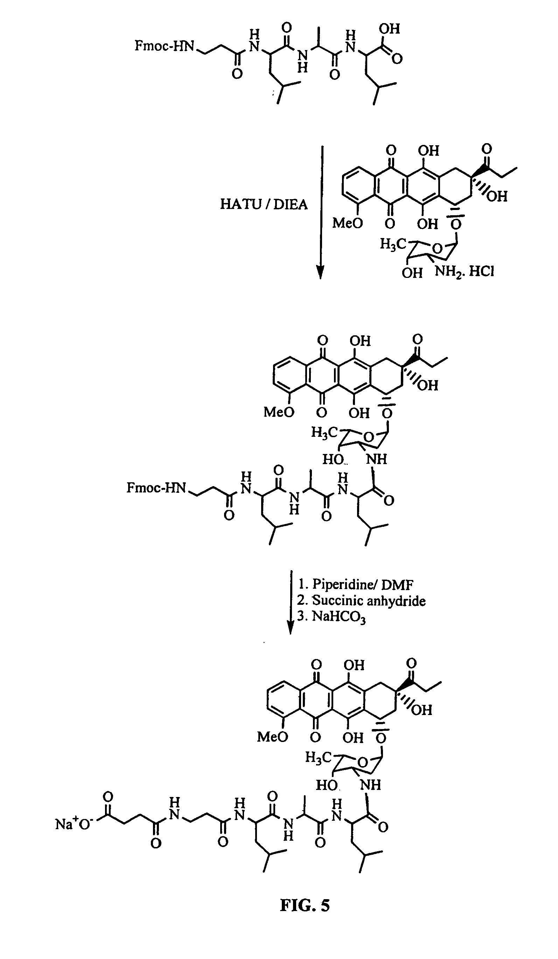

**Fig. 5** illustrates an "Fmoc route" synthesis of the salt form of Suc-βAla-Leu-Ala-Leu -DOX, a typical intermediate of the invention.

**Fig. 6** illustrates an "Succinyl Ester route" synthesis of the salt form of Suc-βAla-Leu-Ala-Leu -DOX, a typical compound of the invention.

**Fig. 7** illustrates a synthesis of an amino-protected βAla-Leu-Ala-Leu-DOX, a typical intermediate of the invention.

**Fig. 8** illustrates an "Allyl ester route" synthesis of the salt form of Suc-βAla-Leu-Ala-Leu-DOX, a typical intermediate of the invention.

**Fig. 9** illustrates a "Resin route" synthesis of Suc-βAla-Leu-Ala-Leu-DOX, a typical intermediate of the invention.

**Figs. 10A-10C** are a table of oligopeptides useful in the prodrug of the invention.

**Fig. 11** is a graph of survival in a mouse xenograft model for animals given vehicle with or without drug.

**Fig. 12** is a graph of survival in a mouse xenograft model comparing a doxorubicin prodrug and doxorubicin.

## DETAILED DESCRIPTION

### Abbreviations

Aca = 6-Aminocaproic acid
ACN = Acetonitrile
Aib = Aminoisobutyric acid
All = Allyl
Aloc = Allyloxycarbonyl
Amb = 4-(Aminomethyl)benzoic acid
APP = 3-Amino-3-phenylpropionic acid
DCC = N,N'-Dicyclohexylcarbodiimide
Boc = t-butyloxycarbonyl
Cap = caproic acid
DBN = 1,5 Diazabicyclo [4.3.0] non-5-ene
DBO = 1,4 Diazabicyclo [2.2.2] octane
DBU = 1,8-Diazabicyclo [5.4.0] undec-7-ene
DCM = Dichloromethane
DIC = N,N' - Diisopropylcarbodiimide
DIEA = Diisopropylethylamine
Dg = Diglycolic Acid
DMF = Dimethylformamide
Dnr = Daunorubicin
Dox = Doxorubicin
$Et_2O$ = diethyl ether
Fmoc = 9-Fluorenylmethyloxycarbonyl
Gl = Glutaric Acid
HATU = O-(7-Azabenzotrazol-1-yl)-1,1,3,3-tetramethyluronium-hexafluorophosphate
HBTU = 2-(1H-Benzotriazole-1-yl)1,1,3,3-tetramethyluronium-hexafluorophosphate HEPES - Hydroxethylpiperidine
HOBt = N-Hydroxybenzotriazole
HPLC = High pressure liquid chromatography
MeOH = Methanol
NAA = 3-Amino-4,4-diphenylbutyric Acid
Nal = 2-Naphthylalanine
Naph=1,8 - Napthalene dicarboxylic acid
Nle = Norleucine
NMP = N-methylpyrrolidine
Nva = Norvaline
PAM resin = 4-hydroxymethylphenylacetamidomethyl
Phg = Phenylglycine
Pyg = Pyroglutamic acid
Pyr = 3-Pyridylalanine
RT, rt = Room temperature

Suc = Succinic Acid
TCE = trichloroethyl
TFA = Trifluroacetic acid
THF = Tetrahydrofuran
Thi = 2-Thienylalanine
Thz = Thiazolidine-4-carboxylic acid
Tic = Tetrahydroisoquinoline-3-carboxylic acid

The compound of the invention as defined in the claims is a prodrug form of a therapeutic agent linked directly or indirectly to an oligopeptide, which in turn, is linked to a stabilizing group.

More generally, the present invention may be described as new prodrug compounds of a therapeutic agent, especially prodrugs comprising an antitumor therapeutic agent, displaying improved therapeutic properties relative to the products of the prior art, especially improved therapeutic properties in the treatment of cancerous tumors and/or in the treatment of inflammatory reactions such as rheumatic diseases. Improved therapeutic properties include decreased toxicity and increased efficacy. Particularly desired are prodrugs which display a high specificity of action, a reduced toxicity, an improved stability in the serum and blood, and which do not move into target cells until activated by a target cell associated enzyme. Prodrug compounds of a marker enabling tumors to be characterized (diagnosis, progression of the tumor, assay of the factors secreted by tumor cells, etc.) are also contemplated.

The present invention also relates to the pharmaceutical composition comprising the compound according to the invention and optionally a pharmaceutically acceptable adjuvant or vehicle.

Further, a method of decreasing toxicity by modifying a therapeutic agent to create a prodrug is disclosed.

Several processes for creating a prodrug of the invention are described.

Prodrug

The prodrug of the claimed invention is a modified form of a therapeutic agent and comprises several portions, including:

(1) a therapeutic agent,
(2) an oligopeptide, and
(3) a stabilizing group, and
(4) optionally, a tinker group.

Each of the portions of the prodrug are discussed in greater detail below. The typical orientation of these portions of the prodrug is as follows:

(stabilizing group)-(oligopeptide)-(optional linker group)-(therapeutic agent).

The stabilizing group is directly linked to the oligopeptide at a first attachment site of the oligopeptide. The oligopeptide is directly or indirectly linked to the therapeutic agent at a second attachment site of the oligopeptide. If the oligopeptide and the therapeutic agent are indirectly linked, then a linker group is present.

Direct linkage of two portions of the prodrug means covalent binding between the two portions. The stabilizing group and the oligopeptide are therefore directly linked via covalent binding at the first attachment site of the oligopeptide, which is the N-terminus of the oligopeptide. When the oligopeptide and the therapeutic agents are directly linked then they are covalently bound to one another at the second attachment site of the oligopeptide. The second attachment site of the oligopeptide is typically the C-terminuss of the oligopeptide, but may be elsewhere on the oligopeptide.

Indirect linkage of two portions of the prodrug means each of the two portions is covalently bound to a linker group. In an alternative embodiment, the prodrug has indirect linkage of the oligopeptide to the therapeutic agent Thus, typically, the oligopeptide is covalently bound to the linker group which, in turn, is covalently bound to the therapeutic agent.

The prodrug of the invention is cleavable within the oligopeptide directly or indirectly linked to the therapeutic agent. In order for the prodrug to be effective, the oliglopeptide linked to the therapeutic agent is either the active portion of the prodrug itself or is readily convertible to the active portion of the prodrug usually by one or more exopeptidases. The active portion of the prodrug is that part of the prodrug which upon release from the remaining portion of the prodrug compound enter the target cell and exert the therapeutic effect directly or often further conversion within the target cell.

The structures of the stablilizing group and oligopeptide are further selected to limit clearance of the oligopeptide by enzymes other than those which may be present in blood or non-target tissue. The stabilizing group blocks degradation of the prodrug and may act in providing preferable charge or other physical prodrug characteristics of the prodrug by exopeptidoses. The amino acid sequence of the olgopeptide is designed to further ensure specificity for trouase.

It is desirable to make a therapeutic agent, especially an antitumor and/or anti-inflammatory therapeutic agent, inactive by modification of the therapeutic agent to a prodrug form. According to the invention, the target cells are usually tumor cells or cells participating in anti-inflammatory reactions, especially those associated with rheumatic diseases, such as macrophages and monocytes. Modification of the therapeutic agent to a prodrug form also tends to reduce some of the side effects of the therapeutic agents.

In the target cell, the therapeutic agent (optionally attached to one or two amino acids and possibly also linker group) acts either directly on its specific intracellular action site or, after a modification under the action of intracellular proteases, kills the target cell or blocks its proliferation. Since normal cells liberate little to no trouase *in vivo*, the compound according to the invention is maintained inactive and does not enter the normal cells or does so in a relatively minor amount.

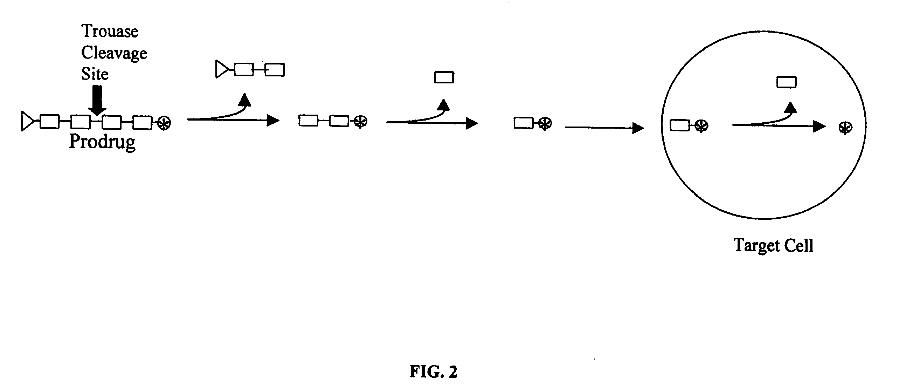

The prodrug is administered to the patient, carried through the blood stream in a stable form, and when in the vicinity of a target cell, is acted upon by trouase. Since the enzyme is only minimally present within the extracellular vicinity of normal cells, the prodrug is maintained and its active portion (including the therapeutic agent) and gains entry into the normal cells only minimally, at best. In the vicinity of tumor or other target cells, however, the presence of the relevant enzyme in the local environment causes cleavage of the prodrug. The example shown in the Fig. 2 depicts N-capped an tetrapeptide prodrug being cleaved from the remainder of the prodrug extracellularly and gaining entry into the target cell. Once within the target cell, it may be further modified to provide therapeutic effect. While the active portion of the prodrug may also enter the normal cells to some extent, the active portion is freed from the remainder of the prodrug primarily in the vicinity of target cells. Thus, toxicity to normal cells is minimized.

Release of the active portion of the prodrug including the therapeutic agent preferably occurs in the immediate environment of the target cell. In the target cell, the therapeutic agent acts either directly on its specific intracellular action site or, after a modification under the action of intracellular proteases or other enzymes, it may be modified to another form which kills the target cell or blocks its proliferation. A schematic diagram of this action for an exemplary prodrug of the invention is shown in Fig. 2.

This process is particularly useful for, and is designed for, target cell destruction when the target tissue excretes an enzyme or other factor that is not secreted by normal cells. Here "normal cells" means non-target cells that would be encountered by the prodrug upon administration of the prodrug in the manner appropriate for its intended use. Since normal (i.e., non-target) cells liberate little or none of the target-cell enzyme(s) that are responsible for cleaving the bond that links the active portion (including the therapeutic agent) of the prodrug from the remainder of the prodrug *in vivo*, the compound of the invention is maintained inactive and does not enter the normal cells.

In an alternative embodiment, the orientation of the prodrug may be reversed so that a C terminus block is attached to the oligopeptide and the therapeutic agent is directly or indirectly linked to the N terminus of the oligopeptide.

## Trouase

Trouase is the enzyme which is thought to be critical for specific activation of prodrug at the target tissue. Trouase is an endopeptidase which shows a remarkable degree of discrimination between leucine and isoleucine at the carboxyl side of the oligopeptide cleavage site. A defining characteristic is that under apropriate assay conditions, trouase readily cleaves succinyl-βAlaLeuAlaLeu-Daunorubicin while it is at least twenty-fold less active with succinyl-βAlalleAla-Leu-Daunorubicin.

Trouase is believed to be associated with target cells. Most likely it is generated either by target cells or by normal cells that are associated with the target cells, such as stromal tissue or macrophages. So, for example, the trouase may be secreted or present in some other manner in the extracellular vicinity of the target cell. In many cases, the prodrug of the invention includes a therapeutic agent for the treatment of cancer and the target cell is a tumor cell. Thus, trouase may be secreted extracellularly by the target cell or it may be present extracellularly because there is a fair amount of cell lysis associated with tumors generally. Cell lysis is also associated with inflammatory tissue, another target site.

Trouase activity is low in human plasma, however. Trouase activity has been observed in carcinoma cell extracts and conditioned media from cultured carcinoma cells, red blood cells and various human tissues, especially kidney. Carcinoma cell trouase has an apparent pI of ~5.1, a molecular weight by gel filtration of about 68 kD and a neutral pH activity optimum. It is inhibited by the metalloproteinase inhibitors EDTA and 1,10-phenanthroline but not serine, thiol, or acid proteinase inhibitors such as aminoethylbenzene-sufonate, E64, pepstatin, leupeptin, aprotinin, CA074, or fumagillin. Furthermore EDTA inactivated trouase can be re-activated by cobalt (50-100 $\mu$M) and manganese (50-1000 $\mu$M) but not zinc or cupric cations.

A partial purification scheme of trouase from HeLa (cervical carcinoma) cell homogenate ultracentrifugation (145,000x 30 min) supernatant consists of four steps as follows:

1. Anion exchange chromatography using a 15Q column (*Pharmacia*) eluted with a 0 to 0.5 M NaCl linear gradient in 20 mM triethylamine chloride pH 7.2,0.01% Triton X-100,
2. Affinity chromatography using Chelating Sepharose Fast Flow (*Pharmacia*) pre-loaded with $CoCl_2$ and eluted with a 0 to 100 mM imidazole linear gradient in 10 mM sodium phosphate, 0.5 M NaCl, pH 7.2, 0.01% Triton X-100, 0.02% $NaN_3$.
3. Preparative native electrophoresis
4. Gel filtration high performance liquid chromatography using a 7.8 mm X 60 cm TSK Gel G-3000SWXL (TosoHaas) eluted with 0.3 mL/min 50 mM potassium phosphate, 200 mM potassium sulfate, pH 7.2.

Further cleavage of the active portion of the prodrug released after trouase cleavage may occur intracellularly or extracellularly and is believed to be catalyzed by amino-exopeptidases. In vitro experiments indicate that amino-exopeptidases of broad specificities are present in human blood as well as the carcinoma cell environment.

**Stabilizing Group**

An important portion of the prodrug is the stabilizing group, which serves to protect the prodrug compound from degradation in circulating blood when it is administered to the patient and allows the prodrug to reach the vicinity of the target cell relatively intact. The stabilizing group protects the prodrug from degradation by proteinases and peptidases present in blood, blood serum, and normal tissue. Particularly, since the stabilizing group the N-terminus of the oligopeptide, and is therefore sometimes referred to as an N-cap or N-block, it serves to ward against exopeptidases to which the prodrug may otherwise be susceptible.

The compound is less toxic *in vivo* than the starting therapeutic agent because the prodrug is not cleaved in blood, heart, brain, bone marrow, in the mucosa and the like. This decrease in toxicity applies, in particular, to the acute effects such as marrow and mucosal toxicity, as well as possible cardiac or neurological toxicity.

Ideally, the stabilizing group is useful in the prodrug of the invention if it serves to protect the prodrug from degradation, especially hydrolysis, when tested by storage of the prodrug compound in human blood at 37°C for 2 hours and results in less than 20%, preferably less than 2%, cleavage of the prodrug by the enzymes present in the human blood under the given assay conditions.

The stabilizing groups of the invention are Succinic acid, Diglycolic acid, Maleic acid, Pyroglutamic acid, and Glutaric acid.

Additionally, intravascular administration of an aggregating positively charged prodrug in mice resulted in acute toxicity. However, no such toxicity was observed when the charge on this prodrug was reversed by derivitization with a negatively charged stabilizing group. This effect is discussed in greater detail below.

Thus, where aggregation of the therapeutic agent is a concern, it is preferred that the linked stabilizing group be negatively charged or neutral.

*In vivo* Toxicity

Many cytotoxic compounds inherently have low solubility. Positively charged anthracyclines for example form aggregates at high concentration and these aggregates induce intravenous coagulation when the aggregates are administered intravenously. We have discovered that trouase recognizes a specific set of hydrophobic peptide sequences. When one of these hydrophobic sequences (e.g., β-Ala-Leu-Ala-Leu) is conjugated to a cytotoxic compound (for example: Doxorubicin), it results in a less soluble compound which forms large aggregates when in aqueous formulations for IV infection (the preferred method of delivery of anti-cancer drugs). Since most peptides have exposed, positively-charged amino termini at physiological pH, these aggregates form a polypositively charged surface in vivo. These aggregates given intravenously induced a coagulation cascade and death in mice within a few minutes (usually less than 30 min) of administration. This renders any positively charged prodrugs that are formulated with aggregate suspension unsuitable for therapeutic use.

Several experiments support the hypothesis that aggregates are formed with peptide conjugated Doxorubicins. The examination of similarly formulated solutions by laser light scattering and size exclusion ultrafiltration demonstrated that only a small amount of the material had a molecular weight below 10 kD. The average molecular size of the aggregates were found to be around 70 kD. When the animals were concomitantly administered (see example 6) heparin with the IV dose the acute toxicity was greatly reduced or eliminated. When the animals were given dilute solutions of the same drug (same total dose) there was no acute toxicity. These results, taken together with literature reports, support the conclusion that peptide prodrugs of compounds that form aggregates because of insufficient solubility do not make optimal therapeutics. A solution to this aggregate problem makes these peptide prodrugs more practical. When these peptide prodrugs form aggregates because of insufficient solubility at the desired formulated concentrations, the stabilizing group on the peptide chain must terminate in a negatively charged or a neutral functionality. The use of succinyl as a stabilizing group on the peptide prodrug renders the produg not acutely toxic (see example 6). This solves an important problem in the use of peptide prodrugs as practical therapies for humans.

Since chemical radicals having more than two carboxylic acids are also acceptable as part of the prodrug, the end group having dicarboxylic (or higher order) carboxylic) acids is more generally defined as N-cap. The N-cap as used herein is a monoamide derivative of a chemical radical containing two or more carboxylic acids where the amide is attached onto the amino terminus of the peptides and the remaining carboxylic acids are free and uncoupled. For this purpose, the N-cap is preferably succinic acid, or glutaric acid, with succinic acid being most preferred. Other examples of useful N-caps in the prodrug compound of the invention include diglycolic acid, and maleic acid,

**Oligopeptide**

Oligopeptides are generally defined as polypeptides of short length, typically twenty amino acids or fewer. An oligopeptide useful in the prodrug of the invention is of at least four amino acids in length, however. At the upper end, oligopeptides of less than or equal to twelve amino acids are most useful, although an oligopeptide may have a chain length greater than twelve amino acids and fall within both the definition of the term as generally recognized in the scientific field. Thus, the oligopeptide portion of the prodrug of the invention has four to twelve amino acids, inclusive.

Numbering Scheme,

The oligopeptide has a formula or sequence $(AA)_n$-$AA^4$-$AA^3$-$AA^2$-$AA^1$ wherein:

each AA independently represents any genetically encoded amino acid;
n is an integer from 0 to 12;
$AA^4$ represents a non-genetically-encoded amino acid;
$AA^3$ represents any amino acid;
$AA^2$ represents any amino acid; and
$AA^1$ represents any amino acid.

This corresponds to a position sequence P(n+2)...P2-P1-P1'-P2'.
The trouase is believed to cleave between the P1 and P1' positions.
Unless otherwise indicated, all amino acids are in the L configuration.

Preferred amino acids in the oligopeptide are as follows:

In the P2 position, one of the following: β-Alanine, Thiazolidine-4-carboxylic acid, 2-Thienylalanine, 2-Naphthylalanine, D-Alanine, D-Leucine, D-Methionine, D-Phenylalanine, 3-Amino-3-phenylpropionic acid, γ-Aminobutyric acid, 3-amino-4,4-diphenylbutyric acid.

Also possible are Tetrahydroisoquinoline-3-carboxylic acid, 4-Aminomethylbenzoic acid, or Aminoisobutyric acid in the P2 position.

In the P1 position, one of the following: Leucine, Tyrosine, Phenylalanine, *p*-Cl-Phenylalanine, p-Nitrophenylalanine, Valine, Norleucine, Norvaline, Phenylglycine, Tryptophan, Tetrahydroisoquinoline-3-carboxylic acid, 3-Pyridylalanine, Alanine, Glycine, or Thienylalanine.

Also possible are Methionine, Valine, or Proline in the P1 position.

In the P1' position, one of the following: Alanine, Leucine, Tyrosine, Glycine, Serine, 3-Pyridylalanine, or 2-Thienylalanine.

Also possible are Aminoisobutyric Acid, Threonine, or Phenylalanine.

In the P2' position, one of the following: Leucine, Phenylalanine, Isoleucine, Alanine, Glycine, Tyrosine, 2-Naphthylalanine, or Serine.

Also possible is β-Alanine in the P2' position.

Oligopeptides used in the prodrug of the invention are: D-AlaThiβAlaβAlaLeuAlaLeu (SEQ ID NO: 1), ThiβAlaβAlaLeuAlaLeu (SEQ ID NO: 2), βAlaβAlaLeuAlaLeu (SEQ ID NO: 3), βAlaAlaAlaIle (SEQ ID NO: 4), βAlaAlaAlaLeu (SEQ ID NO: 5), βAlaPheTyrLeu (SEQ ID NO: 6), βAlaPheThrPhe (SEQ ID NO: 7), βAlaPheGlyIle (SEQ ID NO: 8), βAlaPheGlyLeu (SEQ ID NO: 9), βAlaPhePhePhe (SEQ ID NO: 10), βAlaPhePheIle (SEQ ID NO: 11), βAlaPhePheLeu (SEQ ID NO: 12), βAlaPheAlaIle (SEQ ID NO: 13), AlaPheAlaLeu (SEQ ID NO: 14), ThiGlyAlaLeu (SEQ ID NO: 15), NalGlyAlaLeu (SEQ ID NO: 16), βAlaLeuTyrLeu (SEQ ID NO: 17), βAlaLeuThiLeu (SEQ ID NO: 18), βAlaLeuThrPhe (SEQ ID NO: 19), βAlaLeuThrIle (SEQ ID NO: 20), βAlaLeuThrLeu (SEQ ID NO: 21), βAlaLeu (SerLeu (SEQ ID NO: 22), βAlaLeuPyrLeu (SEQ ID NO: 23), βAlaLeuLeuLeu (SEQ ID NO: 24), βAlaLeuGlyPhe (SEQ ID NO: 25), βAlaLeuGlyIle (SEQ ID NO: 26), ThiLeuGlyLeu (SEQ ID NO: 27), βAlaLeuGlyLeu (SEQ ID NO: 28), AibLeuGlyLeu (SEQ ID NO: 29), βAlaLeuPheIle (SEQ ID NO: 30), βAlaLeuPheLeu (SEQ ID NO: 31), βAlaLeuAibLeu (SEQ ID NO: 32), βAlaLeuAlaAla (SEQ ID NO: 33), βAlaLeuAlaβAla (SEQ ID NO: 34), βAlaLeuAlaPhe (SEQ ID NO: 35), βAlaLeuAlaGly (SEQ ID NO: 36), βAlaLeuAlaIle(SEQ ID NO: 37), βAlaLeuAlaLeu (SEQ ID NO: 38), TicLeuAlaLeu (SEQ ID NO: 39), ThzLeuAlaLeu (SEQ ID NO: 40), ThiLeuAlaLeu (SEQ ID NO: 41), NalLeuAlaLeu (SEQ ID NO: 42), NAALeuAlaLeu (SEQ ID NO: 43), D-LeuLeuAlaLeu (SEQ ID NO: 44), D-AlaLeuAlaLeu (SEQ ID NO: 45), D-MetLeuAlaLeu (SEQ ID NO: 46), APPLeuAlaLeu (SEQ ID NO: 47), AmbLeuAlaLeu (SEQ ID NO: 48), βAlaLeuAlaNal (SEQ ID NO: 49), βAlaLeuAla (Ser (SEQ ID NO: 50), βAlaLeuAlaTyr (SEQ ID NO: 51), βAlaMetTyrPhe (SEQ ID NO: 52), βAlaMetTyrLeu (SEQ ID NO: 53), βAlaMetGlyIle (SEQ ID NO: 54), ThiMetGlyLeu (SEQ ID NO: 55), βAlaMetPhePhe (SEQ ID NO: 56), βAlaMetPheIle (SEQ ID NO: 57), TicMetAlaLeu (SEQ ID NO: 58), NalMetAlaLeu (SEQ ID NO: 59), NAAMetAlaLeu (SEQ ID NO: 60), βAlaMetAlaLeu (SEQ ID NO: 61), APPMetAlaLeu (SEQ ID NO: 62), βAlaNleTyrIle (SEQ ID NO: 63), βAlaNleTyrLeu (SEQ ID NO: 64), βAlaNleThrIle (SEQ ID NO: 65), βAlaNleThrLeu (SEQ ID NO: 66), βAlaNleGlyPhe (SEQ ID NO: 67), βAlaNleGlyIle (SEQ ID NO: 68), βAlaNleGlyLeu (SEQ ID NO: 69), βAlaNlePheIle (SEQ ID NO: 70), βAlaNleAlaIle (SEQ ID NO: 71), βAlaNleAlaLeu (SEQ ID NO: 72), βAlaNleAlaPhe (SEQ ID NO: 73), βAlaNvaAlaLeu (SEQ ID NO: 74), βAlaPheTyrIle (SEQ ID NO: 75), ThiProGlyLeu (SEQ ID NO: 76), ThiProAlaLeu (SEQ ID NO: 77), NalProAlaLeu (SEQ ID NO: 78), βAlaProAlaLeu (SEQ ID NO: 79), βAlaPhe(Cl),AlaLeu (SEQ ID NO: 80), βAlaPhe(NO$_2$), AlaIle(SEQ ID NO: 81), βAlaPhe(NO$_2$),AlaLeu (SEQ ID NO: 82), βAlaPhgAlaLeu (SEQ ID NO: 83), βAlaPyrAlaLeu (SEQ ID NO: 84), TicThrGlyLeu (SEQ ID NO: 85), βAlaThiGlyIle (SEQ ID NO: 86), βAlaThiAlaLeu (SEQ ID NO: 87), βAlaTicAlaIle (SEQ ID NO: 88), βAlaTicAlaLeu (SEQ ID NO: 89), βAlaValAlaLeu (SEQ ID NO: 90), βAlaTrpAlaLeu (SEQ ID NO: 91), βAlaTyrTyrPhe (SEQ ID NO: 92), βAlaTyrTyrIle (SEQ ID NO: 93), βAlaTyrTyrLeu (SEQ ID NO: 94), βAlaTyrThrLeu (SEQ ID NO: 95), βAlaTyrPheLeu (SEQ ID NO: 96), βAlaTyrGlyIle (SEQ ID NO: 97), ThiTyrGlyLeu (SEQ ID NO: 98), βAlaTyrGlyLeu (SEQ ID NO: 99), βAlaTyrPheIle (SEQ ID NO: 100), βAlaTyrAlaIle (SEQ ID NO: 101), ThiTyralaLeu (SEQ ID NO: 102), and βAlaTyrAlaLen (SEQ ID NO: 103).

Blocking Amino Acid

The oligopeptide portion of the prodrug includes a blocking amino acid as AA$^4$ of the oligopeptide sequence, i.e. at position P2 of the position sequence, according to the numbering scheme described above. The blocking amino acid is a non-genetically-encoded amino acid.

The function of the blocking amino acid at position P2 is to maintain selectivity for cleavage of the prodrug by trouase and inhibit cleavage of the oligopeptide by other enzymes in that portion of the oligopeptide most closely linked (directly linked or indirectly linked) to the therapeutic agent portion of the prodrug compound. More particularly, by placing a blocking amino acid at position P2, undesirable cleavage within the peptide linkages of the four amino acids of the oligopeptide sequence AA$^4$-AA$^3$-AA$^2$-AA$^1$ and position sequence P2-P1-P1'-P2' is reduced. It is believed that trouase cleaves between the P1 and P1' positions of the oligopeptide. Since it is known that blood and normal cells are associated with a variety of peptidases, placing a blocking amino acid at position P2 serves to protect the oligopeptide portion of the prodrug *in vivo* until the prodrug is in the vicinity of the target cell. Specifically, by placing a blocking amino acid at position P2, it is believed that the oligopeptide is protected from undesirable clevage between P2 and P1. Without the blocking amino acid, the prodrug might be vulnerable to both exopeptidases and endopeptidases present in blood and normal tissue, both classes of enzymes which might otherwise degrade the prodrug before it reaches its target. Example 2 below illustrates this important feature of the prodrug.

**Therapeutic Agents**

Therapeutic agents that are particularly useful for modification to a prodrug form according to the invention are those with a narrow therapeutic window. A drug or therapeutic agent with a narrow therapeutic window is one in which the dose at which toxicity is evident, by general medical standards, is too close to the dose at which efficacy is evident.

The therapeutic agent conjugated to the stabilizing group and oligopeptide and, optionally, the linker group to form the prodrug of the invention may be useful for treatment of cancer, inflammatory disease, or some other medical condition. Preferably, the therapeutic agent is selected from the following classes of compounds: Alkylating Agents, Antiproliferative agents, Tubulin Binding agents, Vinca Alkaloids, Enediynes, Podophyllotoxins or Podophyllotoxin derivatives, the Pteridine family of drugs, Taxanes, Anthracyclines, Dolastatins, Topoiosomerase inhibitors, cis-Platins.

Particularly, the therapeutic agent is advantageously selected from the following compounds: Doxorubicin, Daunorubicin, Vinblastine, Vincristine, Calicheamicin, Etoposide, Etoposide phosphate, CC-1065, Duocarmycin, KW-2189, Methotrexate, Methopterin, Aminopterin, Dichloromethotrexate, Docetaxel, Paclitaxel, Epithiolone, Combretastatin, Combretastatin A$_4$ Phosphate, Dolastatin 10, Dolastatin 11, Dolastatin 15, Topotecan, Camptothecin, Mitomycin C, Porfiromycin, 5-Fluoracil, 6-Mercaptopurine, Fludarabine, Tamoxifen, Cytosine arabinoside, Adenosine Arabinoside, Colchicine, Carboplatin, Mitomycin C, Bleomycin, Melphalan or a derivative or analog thereof.

**Linker Groups**

A linker group between the oligopeptide and the therapeutic agent may be advantageous for reasons such as the following:

1. As a spacer for steric considerations in order to facilitate enzymatic release of the AA$^1$ amino acid.
2. To provide an appropriate attachment chemistry between the therapeutic agent and the oligopeptide.
3. To improve the synthetic process of making the prodrug conjugate (e.g., by pre-derivitizing the therapeutic agent or oligopeptide with the linker group before conjugation to enhance yield or specificity.)
4. To improve physical properties of the prodrug.
5. To provide an additional mechanism for intracellular release of the drug.

Linker structures are dictated by the required functionality. Examples of potential linker chemistries are hydrazide, ester, ether, and sulphydryl. Amino caproic acid is an example of a bifunctional linker group. When amino caproic acid is used in the linker group, it is not counted as an amino acid in the numbering scheme of the oligopeptide.

**Pharmaceutical Compositions**

The invention also includes a pharmaceutical composition comprising a compound, particularly a prodrug compound, according to the invention and, , a pharmaceutically acceptable adjuvant or vehicle.

The invention also relates to the use of the pharmaceutical composition for the preparation of a medicinal product intended for the treatment of a medical condition.

The pharmaceutical composition may, for example, be administered to the patient parenterally, especially intravenously, intramuscularly, or intraperitoneally. Pharmaceutical compositions of the invention for parenteral administration comprise sterile, aqueous or nonaqueous solutions, suspensions, or emulsions. As a pharmaceutically acceptable solvent or vehicle, propylene glycol, polyethylene glycol, injectable organic esters, for example ethyl oleate, or cyclodextrins may be employed. These compositions can also comprise wetting, emulsifying and/or dispersing agents.

The sterilization may be carried out in several ways, for example using a bacteriological filter, by incorporating sterilizing agents in the composition or by irradiation. They may also be prepared in the form of sterile solid compositions which may be dissolved at the time of use in sterile water or any other sterile injectable medium.

The pharmaceutical composition may also comprise adjuvants which are well known in the art (e.g., vitamin C, antioxidant agents, etc.) and capable of being used in combination with the compound of the invention in order to improve and prolong the treatment of the medical condition for which they are administered.

Doses for administration to a patient of the compounds according to the invention are generally at least the usual doses of the therapeutic agents known in the field, described in Bruce A. Chabner and Jerry M. Collins, Cancer Chemotherapy, Lippincott Ed., ISBN 0-397-50900-6 (1990) or they may be adjusted, within the judgment of the treating physician, to accommodate the superior effectiveness of the prodrug formulations or the particular circumstances of the patient being treated. The doses administered hence vary in accordance with the therapeutic agent used for the preparation of the compound according to the invention.

**Treatment with Prodrug Compound**

A method for the therapeutic treatment of a medical condition comprising administering, especially parenterally or intravenously, to the patient a therapeutically effective dose of the pharmaceutical composition is also disclosed.

The prodrug compound is useful for the treatment of many medical conditions including cancer, neoplastic diseases, tumors, inflammatory diseases, and infectious diseases. Examples of preferred diseases are breast cancer, colorectal cancer, liver cancer, lung cancer, prostate cancer, ovarian cancer, brain cancer, and pancreatic cancer. Formulated in pharmaceutically acceptable vehicles (such as isotonic saline), the prodrug compound can be administered to animals or humans in intravenous doses ranging from 0.05 mg/kg/dose/day to 300 mg/kg/dose/day. It can also be administered as intravenous drip or other slow infusion method.

Human patients are the usual recipients of the prodrug of the invention, although veterinary usage is also contemplated.

**PROCESS CHEMISTRY GENERAL PROCEDURES**

**Oligopeptide: General Method for the synthesis of peptides**

The peptide, or oligopeptide, sequences in the prodrug conjugates of this invention may be synthesized by the solid phase peptide synthesis (using either Boc or Fmoc chemistry) methods or by solution phase synthesis. The general Boc and Fmoc methods are widely used and are described in the following references: Merrifield, J. A. Chem. Soc., 88: 2149 (1963); Bodanszky and Bodanszky, The Practice of Peptide Synthesis, Springer-Verlag, Berlin, 7-161 (1994); Stewart, Solid Phase Peptide Synthesis, Pierce Chemical, Rockford, (1984).

**General Fmoc Solid Phase Method**

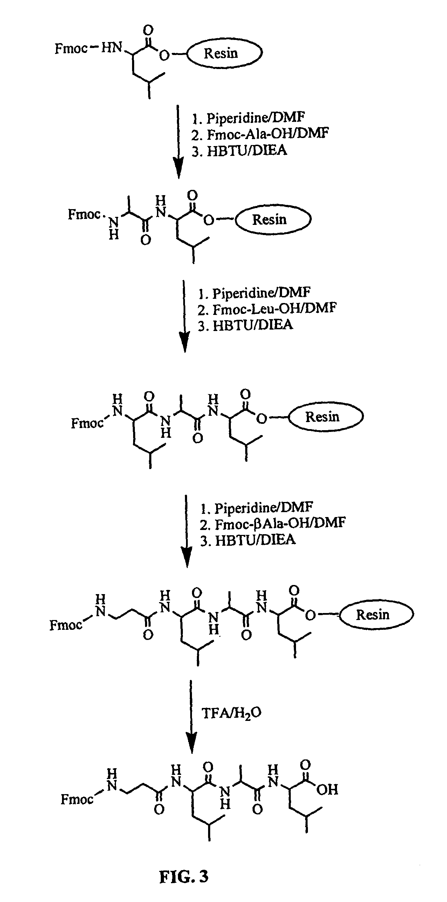

Using the preferred solid phase synthesis method, either automated or manual, a peptide of desired length and sequence is synthesized through the stepwise addition of amino acids to a growing chain which is linked to a solid resin. Examples of useful Fmoc compatible resins include, but are not limited to, Wang resin, HMPA-PEGA resin, Rink acid resin, or a hydroxyethyl-photolinker resin. The C-terminus of the peptide chain is covalently linked to a polymeric resin and protected α-amino acids were added in a stepwise manner with a coupling reagent. A preferred α-amino protecting group is the Fmoc group, which is stable to coupling conditions and can readily be removed under mild alkaline conditions. The reaction solvents are preferably but not limited to DMF, NMP, DCM, MeOH, and EtOH. Examples of coupling agents are: DCC, DIC, HATU, HBTU. Cleavage of the N-terminal protecting group is accomplished in 10 - 100% piperidine in DMF at 0 - 40°C, with ambient temperature being preferred. At the end of synthesis the final Fmoc protecting group is removed using the above N-terminal cleavage procedure. The remaining peptide on resin is cleaved from the resin along with any acid sensitive side chain protecting groups by treating the resin under acidic conditions. For example an acidic cleavage condition is a mixture of trifluroacetic acid (TFA) in dichloromethane. If the hydroxyethyl-photolinker resin is used, the appropriate wavelength for inducing cleavage is λ 365 nm ultraviolet light. A diagramatic representation of this process is given in Fig. 3.

**General N-cap Method via Solid Phase Synthesis**

The preparation of N-terminus derivatized peptides is conveniently accomplished on solid phase. When the peptide synthesis is complete and the terminal Fmoc is removed while the peptide is still on the solid support. The N-cap of choice is coupled next using standard peptide coupling conditions onto the N-terminus of the peptide. On completion of the N-cap coupling the peptide is cleaved from the resin using the procedure described above.

**General Boc Solid Phase Method**

For the solid phase method using Boc chemistry, either the Merrifield resin or PAM resin is useful. The amino acids are coupled to the growing chain on solid phase by successive additions of coupling agent activated Boc-protected amino acids. Examples of coupling agents are: DCC, DIC, HATU, HBTU. The reaction solvents may be DMF, DCM, MeOH, and NMP. Cleavage of the Boc protecting group is accomplished in 10 - 100% TFA in DCM at 0 - 40°C, with ambient temperature being preferred. On completion of the peptide chain assembly the N-terminus protecting group (usually Boc) is removed as described above. The peptide is removed from the resin using liquid HF or trifluoromethane sulfonic acid in dichloromethane.

**General Procedure for the Preparation of Fmoc Oligopeptide by Solution Phase Synthesis**

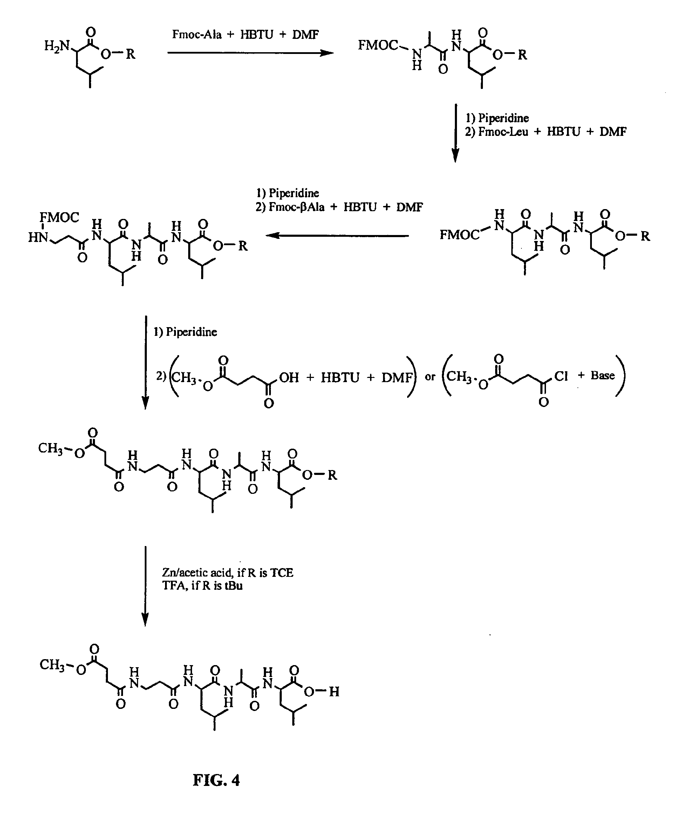

Alternatively, the prodrug peptide intermediate may be made via a solution phase synthesis, utilizing either Boc or Fmoc chemistry. In the diagrammatic presentation of the methods (Fig. 4), the C-terminal Leu tetrapeptide is generally used as an example, but it will be understood that similar reactions may be performed with other C-terminal tetrapeptides, as well. The peptide can be built up by the stepwise assembly in analogy to the solid phase method (in the N-terminal direction or in the C-terminal direction) or through the coupling of two suitably protected dipeptides or a tripeptide with a single amino acid.

One method of solution phase synthesis is a stepwise building up of the prodrug peptide intermediate using Fmoc chemistry, shown in Fig. 4. The C-terminus must be protected to reduce the formation of side products. The C-terminal R group in Fig. 4 is Me, tBu, benzyl or TCE. (Note when the N-cap is methyl succinyl the C-terminus R group cannot be Methyl.) Although DMF is given as the solvent, other solvents such as DMSO, CH₃CN, or NMP (or mixtures thereof) may be substituted therefor. Pyridine, Et₃N or other bases may be substituted for piperidine in deprotecting the growing peptide chain protected amino terminus. Similarly, although HBTU is given in the diagram above as the activating agent, other activating agents such as DCC, DIC, DCC + HOBt, OSu, activated esters, azide, or triphenyl phosphoryl azide may be used. Additionally, the protected peptide acid chloride or acid bromide may be used to couple directly to the amino acid or peptide fragment. On completion of the Oligopeptide assembly the N-terminus deprotected and the C-terminus protected peptide is ready to accept the desired N-cap.

**General Procedure for the Preparation of N-cap Oligopeptide via Solution Phase Synthesis**

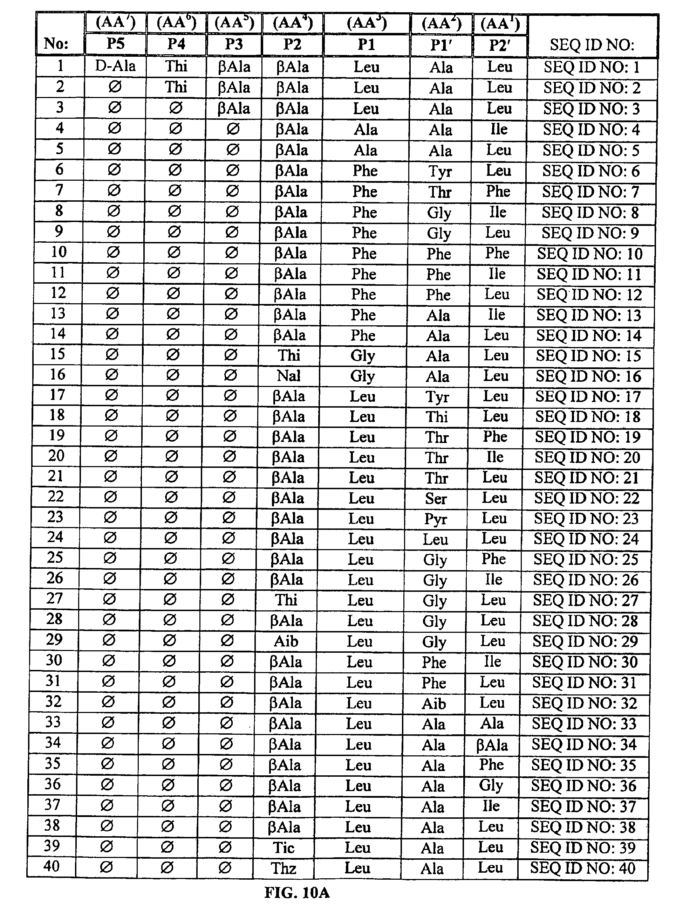

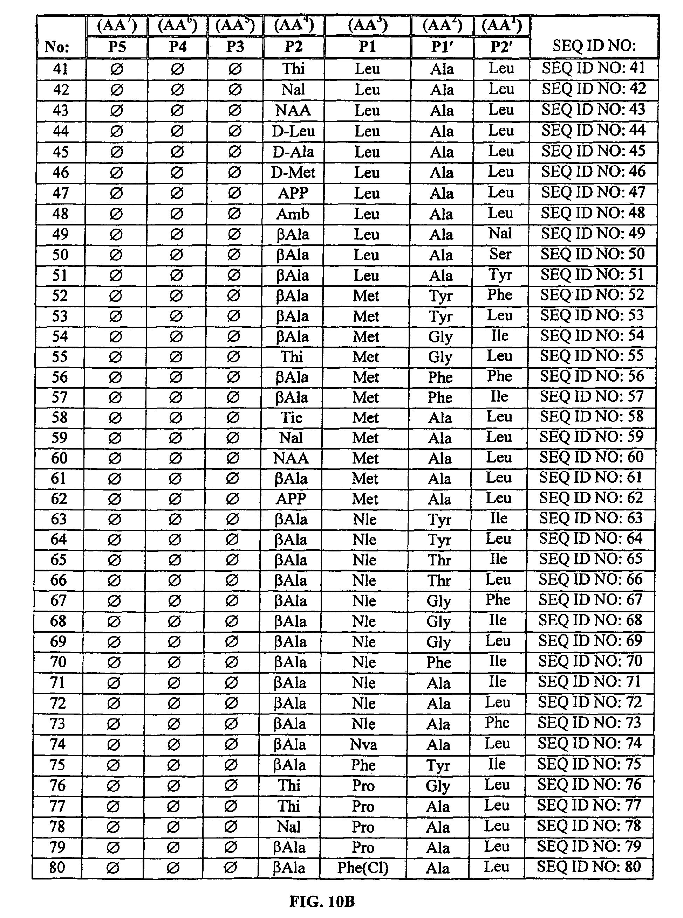

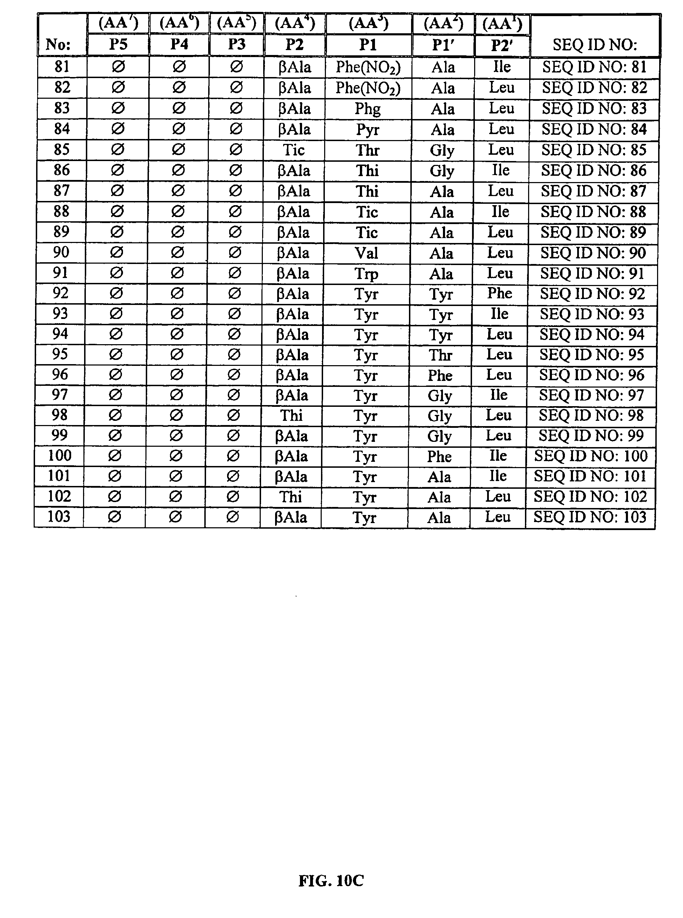

When constructing the N-capped Oligopeptide by solution phase synthesis, the N-cap needs to be synthesized by a slightly modified procedure (Fig. 4). First the C-terminus of the Fmoc oligopeptide needs to be protected with an acid labile or hydrogenation sensitive protecting group compatible with the selective deprotection of the C-terminus over the N-cap. Then the Fmoc protecting group needs to be removed from the oligopeptide to reveal the N-terminus. With the N-terminus deprotected and the C-terminus protected, the Oligopeptide is reacted with the activated hemiester of the desired N-cap. The N-cap can be activated using methods for activating amino acids such as DCC or HATU in base and an appropriate solvent. Alternatively, where the methyl-hemisuccinate is used, the coupling may also be done via methyl hemisuccinyl chloride (or other acid halide) (Fig. 4) using an inert solvent in the presence of an organic or inorganic base, such as DIEA, triethylamine or $Cs_2CO_3$. One example of such a synthesis can be by reacting methyl-hemisuccinate and Oligopeptide 38 (see Fig. 10A) benzyl ester. The coupling method can be any one of the methods generally used in the art (see for example: Bodanszky, M., The Practice of Peptide Synthesis, Springer Verlag, 185 (1984); Bodanszky, M., Principles of Peptide Synthesis, Springer Verlag, 159 (1984). The benzyl group then can be removed by catalytic hydrogenation providing the desired N-cap methyl-succinyl form of Oligopeptide 38. Other examples of suitable, selectively removable C-terminal protecting groups can be, but are not limited to, tBu, alkoxy-methyl and TCE. Other methods of accomplishing this step are described in the literature.

Any combination of the above method can be considered, such as "fragment condensation" of di-, or tripeptides. The reaction conditions are well known in the art and detailed in the citations given. The advantage of the above described methods is the facile purification of the product produced by solution phase synthesis.

**PRODRUG CONJUGATE**

**General Methods for the Conjugation and Deprotection steps**

The N-cap form of the Oligopeptide-therapeutic agent (prodrug conjugates) described in this invention can be synthesized by coupling an Fmoc form (which means Fmoc is attached to the N-terminus of the Oligopeptide) of the oligopeptide with daunorubicin or any appropriate therapeutic agent using any of the standard activating reagents used in peptide synthesis. The solvent may be Toluene, ethyl acetate, DMF, DMSO, $CH_3CN$, NMP, THF, DCM or any other suitable inert solvent as is known in the art and the reagents are soluble therein. The preferred solvents are DMF and NMP. The appropriate temperature range is -25 to +25°C, with ambient temperature being preferred. The activating agent may be selected from one of the following: PyBOP, HBTU, HATU, EDC, DIC, DCC, DCC+HOBT, OSu activated esters, azide, or triphenylphosphorylazide. HBTU or HATU is the preferred activating agent. Alternatively, the acid chloride or the acid bromide of the protected peptide can also be used for this coupling reaction. 2-4 equivalent, advantageously 2-2.5 equivalent of a base is required for the coupling reaction. The base can be selected from inorganic bases such as $CsCO_3$, Na- or $K_2CO_3$, or organic bases, such as TEA, DIEA, DBU, DBN, DBO, pyridine, substituted pyridines, N-methyl-morpholine etc., preferably TEA, or DIEA. The reaction can be carried out at temperatures between -15 °C and 50 °C, advantageously between -10 °C and 10 °C. The reaction time is between 5-90 minutes and is advantageously 20-40 minutes. The product is isolated by pouring the reaction mixture into water and filtering the precipitate formed. The crude product can be further purified by recrystallization from DCM, THF, ethyl acetate, or acetonitrile, preferably from dichloromethane or acetonitrile. The isolated Fmoc form of the Oligopeptide therapeutic agent conjugate is then deprotected over 2-90 minutes, preferably 3-8 minutes, using a ten- to hundred- fold excess of base at a temperature between -10 °C and 50 °C. Ideally 5-60 equivalents of the base are preferred. Piperidine is the preferred base to deprotect Fmoc groups. The deprotected amino terminus of the Oligopeptide therapeutic agent conjugate is acylated by a diacid anhydride as an activated hemi-ester to give the final N-cap form of the Oligopeptide-therapeutic agent (prodrug).

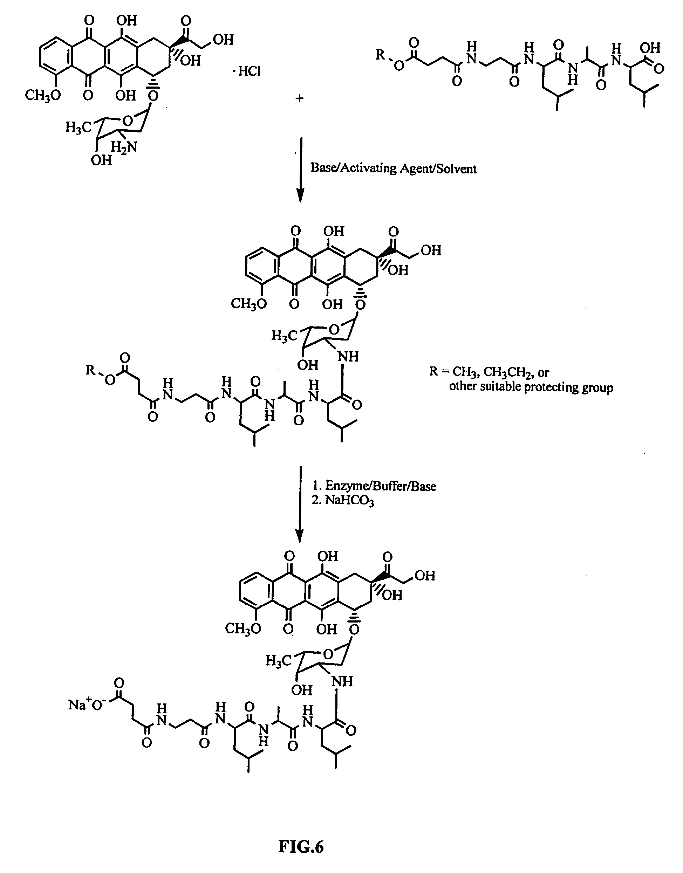

Alternatively, the final prodrug can be similarly prepared from the protected N-cap form of the Oligopeptide such as a methyl-hemi ester form of succinyl-N-cap Oligopeptide and conjugated to a therapeutic agent. This method is illustrated in Fig. 6.

The protected N-Cap-oligopeptide therapeutic agent is now deprotected by methods compatible to the stability of the Therapeutic agent. For example, for anthracyclines we protect with a methyl group and deprotect with an esterase. For other therapeutic agents we might select benzyl protecting groups and catalytic hydrogenation to deprotect.

The salt form of the negatively charged N-cap Oligopeptide therapeutic agent is carried out with a solvent selected from the following group: alcohol (including methanol, ethanol, or isopropanol), water, acetonitrile, tetrahydrofuran, diglyme or other polar solvents. The sodium source is one molar equivalent of $NaHCO_3$, NaOH, $Na_2CO_3$, NaOAc, $NaOCH_3$ (in general sodium alkoxide), or NaH. An ion exchange column charged with $Na^+$ (such as strong or weak ion exchangers) is also useful for this last step of making the salt form of the N-cap Oligopeptide therapeutic agent when appropriate. Sodium is described in this application as an example only. Any pharmaceutically acceptable salt can be used for negatively charged N-caps.

Generally, the prodrug may be converted to a pharmaceutically acceptable salt form to improve solubility of the prodrug. The N-cap-oligopeptide therapeutic agent is neutralized with a pharmaceutically acceptable salt e.g., $NaHCO_3$, $Na_2CO_3$, NaOH tris(hydroxymethyl)aminomethane, $KHCO_3$, $K_2CO_3$, $CaCO_3$, $NH_4OH$, $CH_3NH_2$, $(CH_3)_2NH$, $(CH_3)_3N$, acetyltriethylammonium. The preferred salt form of prodrug is sodium and the preferred neutralizing salt is $NaHCO_3$.

It is well documented that anthracycline type molecules, including doxorubicin and daunorubicin form gels in organic solvents in very low concentrations (Matzanke, B. F., et al., Eur. J. Biochem., 207:747-55 (1992); Chaires, J. B., et al., Biochemistry, 21:3927-32 (1982); Hayakawa, E., et al., Chem. Pharm. Bull., 39:1282-6 (1991). We have found this to be a considerable obstacle to getting high yields of clean product when making peptide anthracycline conjugates. The gel formation contributes to the formation of undesirable side reactions. One way to minimize this problem is to use very dilute solutions (1-2%) for the coupling reaction, however it is not practical in a process environment (large amounts of waste, complicated isolation). To overcome this problem we have invented a method wherein urea and other chaotropic agents are used to break up the strong hydrophobic and hydrogen bonding forces forming the gel. Thus if the coupling reaction is carried out in a urea containing solvent, advantageously 20% to saturated solution of urea in DMF or NMP the side reactions can be kept below 2% even if the concentration of reactants exceeds 10%. This invention makes practical the conjugation step at high concentrations and produces good yields and improved purity over the procedures that do not use urea as other isotopic agents.

**General Enzyme Method**

Hydrolyses of protected N-cap-oligopeptide therapeutic agents to the full N-cap compound catalyzed by acids or bases leads to complex reaction mixture due to the lability of many therapeutic agents even under moderately acidic or basic conditions. We have found that enzymes can promote the hydrolysis without destroying the substrate or the product. Enzymes suitable for this reaction can be selected from esterases, lipases and can be in their natural, water soluble forms or immobilized by cross coupling, or attachment to commercially available solid supporting materials. Of the soluble enzymes evaluated Candida Antarctica "B" lipase (Altus Biologics) is especially useful. Examples of enzymes immobilized by cross coupling is ChiroCLEC-PC™ (Altus Biologics). Candida Antarctica "B" lipase (Altus Biologics) can be immobilized by reaction with NHS activated Sepharose™ 4 Fast Flow (American Pharmacia Biotech). The pH of the reaction mixture during the hydrolysis is carefully controlled and maintained by a pH-stat between 5.5 and 7.5, advantageously between 5.7 and 6.5, via controlled addition of $NaHCO_3$ solution. When the reaction is completed the product is isolated by lyophilization of the filtered reaction mixture. The immobilized enzymes remains on the filter cake and can be reused if desired.

General Allyl Ester Method

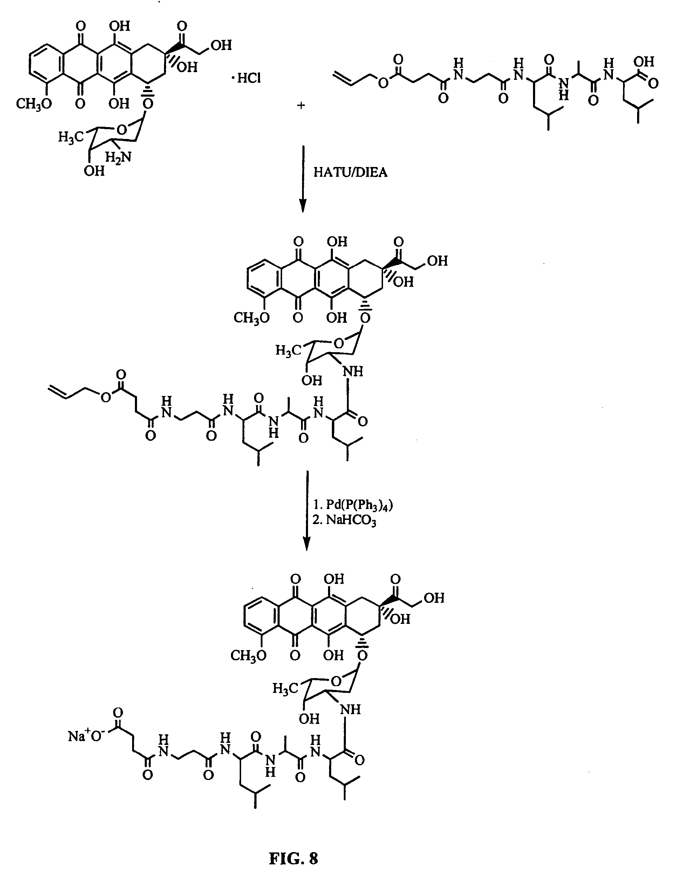

The prodrug can also be prepared via coupling allyl-hemiesters form of the N-cap oligopeptide with a therapeutic agent and then liberating the free acid from the conjugate. Fig. 8 illustrates this process with Succinyl-β-Ala-Leu-Ala-Leu and doxorubicin.

The coupling of allyl-succinyl-β-Ala-Leu-Ala-Leu with doxorubicin can be carried out via any one of the oligopeptide conjugation methods.

Allyl-succinyl-β-Ala-Leu-Ala-Leu-doxorubicin can also be synthesized by reacting allyl hemisuccinate, which was prepared via known methods (Casimir, J. R., et.al., Tet. Lett. 36/19 3409 (1995)), with β-Ala-Leu-Ala-Leu-doxorubicin similarly as coupling of the protected tetrapeptide precursors to doxorubicin was described in the previous methods. Suitable inert solvents are THF, dichloromethane, ethyl acetate, toluene, preferably THF from which the acid form of the product precipitates as the reaction progresses. The isolated acid is converted to its sodium salt as described earlier. Reaction times vary between 10-180 minutes, advantageously 10-60 minutes, at temperatures between 0-60 °C, preferably 15-30 °C.

Removal of the allyl-group can be done with Pd (0), or Ni(0), advantageously Pd(0) promoted transfer of the allyl group to acceptor molecules, as it is well known in the art and documented in the professional literature (Genet, J-P, et al., Tet. Lett., 50, 497, 1994; Bricout, H., et.al. Tet. Lett., 54:1073 (1998), Genet, J-P. et.al. Synlett, 680 (1993); Waldmann, H., et.al., Bioorg. Med. Chem., 7:749 (1998); Shaphiro, G., Buechler, D., Tet. Lett., 35:5421 (1994)). The amount of catalyst can be 0.5-25 % mol to the substrate.

General Trityl or Substituted Trityl Method

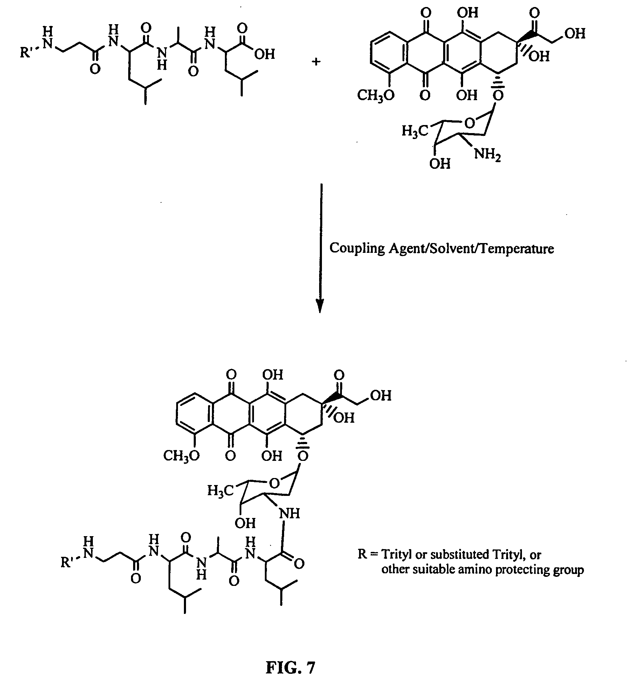

The prodrug may also be synthesized via the method shown in Fig. 7 this approach utilizes an R'-tetrapeptide, where R' is trityl or substituted trityl. The Coupling of R'-tetrapeptide with a therapeutic agent can be carried out via any one of the methods described earlier for conjugation of an protected oligopeptide with a therapeutic agent at 30-120 minutes at 0-20°C.

Removal of trityl or substituted trityl group can be achieved under acidic conditions to give the positively charged prodrug. This positively charged prodrug is N-capped as illustrated in Fig. 4 and described earlier. The trityl deprotection can be accomplished with acetic acid, formic acid and dilute hydrochloric acid.

The prodrug can be converted into succinyl or glutaryl oligopeptide 38 therapeutic agent by reacting with succinic anhydride. Succinyl or glutaryl Oligopeptide 38 therapeutic agent can be converted to any pharmaceutically acceptable salt. The solvent for coupling step DMF, DMSO, $CH_3CN$, NMP, or any other suitable solvent is known in the art.

General Inverse Direction Solid Phase Conjugation Method

The prodrug compound of the present invention can be synthesized by using solid phase chemistry via "step wise" inverse (from the N-terminal to the C-terminal) direction methods.

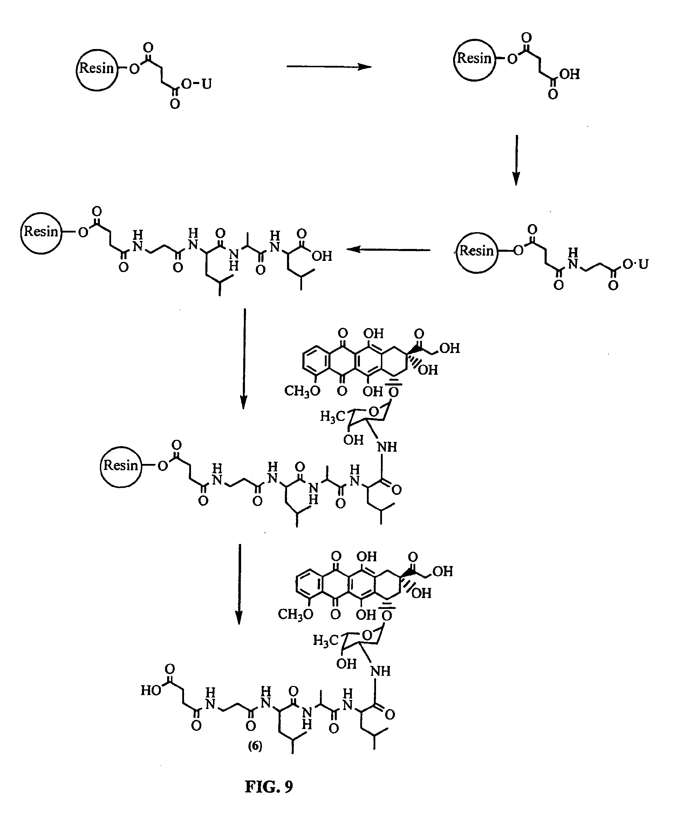

One way is to use resins to immobilize a succinyl-hemi ester, for example succinyl-mono-benzyl ester or -allyl ester. Examples of resins could be selected are "Wang Resins" (Wang, S. S., J. Am. Chem. Soc., 95:1328 (1973); Zhang, C., Mjaili, A. M. M., Tet. Lett., 37:5457(1996)), "Rink Resins" (Rink, H., Tet, Lett., 28:3787 (1987)), "Trityl-, or substituted-trityl Resins" (Chen, C., et.al., J. Am. Chem. Soc., 116:2661 (1994); Bartos, K. et.al., Peptides, Proc. 22nd European Peptide Symposium (1992); Schneider, C. H.; Eberle, A. N. (Eds.), ESCOM, Leiden, pp. 281 (1993). The immobilized ester is then deprotected and reacted with similarly C-terminal protected β-alanine. These steps are than repeated with leucine-, alanine, and finally leucine esters, followed by the coupling of doxorubicin to the immobilized succinyl-tetrapatide. The molecule is than liberated from the resin by using mildly acidic conditions to form free succ-Oligopeptide 38-Doxorubicin. This methodology is represented on the scheme of Fig. 9. Another version of phase synthesis would be if the succinyl tetrapeptide ester is immobilized. It is then C-terminally deprotected, followed by the coupling step to doxorubicin and finally liberated from the resin as represented on the scheme of Fig. 9. The acid form of the prodrug molecule is converted finally into its sodium salt as described above.

Specific Compounds

Compounds of the invention include the prodrugs, Suc-βAla-Leu-Ala-Leu-Dox, Suc-βAla-Leu-Ala-Leu-Dnr, and Glutaryl-βAla-Leu-Ala-Leu-Dox.

Additionally, the following intermediate compounds are important to the process of preparation of the prodrugs of the invention

**Intermediates:**

βAla-Leu-Ala-Leu-Dox
Trityl-βAla-Leu-Ala-Leu-Dox
Diphenylmethyl-βAla-Leu-Ala-Leu-Dox
Benzyloxycarbonyl- βAla -Leu-Ala-Leu-Dox
Fmoc- pAla -Leu-Ala-Leu-OBn
βAla-Leu-Ala-Leu-OBn
Methyl-succinyl- pAla -Leu-Ala-Leu-OBn
Methyl-succinyl- βAla -Leu-Ala-Leu
Fmoc-βAla-Leu-Ala-Leu
Fmoc-Thi-Tyr-Gly-Leu
Fmoc- βAla -Leu-Ala-Leu-Dnr
Fmoc-Thi-Tyr-Gly-Leu-Dnr
Suc-Thi-Tyr-Gly-Leu-Dnr
Gl-βAla -Leu-Ala-Leu-Dox
βAla -Leu-Ala-Leu-Dox Lactate
Allyl-succinyl-βAla -Leu-Ala-Leu-Dox
Suc-βAla -Leu-Ala-Leu
Methyl esters of Suc-βAla -Leu-Ala-Leu
Fmoc-βAla -Leu-Ala-Leu-Dox
Methyl-succinyl-Aβla -Leu-Ala-Leu-Dox, and
Allyl-hemi succinate.

## EXAMPLES

**Example 1:**

**Screening of potential prodrugs with trouase and human blood**

Based on HPLC analysis of digestion products, activation of peptidyl-toxin to free toxin occurs via a series of enzyme catalyzed cleavage reactions. For example, the N-capped tetrapeptidyl toxin, succinyl-βAlanyl-leucyl-alanyl-leucyl-doxorubicin is converted to leucyl-doxorubicin in extracts of carcinoma cells or carcinoma cell conditioned media in two steps catalyzed by at least two enzymes. Initial endopeptidase cleavage occurs between the $AA^3$ (P1) and $AA^2$ (P1') amino acids to yield alanyl-leucyl-doxorubicin. Subsequently, exopeptidase removes alanine to give leucyl-doxorubicin which is known to be taken up into cells where the active toxin, doxorubicin, is released.

A good candidate for a higher therapeutic index N-capped peptidyl-toxin prodrug should be activated by cancer cells but be relatively stable in whole human blood. Three different preparations of carcinoma were used to screen various N-capped peptidyl-toxins. These three preparations were as follows:

(a) MCF 7/6 (breast carcinoma) cell homogenate
(b) MCF 7/6 (breast carcinoma) conditioned media, and
(c) HeLa (cervical carcinoma) cell extract anion exchange fraction pool.

Compounds which could be hydrolyzed to a single amino acid toxin conjugate were further tested for stability in whole human blood.

Test samples were incubated at 37°C for 2 hr with the three different preparations of carcinoma enzyme and with whole blood, extracted with acetonitrile, and analyzed by HPLC using fluorescence detection. With few exceptions, results for carcinoma enzyme cleavage were the same for a partially purified fraction from HeLa cells, MFC 7/6 cell homogenate, or MCF 7/6 conditioned media.

Preparation of carcinoma cell enzyme solutions:

(a) MCF 7/6 cell homogenate:

MCF 7/6 cells were grown to confluence in a serum free medium containing DMEM:F12 (1:1), 50 mg/L bovine serum albumin, ITS-X, and Lipid Concentrate. 100 mL of cells were harvested by centrifugation at 4°C 10,000xg, for 20 min and decanting the supernatant. The pellet was resuspended in 2 mL phosphate buffered saline *(Gibco)* and centrifuged at 18,000xg for 10 min. After decanting the supernatant, the cells (approximately 300 μL wet) were homogenized by grinding in 1.7 mL 10 mM pH 7.2 HEPES buffer (sodium salt). The homogenate was centrifuged at 18,000xg at 4°C for 5 min and the supernatant was aliquoted and stored at ≤-20°C for subsequent use in the compound screen.

(b) MCF 7/6 conditioned media:

MCF 7/6 cells were grown to confluence in DMEM/F12 (1:1) medium containing 10 % fetal bovine serum, 0.05% (w/v) L-glutamine, 250 IU/mL penicillin, and 100 μg/mL streptomycin. Cells were then washed twice with phosphate buffered saline and incubated 24 hr at 5% $CO_2$, 37°C, in DMEM/F12 (1:1), 0.02% BSA, ITS-X. The conditioned media was then decanted and, using a stirred cell apparatus with a YM10 (10.000 MW cutoff) ultrafiltration membrane(*Millipore*), exchanged once with 10 mM HEPES buffer, pH 7.2 and concentrated twenty-fold. This solution was stored in aliquots at-20°C for use in the compound screen.

(c) HeLa Cell anion exchange fraction pool:

Thirty billion commercially produced HeLa Cells (human cervical carcinoma, *Computer Cell Culture Center,* Seneffe, Belgium) were homogenized with a sonicator and with a Dounce homogenizer in 108 mL of aqueous lysis solution. The lysis solution contained 0.02% w/v Triton X-100, 0.04% w/v sodium azide, and a cocktail of protease inhibitors (2 tablets/ 50 mL Complete™, EDTA-free tablets, *Roche Molecular Biochemicals).* The cell homogenate was centrifuged 30 minutes at 4°C at 5000xg and the pellet was homogenized in a second 108 mL of lysis solution using a Dounce homogenizer and centrifuged as before. The supernatants were combined and centrifuged for 90 min at 60,000xg at 4°C.

**Chromatography**

A portion of the ultracentrifugation supernatant was diluted 2-fold with a 20 mM triethanolamine-HCl pH 7.2 buffer containing 0.01% (w/v) Triton X-100 and 0.02% (w/v) sodium azide (equilibration buffer). Thirty mL of the resulting solution, corresponding to approximately 180 mg of protein, was loaded at 4°C on a 2.6 x 9.4 cm Source™ 15Q *(Amersham Pharmacia Biotech)* low pressure anion exchange chromatography column (1 ml/minute). The column was then washed with 250 ml of the equilibration buffer and a flow rate of 1 mL/minute. Proteins were eluted in a NaCl linear concentration gradient (0-0.5 M in the equilibration buffer, total volume of the gradient was 1000 ml) at a flow rate of 3 ml/minute. Two-minute fractions were collected and used for enzyme activity determination using β-alanyl-leucyl-alanyl-leucyl-doxorubicin as the substrate. Its transformation into L-alanyl-L-leucyl-doxorubicin was quantified by reverse phase high performance liquid chromatography utilizing fluorescence detection of the anthracycline moiety. The fractions containing the highest activity levels were pooled (fractions #43-46; ~0.13 M NaCl), supplemented with protease inhibitors (Complete™, EDTA-free tablets, Roche Molecular Biochemicals), and stored as aliquots at -80°C.

Whole Human blood

Human blood was collected using commercial acid buffered citrate whole blood collection tubes.

Compound screen

Test compounds were incubated for 2 hr at 37°C at a concentration of 12.5 μg/mL with the following enzyme solutions:

a) MFC 7/6 cell homogenate diluted 1:27 in 10 mM HEPES, 1 mM $CoCl_2$, pH 7.2
b) MFC 7/6 conditioned media
c) Hela Cell anion exchange fraction pool 1 diluted 1:57 in 10 mM HEPES, 1 mM $CoCl_2$, pH 7.2.
d) Whole human blood containing 1 mM $CoCl_2$

Following incubation, three volumes of acetonitrile were added to stop the reaction and remove protein from the mixture. The sample was centrifuged at 18,000g for 5 minutes and 100 μL of supernatant was mixed with 300 μL of water prior to analysis by HPLC.

For HPLC analysis 50 μL of sample was injected on a 4.6 x 50 mm 2μ TSK Super-ODS chromatography column at 40°C and eluted with a 3 minute linear gradient from 26% to 68% acetonitrile in aqueous 20 mM ammonium acetate pH 4.5 buffer at 2 mL/min. Detection was by fluorescence using an excitation wavelength of 235 nm and an emission wavelength of 560 nm.

Oligopeptides that were cleaved by the trouase under the given conditions and were stable in human blood are shown in Figs. 10A-10C.

**Example 2:**

**Specificity for trouase is provided by a non-genetically encoded amino acid at position P2**

Specificity is afforded by incorporation of a non-genetically encoded rather than a genetically encoded amino acid at position P2. Specifically, (succinyl N-capped)-(Oligopeptide 38)-daunorubicin, which contains the non-genetically encoded amino acid β-alanine at position P2, was incubated for 2 hr at 37°C with each enzyme preparations prepared as described in Example 1. The extent of cleavage was then estimated by HPLC analysis of the resulting mixtures. These results were compared to results for the same incubations performed with the same compound except for a substitution of the genetically encoded amino acid L-alanine position at P2. The extent (rate) of cleavage by cell homogenate was 1.3 fold greater for the P2 L-alanine compound versus the P2 β-alanine compound. The extent of cleavage by conditioned medium was about the same for the two compounds. However, with the partially purified trouase preparation, the extent of cleavage of the P2 L-alanine compound was only 0.6 fold that of the P2 β-alanine compound. These results suggest that the presence of L-alanine at P2 may have provided a second cleavage site for the cruder mixtures of enzymes; thus reducing the likelihood that, *in vivo,* release of the active drug would be localized to tumor tissue.

**Example 3:**

**The Prodrug is Effective and Well-Tolerated in Tumor Xenograft Models**

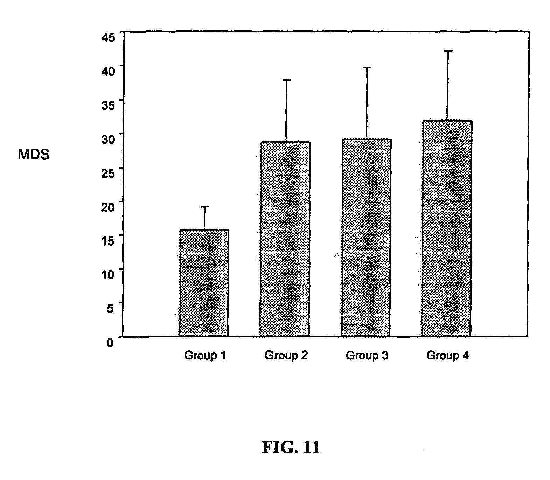

(Succinyl N-Cap)-(Oligopeptide 38)-Dox therapeutic agent has proven to be efficacious in inhibiting the growth of human tumors in several nude mouse xenograft models, including the estrogen-dependent MCF-7/6 mammary tumor and the adriamycin-resistant colorectal carcinomas CXF280/10 and LS-174T. For example, when groups of 10 mice with subcutaneously-implanted LS174T tumors were treated with five weekly intravenous doses of (Succinyl N-Cap)-(Oligopeptide 38)-Dox therapeutic agent, a significant, dose-dependent, replicable extension in the Mean Day of Survival (MDS) was observed, as well as in decreased size of the tumor (tumor volume) compared with vehicle-treated controls (Group 1) at doses of 57 (Group 2), 64 (Group 3) and 71 (Group 4) mg/kg of (Succinyl N-Cap)-(Oligopeptide 38)-Dox therapeutic agent, with the highest dose being equivalent to 40 mg/kg of doxorubicin (See Fig. 11). The drug was safe and well-tolerated under repeat-dose levels and frequencies of dosing that demonstrated anti-tumor efficacy. Some dose-dependent body weight loss was observed. In suporting studies, kidney toxicity and myelosuppression were not observed at doses of up to 106.8 mg/kg of (Succinyl N-Cap)-(Oligopeptide 38)-Dox therapeutic agent.

**Example 4**

**The Prodrug is Safer and More Effective than Comparators**

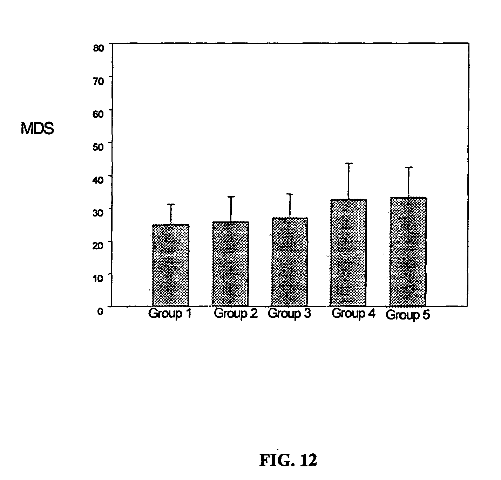

Significantly higher doses of (Succinyl N-Cap)-(Oligopeptide 38)-Dox therapeutic agent could be administered compared with doxorubicin, achieving efficacy without significant toxicity in the LS-174T human colorectal carcinoma xenograft model. (Succinyl (Group 5) N-Cap)-(Oligopeptide 38)-Dox therapeutic agent, at efficacious doses of 49 (Group 3), 57 (Group 4) and 64 mg/kg (Group5); showed superior efficacy compared to doxorubicin at 3.0 mg/kg (Group 2) and saline (Group 1), in inhibiting the rapidly-growing adriamycin-resistant LS-174T tumor (Fig. 12). Dose limiting toxicity (cardiotoxicity and myelosuppression) has been observed with repeated administration of doxorubicin at or above 3 mg/kg (Group 2). Thus we have demonstrated that higher doses of (Succinyl N-Cap)-(oligopeptide 38)-Dox therapeutic agent than doxorubicin can be administered, favoring tumor inhibition over systemic toxicity.

**Example 5**

**βAlaLeuAlaLeu-Dox Aggregation**

Poorly soluble anthracycline drugs have been shown to form aggregates when - prepared in aqueous buffers. Menozzi, et al., Self-association of doxorubicin and related compounds in aqueous solutions, J. Pharmaceut. Sci., 73 (6):766-770 (1984). Confalonieri, C. et al., The use of new laser particle sizer and shape analyser to detect and evaluate gelatinous microparticles suspended in reconstituted anthracycline infusion solutions, J. Pharmaceut. Biomed. Anal., 9 (1):1-8 (1991). An estimation of βAlaLeuAlaLeu-Dox aggregate size in a 17.4 μMol/ml aqueous solution by attempting to filter these solutions through Amicon Centricon™ filter units. Doxorubicin (17.4 μMol /ml) and βAlaLeuAlaLeu-Dox (17.4 μMol /ml) were each dissolved in distilled water and placed into Centricon filters with 3,000, 10,000, 30,000 and 50,000 molecular weight cutoff (MWCO). Each filter unit was centrifuged for 2 hr at 1500g force. The amount of the drug retained and passing through the filter was quantitated at λ475 nm and converted to a percent. Table 1 below shows that 81 % of the doxorubicin passed through the 3,000 MWCO filter while only 5 % of the conjugate, βAlaLeuAlaLeu-Dox passed through the 3,000 MWCO filter. The data also show that the 50,000 MWCO unit retains over 40 % of the βAlaLeuAlaLeu-Dox. These data demonstrate that a significant percentage of βAlaLeuAlaLeu-Dox aggregates were larger that 50 kD (>50 molecules/aggregate). This demonstrates that at the specified dose of Example 6, below, the conjugate was in an aggregated state. This data, therefore, support the hypothesis that Oligopeptide 38-Doxorubicin Therapeutic Agent aggregates contribute to the acute toxicity seen in these positively charged molecular aggregates.

**Table 1**

|  | 3000 MWCO | | 10000 MWCO | | 30000 MWCO | | 50000 MWCO | |
|---|---|---|---|---|---|---|---|---|
|  | Filt. | Ret. | Filt. | Ret. | Filt. | Ret. | Filt. | Ret. |
| **Dox** | 81% | 10% | 82% | 2% | n.d. | n.d. | 93% | 0.5% |

(continued)

| | 3000 MWCO | | 10000 MWCO | | 30000 MWCO | | 50000 MWCO | |
|---|---|---|---|---|---|---|---|---|
| | Filt. | Ret. | Filt. | Ret. | Filt. | Ret. | Filt. | Ret. |
| Conj. | 4.9% | 89% | 10% | 76% | 36% | 64% | 53% | 43% |

*Dox: doxorubicin; Conj: N-β-Ala-L-Leu-L-Ala-L-Leu-Doxorubicin;*

### Example 6

### Intravenous Injection of βAlaLeuAlaLeu-Dox in Mice

It is known that acute toxicity likely occurs through the interaction of positively charged polymers, such as protamines, polylysine, or their aggregates, and the luminal surface of blood vessels. DeLucia III, A., et al., Efficacy and toxicity of differently charged polycationic protamine-like peptides for heparin anticoagulation reversal, J. Vasc. Surg. 18:49-60 (1993). Ekrami, H. M. and Shen, W. C., Carbamylation decreases the cytotoxicity but not the drug-carrier properties of polylysines, J. Drug Targ., 2:469-475 (1995). It has been further shown that heparin reduces the toxic effects of protamine sulfate on rabbit myocardium. Wakefield, T. W., et al., Heparin-mediated reductions of the toxic effects of protamine sulfate on rabbit myocardium, J. Vasc. Surg., 16:47-53 (1992). To test the hypothesis that the acute toxicity seen here was due to positively charged prodrug aggregates, βAlaLeuAlaLeu-Dox (174 μMol /ml) was given to mice following a 1 hr pretreatment with either 4,000 I.U. heparin iv as compared to control (iv). Table 2 shows that following heparin, a formerly acutely lethal dose of βAlaLeuAlaLeu-Dox was significantly less toxic.

These data support the hypothesis that the acute toxicity is due to a positively charged aggregate causing a similar effect to that seen for protamines or polylysine. Negatively and neutrally charged prodrugs of the invention overcome this undesirable side effect.

**Table 2**

| ROUTE OF Pretreatment | HEPARIN DOSE LEVEL (I. U.) | Survival time (days) [Proportion] | Acute toxicity (Proportion) |
|---|---|---|---|
| *Control (iv)* | 0 | 0 | 5/5 |
| *i.p.* | 4000 | >9 [5/8] | 3/8 |
| | 8000 | >11 [3/3] | 0/3 |
| *i.v.* | 4000 | > 11 [2/3] | 1/3 |

In agreement with the aforementioned hypothesis, capping the terminal amino group of βAlaLeuAlaLeu-Dox with a negatively charged moiety resulted in the complete disappearance of the acute toxicity effect at dose levels as high as 250 mg doxorubicin, HCl eq./Kg.

As evidence of this, in a related experiment, all animals survived up to 8 days when three to five mice per group were treated with an iv bolus of 250 mg/kg (Dox-HCl eq.) succinyl βAlaLeuAlaLeu-Dox and glutaryl βAlaLeuAlaLeu-Dox.

### Analytical Methods for the remaining examples

The peptide sequences, synthesized using either solid or solution phase approaches, were used without further purification if the analytical HPLC (methods A, B & D) showed the crude product to be greater than 80% pure. If not, the material was purified using preparative HPLC Method C.

### HPLC Method A

Analytical HPLC analyses were performed on a Waters 2690 using a C-18 column (4μm, 3.9 x 150mm ID, flow rate 1mL/min) eluting with a gradient of solvent A (0.1 % TFA/$H_2O$) and solvent B (0.1 % TFA/ACN) and the data was processed at λ 254 nm using the Waters Millennium system. Analytical HPLC gradient started with 90% of solvent A and ended with 100 % of solvent B over a period of 14 minutes (linear). Purity of the compounds for this method and the following ones was assessed as the relative percentage area under the curve of the peaks.

**HPLC Method B**

Analytical HPLC analyses were performed on a Waters 2690 using a C-8 column (3.5μm, 4.6 x 150mm ID, flow rate 1mL/min) eluting with a gradient of solvent A (80% 20mM ammonium formate and 20% acetonitrile) and solvent B (20% 20mM ammonium formate and 80% acetonitrile) and the data was processed at λ 254 nm using the Waters Millennium system. Analytical HPLC gradient started with 100% of solvent A to 100% of solvent B over a period of 30 minutes (linear).

**HPLC Method C**

Preparative purification of crude products was achieved using a Waters Delta Prep 4000 system using a C-4 column (15μm, 40 x 100mm ID, flow rate 30 mL/min) eluting with a gradient of solvent A ($H_2O$) and solvent B (MeOH). The preparatory HPLC gradient started with 80% of solvent A and goes to 100 % of solvent B over a period of 70 minutes (linear). The data was processed at λ 254 nm using the Waters Millennium System.

**HPLC Method D**

Analytical HPLC was accomplished on a Hewlett Packard instrument using a TSK superODS column (Toso-Haas); solvent A (TFA 0.1% in water); solvent B (TFA 0.1% in acetonitrile); gradient: 30 to 36% of B in 2 minutes, 36 to 41% of B in 10 minutes, 41 to 90% of B in 3 minutes, 5 minutes at 90% B, detection wavelength λ 254 nm.

**NMR and MS**

Additional structural determinations were done by NMR and MS techniques and the results supported the claimed compounds.

**TLC Method**

TLC analysis was carried out on silica gel 60F-254nm-0.25mm plates (Merck) with DCM/MeOH/$H_2O$/Formic acid 88% 85/15/1/2 for elution.

**Ninhydrin Test**

A few milligrams of product were introduced in a test tube, and two drops of Solution A (50 mg/mL ninhydrin in ethanol), two drops of Solution B (4 mg/mL phenol in ethanol), then two drops of Solution C (2 mL 0.0 1 M KSCN, aqueous in 100 mL pyridine) were added. The mixture was left in a boiling water bath for five minutes. In the presence of a free amine the solution becomes purple.

**Specific Oligopeptide Synthetic Examples**

**Sources of commercially available reagents**

Doxorubicin and Daunorubicin were supplied by Meiji (Japan), Pd(PPh$_3$)$_4$ by Strem chem (Newburyport, MA), PEG by Shearwater( Huntsville, Alabama), solvents, HATU by Aldrich (Milwaukee, WI); all resins and amino acids were supplied by ABI (Foster City, CA), Novabiochem (San Diego, CA), Advanced ChemTech (Louisville, KY), Peptide International (Louisville, KY), or SynPep (Dublin, CA).

**Example 7**

**Fmoc form of Oligopeptide 38 benzyl ester [Fmoc-β-Ala-Leu-Ala-Leu-OBn]**

The Fmoc form of Oligopeptide 38 (24.34 g, 0.04 mol) was added into a round bottom flask with DMF (350 mL) and a magnetic stirrer. After the tetrapeptide was dissolved, benzyl bromide (4.76 mL, 0.04 mol), followed by cesium carbonate (13.04 g, 0.04 mol), was added to the solution with stirring. The reaction mixture was stirred at room temperature for 1.5 hrs. Then, the reaction mixture was slowly poured into a flask with 450 mL of iced water. A large amount of white solid precipitated out which was collected by suction filtration. The product was washed with water (2x200 mL) and placed in a vacuum desiccator. The product (24.2 g, 87%) was identified by HPLC (Purity: 95%). MS *m/z* calcd, for $C_{40}H_{50}N_4O_7$ 698.4, found 699.5.

**Example 8**

**Oligopeptide 38 benzyl ester [β-Ala-Leu-Ala-Leu-OBn]**

In a round bottom flask (25 mL), Fmoc form of Oligopeptide 38 benzyl ester (0.7 g, 1.0 mmol) was dissolved in 5 mL of anhydrous DMF. Piperidine (1.2 mL, 12.1 mmol) was added to the solution and the mixture was stirred at room temperature for 25 minutes. The reaction was quenched with water (6 mL) and extracted with ethyl acetate (2x10 mL). The combined organic layer was further washed by water (2x5 mL), brine (5 mL) and dried over sodium sulfate. A white solid (0.8 g)was obtained after removal of solvent. The purity of the product was only 67%. MS *m/z* calcd. for $C_{25}H_{40}N_4O_5$ 476.3, found 477.2.

**Example 9**

**Methyl Succinyl-N-cap form of Oligopeptide 38 benzyl ester [mono-Methyl-succinyl-β-Ala-Leu-Ala-Leu-OBn]**

In a round bottom flask (250 mL), methyl hemisuccinnate (3.19 g, 24.2 mmol) was dissolved in anhydrous DMF (50 mL). DIEA (4.22 mL, 24.2 mmol) followed by HBTU (9.17 g, 24.2 mmol) were added into the solution. The mixture was stirred at room temperature for 45 minutes. To this mixture was added a solution of Oligopeptide 38 benzyl ester (crude, containing 10.14 g, 21.3 mmol) in anhydrous DMF (150 mL). The mixture was continually stirred at room temperature for 2.5 hrs. Then, the reaction mixture was slowly poured into a flask with 200 mL of iced water while stirring. A large amount of white solid precipitated out which was extracted by ethyl acetate (3x200 mL). The combined organic layer was further washed by water (2x200 mL), brine (200 mL) and dried over sodium sulfate. A white solid was obtained after removal of solvent. Recrystallization of this crude product in ethyl acetate afforded 7.53 g of product (60%) with purity of 80%. MS *m/z* calcd. for $C_{30}H_{46}N_4O_8$ 591.4, found 590.33.

**Example 10**

**Methyl Succinyl-N-cap form of Oligopeptide 38 [Methyl succinyl-β-Ala-Leu-Ala-Leu]**

Methyl succinyl-N-cap form of Oligopeptide 38 benzyl ester (1.0 g, 86% purity; 1.46 mmol) was added into an Erlenmeyer flask with 100 mL of methanol. The solution was cloudy after stirred for a few minutes. 50 mL of methanol was added, but the solution was still not clear. The solution was transferred into a hydrogenation reaction vessel. To this vessel, Pd-C (90 mg, 10% wet, 50% water; 0.042 mmol) was added. After hydrogenation for 2 hours at room temperature, the reaction was stopped and the catalyst was filtered. A white solid (0.77 g, 78%) was yielded after removal of solvents. MS *m/z* calcd. for $C_{23}H_{40}N_4O_8$ 501.2, found 500.3.

**Example 11**

**Synthesis of N-cap Allyl-Hemisuccinate**

This molecule was prepared according the procedure of Casimir, J. R., et.al. Tet. Lett. 36(19):3409, (1995). 10.07g (0.1 mol) succinic anhydride and 5.808g (0.1 mol) allyl-alcohol are refluxed in 100mL toluene for 6 hours. The reaction mixture is concentrated under reduced pressure. 15.5g; 98%. The resulting material was pure enough to use in subsequent reactions. The purity and identity of the semi-solid product was confirmed by $^1$HNMR and $^{13}$CNMR, by LC/MS.

**Example 12**

**Synthesis of Allyl-Succinyl-oligopeptide 38-Dox.**

In a round bottom flask (50 ml) N-Cap-Allylhemisuccinyl form of oligopeptide 38 (1g, 1.9 mmol) and doxorubicin (1.1g, 1.9 mmol) were dissolved in anhydrous DMF (50 ml). After the mixture was stirred for 5 minutes, DIEA (0.66 ml, 3.8 mmol) followed by HATU (0.76g, 1.9 mmol) was added into the solution the mixture was stirred at room temperature for 2 hours. DMF was removed by a rotary evaporator and the residue was taken up in 4.0 ml 1:1 DCM: MeOH. To this Solutions, 100 ml of ether was slowly added while stirring. A red precipitate was formed and collected by suction filtration.

The sold was washed with ether (2x2 ml) and dried in a vacuum desicator to give Allyl-Succinyl-oligopeptide 38-Dox therapeutic agent with 90% HPLC purity by Method B.

**Example 13**

**Preparation of SSLD from allyl-succinyl-β-Ala-Leu-Ala-Leu-doxorubicin**

To a stirred solution of 0.1g (0.095 mmol) allyl-succinyl-β-Ala-Leu-Ala-Leu-doxorubicin in 2 mL THF, under nitrogen atmosphere 0.05 g (0.095 mmol) tetrakis(triphenylphosphine) palladium is added as a solid. After 10 minutes the precipitate formed during the reaction is filtered off, washed with THF. Dry weight: 0.1g. The solids have been identified by HPLC, $^1$HNMR, LC/MS to be Succinyl-β-Ala-Leu-Ala-Leu-Dox.

**Example 14**

**Synthesis Fmoc form of Oligopeptide 38 [Fmoc-β-Ala-Leu-Ala-Leu]**

Fmoc form of Oligopeptide 38 was synthesized using solid-phase approach with standard Fmoc chemistry. A typical synthesis used Wang's alkoxy resin (0.60 mmol/gm loading). Fmoc-protected amino acids were used for solid-phase peptide synthesis. For a scale of 1mM peptide on resin, 3 equivalent of amino acid was preactivated with HBTU as the activating agent for 5 minutes before being added to the resin together with 2 equivalent of DIEA. The coupling reaction was carried out for 2 h and then washed with DMF (25 mL x 3) and DCM (25 mL x 3). The coupling reaction was repeated using 2 equivalent of amino acid using similar conditions. The reaction progress was monitored using ninhydrin test and if the ninhydrin test indicated incomplete reaction after 2 h then the coupling step was repeated for a third time. Deprotection was accomplished using 20% piperidine in DMF for 15-20 minutes. The coupling step was repeated with the next amino acid until the desired peptide was assembled on resin. The final cleavage of peptide from the resin was accomplished by treating the resin with a solution of 95%TFA and 5% water. After stirring the reaction mixture for 2h at rt, the resin was filtered under reduced pressure and washed twice with TFA. Filtrates were combined and the peptide was precipitated by adding 400 mL of cold ether. The peptide was filtered under reduced pressure and dried to yield Fmoc form of Oligopeptide 38 (94% HPLC purity by method A). Crude peptide was used for the next step without any further purification.

**Example 15**

**Synthesis of Fmoc form of Oligopeptide 98 [Fmoc-Thi-Tyr-Gly-Leu]**

Fmoc form of Oligopeptide 98 was synthesized using solid-phase approach with standard Fmoc chemistry and Wang's alkoxy resin (0.60 mmol/gm loading). Fmoc-protected amino acids and Fmoc-Thi-OH were used for solid-phase peptide synthesis. For a scale of 1mM peptide on resin, 3 equivalent of amino acid was preactivated with HBTU as the activating agent for 5 minutes before being added to the resin together with 2 equivalent of DIEA. The coupling reaction was carried out for 2 h and then washed with DMF (25 mL x 3) and DCM (25 mL x 3). The coupling reaction was repeated using 2 equivalent of amino acid using similar conditions. The reaction progress was monitored using ninhydrin test and if the ninhydrin test indicated incomplete reaction after 2 h then the coupling step was repeated for a third time. Deprotection was accomplished using 20% piperidine in DMF for 15-20 minutes. The coupling step was repeated with the next amino acid until the desired peptide was assembled on resin. The final cleavage of peptide from the resin was accomplished by treating the resin with a solution of 95%TFA and 5% water. After stirring the reaction mixture for 2h at rt, the resin was filtered under reduced pressure and washed twice with TFA. Filtrates were combined and adding 400 mL of cold ether precipitated the peptide. The peptide was filtered under reduced pressure and dried to yield Fmoc form of Oligopeptide 98 (88% HPLC purity by method A). Crude Fmoc form of Oligopeptide 98 was used for the next step without any further purification.

**Example 16**

**Synthesis of Fmoc form of Oligopeptide 38-Dnr Therapeutic Agent [Fmoc-β-Ala-Leu-Ala-Leu-Dnr]**

Daunorubicin.HCl (185 mg, 0.329 mmol) and Fmoc form of Oligopeptide 38 (200 mg, 0.329mmol) were dissolved at room temperature in anhydrous DMF (15 mL). To this rapidly stirred solution, DIEA (0.115 mL, 0.658 mmol) was added in one portion and the reaction mixture was stirred for 15 minutes at room temperature. The reaction mixture was cooled to 0°C using ice bath and 138 mg (0.362 mmol) of HATU was added slowly over 10 minutes. The reaction mixture was stirred for another 90 minutes at room temperature. Ice cold water (200 mL) was added to the reaction mixture which resulted in the formation of a red precipitate. The precipitate was collected over a course frit, washed with 3 x 50 mL water and 3 x 50 diethyl ether and dried under reduced pressure to yield Fmoc form of Oligopeptide 38-Dnr Therapeutic Agent (94% yield, 95% HPLC purity by method A). This product was used for the next step without any further purification.

**Example 17**

**Synthesis of Fmoc form of Oligopeptide 98-Daunorubicin Therapeutic Agent (Fmoc-Thi-Tyr-Gly-Leu-Dnr)**

Daunorubicin.HCl (90 mg, 0.16 mmol) and Fmoc form of Oligopeptide 98 (120 mg, 0.16 mmol) were dissolved at room temperature in anhydrous DMF (15 mL). To this rapidly stirred solution, DIEA (0.56 mL, 0.16 mmol) was added in one portion and the reaction mixture was stirred for 15 minutes at room temperature. The reaction mixture was cooled to 0°C using ice bath and 61 mg (0.16 mmol) of HATU was added slowly over 10 minutes. The reaction mixture was stirred for another 90 minutes at room temperature. Ice cold water (150 mL) was added to the reaction mixture which resulted in the formation of a red precipitate. The precipitate was collected over a course frit, washed with 3 x 50 mL water and 3 x 50 diethyl ether and dried under reduced pressure to yield Fmoc form of Oligopeptide 98-Daunorubicin Therapeutic Agent (94% yield, 91% HPLC purity by method A). This product was used for the next step without any further purification.

**Example 18**

**Preparation of Fmoc-β-Ala-Leu-Ala-Leu-doxorubicin**

3.0g (5.17 mmol) doxorubicin hydrochloride and 3.15 g (5.17 mmol) Fmoc-βAla-Leu-Ala-Leu was dissolved at room temperature in 230 mL dry DMF under nitrogen. To this rapidly stirred solution, 1.798 mL (10.34 mmol) DIEA was added in one portion and the reaction mixture stirred at room temperature for 15 min. The reaction mixture was cooled to ~ -2° C in an ice/brine bath and 2.56 g (6.73 mmol) HATU in 58 mL DMF was added dropwise over 12 minutes with rapid stirring. The reaction mixture was stirred another 30 minutes at -2° C then 0.285 mL (1.64 mmol) DIEA was added in one portion. 580 mL water at 0° C was immediately resulting in formation of a floculent red precipitate. The precipitate collected over a coarse glass frit, washed with 3 X 50 mL water and 3 x 50 mL diethyl ether in water and air dried 16 hours to yield 5.21 g Fmoc-β-Ala-Leu-Ala-Leu-Dox, 89.7% physical yield, 90.23% HPLC purity by Method B.

**Example 19**

**Preparation of succinyl-β-Ala-Leu-Ala-Leu-Dox from Fmoc-β-Ala-Leu-Ala-Leu-dox**

To a solution of 5.0 g (4.41 mmol) Fmoc-β-Ala-Leu-Ala-Leu in 230 mL dry DMF under nitrogen at room temperature, 21.8 mL (220 mmol) piperidine was added in one portion resulting in a color change from red to purple. The reaction mixture was stirred 5 minutes at room temperature then cooled to *ca.* -20°C in a dry ice/acetone bath. 22.5 g (0.225 mol) succinic anhydride was then added in one portion with the reaction temperature maintained below -5° C. After *ca.* 2 minutes stirring at -10°C to -5°C the color changed from purple to red/orange. The cooling bath was removed and the reaction mixture stirred for 10 minutes. The reaction mixture volume was then reduced to *ca.* 100 mL by rotary evaporation and then diluted with 125 mL chloroform. To this solution, 1400 mL diethyl ether was quickly added resulting in formation of a red precipitate. This precipitate was isolated on a medium glass frit and triturated with 5 X 200 mL diethyl ether to yield material of 89.13% HPLC purity. The precipitate was washed again with 1 x 20 mL diethyl ether and air dried to yield 3.62 g succinyl-β-Ala-Leu-Ala-Leu-Dox 81 % physical yield, 88.2% HPLC purity). This material was stirred in 30 mL water at 0°C and 33.98 mL (0.95 eq.) 0.1 M aq. NaHCO$_3$ was added and the resulting suspension stirred until all solids had dissolved. This solution was lyophilized to yield 3.77 g succinyl-β-Ala-Leu-Ala-Leu-Dox, 99% physical yield (89.06% HPLC purity by Method B).

**Example 20**

**Synthesis of N-cap Succinyl form of Oligopeptide 38-Dnr-therapeutic agent [Suc-β-Ala-Leu-Ala-Leu-Dnr]**

Piperidine (0.442 mL, 4.48 mmol) was added to a solution of Fmoc form of Oligopeptide 38-Dnr therapeutic agent (100 mg, 0.089 mmol) in 5 mL of dry DMF. The reaction mixture was stirred for 5 minutes at room temperature and then cooled to -20 °C using dry ice/ acetone bath. Succinic anhydride (458 mg, 4.54 mmol) was added then to the cooled reaction mixture in one portion. The reaction was stirred rapidly at -5°C for 5 minutes then at room temperature for another 90 minutes. Anhydrous diethyl ether, 250 mL, was added to the reaction mixture and the resulting red precipitate was isolated on a medium glass frit. The filter cake was washed with two successive 50 mL portions of diethyl ether and dried under reduced pressure to yield N-cap succinyl form of Oligopeptide 38-Dnr-therapeutic agent (80% yield, 88% HPLC purity by method B). The LC/MS gave a molecular weight of 995 (expected molecular weight 996).

## Example 21

**Synthesis of N-cap Succinyl form of Oligopeptide 98-Daunorubicin Therapeutic Agent [Suc-Thi-Tyr-Gly-Leu-Dnr]**

To a solution of Fmoc form of Oligopeptide 98-Daunorubicin Therapeutic Agent (100 mg, 0.079 mmol) in 5 mL of dry DMF, piperidine (0.391 mL, 3.95 mmol) was added in one portion resulting in a color change from red to purple. The reaction mixture was stirred for 5 minutes at room temperature and then cooled to -20° C using dry ice/ acetone bath. 407 mg (4.02 mmol) of succinic anhydride was then added to the cooled reaction mixture in one portion. The reaction was stirred rapidly at -5°C for 5 minutes then at room temperature for another 90 minutes. Anhydrous diethyl ether, 200 mL, was added to the reaction mixture which resulted in the formation of a red precipitate. This precipitate was isolated on a medium glass frit, washed with 3 x 50 mL of diethyl ether and dried under reduced pressure to yield N-cap succinyl form of Oligopeptide 98-Dnr Therapeutic Agent (80% yield, 81% HPLC purity by method A). The LC/MS gave a molecular weight of 1141 (expected molecular weight 1142).

## Example 22

**Synthesis of Sodium Salt of N-cap Glutaryl form of Oligopeptide 38-Dox therapeutic agent [Gl-β-Ala-Leu-Ala-Leu-Dox**

Piperidine (436 μL, 4.413 mmol) was added to a solution of Fmoc form of Oligopeptide 38-Dox therapeutic agent (100 mg, 0.088 mmol) in DMF (4.5 mL). After stirring for 5 minutes at room temperature, the reaction mixture was cooled to -5 °C and glutaric anhydride (624 mg, 5.472 mmol) was quickly added. The cold bath was removed as soon as the color changed and the mixture was stirred at room temperature for another 10 min. The DMF was removed by rotary evaporation and the residue dissolved in chloroform (2.5 mL). Diethyl ether (14 mL) was added and the resulting precipitate filtered. The filter cake was washed with diethyl ether, air dried and then resuspended in water (14 mL). The sodium salt was formed by addition of 0.025 M NaOH (4 mL, 0.10 mmol) dropwise to the suspension until complete dissolution of the solid. This solution was then lyophilized to give the sodium salt of glutaryl N-cap form of Oligopeptide 38-Dox therapeutic agent in 97% yield with an HPLC purity of 87% by method D.

## Example 23

**"Urea method" for preparing the conjugate. i.e. Precursor for Enzyme Route Coupling of Methyl Succinyl-N-cap form of Oligopeptide 38 and Doxorubicin**

Under dry nitrogen atmosphere 26.04g (52.0 mmol) methyl succinyl-N-cap form of Oligopeptide 38, 23.26g (40.2mmol) doxorubicin hydrochloride was suspended/dissolved in 800 mL dry, urea-saturated (~30% w/v) DMF and 14.8 19.948mL. 114.16 mmol DIEA. This mixture was cooled to 0-3°C over ~25 minutes. At this point 21.2 g (56.0 mmol) HATU was added as a solution in ~100mL urea saturated DMF over 10 minutes (the volume of this solution should be kept minimal). The reaction mixture was stirred for 10 minutes at -2 to 2°C. and poured into 4000 mL ice cold brine, containing 2%v/v acetic acid over approximately five minutes with vigorous stirring. The product was filtered off on a medium porosity fritted glass filter, washed generously with water and dried under reduced pressure. 43 g physical yield: 104.47%, 93.45% pure by HPLC method B.

## Example 24

**Synthesis of Methyl Succinyl-N-Cap form of Oligopeptide 38-Dox therapeutic agent [Methyl Succinyl-βAla-Leu-Ala-Leu-Dox]**

In a round bottom flask (50 mL), N-cap methyl hemisuccinyl form of Oligopeptide 38 (0.25 g, 0.5 mmol) and doxorubicin (0.29 g, 0.5 mmol) were dissolved in anhydrous DMF (20 mL). After the mixture was stirred for 5 minutes, DIEA (0.17 mL, 1.0 mmol) followed by HBTU (0.19g, 0.5 mmol) was added into the solution. The mixture was stirred at room temperature for 4 hrs. DMF was removed by a rotary evaporator and the residue was taken up in 4.0 mL 1:1

23

methylenechloride: methanol. To this solution, 40ml of ether was slowly added white stirring. A red precipitate was formed and collected by suction filtration. The solid was washed with ether (2x10 mL) and dried in a vacuum desiccator. 0.50 g of product (98%) was produced with purity of 96%.

**Example 25**

**Hydrolysis of the Methyl Succinyl-N-cap form of Oligopeptide 38-Dox therapeutic agent via Use of Cross Linked Enzyme**

Methyl succinyl-N-cap form of Oligopeptide 38-Dox therapeutic agent (1.0 g, 0.975 mmol) and 100 mL DMF are placed in a 500 mL flask. The suspension was vigorously agitated with a magnetic stirrer. When the methyl succinyl-N-cap form of Oligopeptide 38-Dox therapeutic agent had completely dissolved, 400 mL deionized water was added and the resulting solution stirred at 35°C. A slurry of 1 g washed CLEC-PC (Altus Biologics) the immobilized enzyme was rinsed in three aliquots of deionized water then resuspended in 10 mL 20 % aqueous DMF prior to use.) suspended in 10 mL of 20% aqueous DMF was then added and the resulting suspension was stirred at 35°C with periodic HPLC monitoring. When all of the methyl succinyl-N-cap form of Oligopeptide 38-Dox therapeutic agent had been consumed (~18 hours), the reaction mixture was filtered through a 0.45µM nylon membrane filter to remove the CLEC-PC enzyme. The CLEC-PC cake was washed with 3 X 10 mL methanol and the methanol washes were combined with the filtered reaction mixture. The filtered reaction mixture plus methanol washes were then concentrated to a red gum on a rotary evaporator equipped with a high vacuum pump and a 30° C water bath. The red gum was then suspended in 50 mL deionized water at room temperature and rapidly stirred via mechanical stirrer. To this suspension a solution of 77.8 mg sodium bicarbonate (0.926 mmol, 0.95 eq.) in 100 mL deionized water was added over 2 minutes. The suspension was stirred at room temperature 20 minutes. The reaction mixture was filtered through a 0.45 µM nylon membrane filter and lyophilized. 0.936 g sodium salt of succinyl-N-cap form of Oligopeptide 38-Dox therapeutic agent was isolated, about 100%yield. 84 % pure HPLC method B. $^1$H and $^{13}$C NMR spectra were recorded on 600 and 150 MHz spectrometers respectively and the electrospray MS, were consistent with the desired structure.

**Example 26**

**Hydrolysis of the methyl succinyl-N-cap form of Oligopeptide 38-Dox therapeutic agent [Methyl Succinyl-β-Ala-Leu-Ala-Leu-dox]**

**Use of soluble enzyme**

11.0 g (10.72 mmol) methyl succinyl-N-cap form of Oligopeptide 38-Dox therapeutic agent was suspended in 800 mL HPLC-grade water and homogenized for 60 minutes with an Ultraturrax T8 homogenizer to yield a finely divided suspension. This suspension was stirred (500 rpm) at 35° C and adjusted to pH=6.05 with aq. 76 mM NaHCO$_3$. 1.0 g C. Antarctica "B" lipase (Altus Biologics) was then added and the reaction mixture stirred at 35° C for 48 hours. During the 48 hr reaction time, pH was maintained between 5.3 and 6.2 by periodic addition of 76 mM NaHCO$_3$ and the reaction was periodically monitored by HPLC. After 48 hours, the reaction was ca. 98% complete by HPLC. The reaction mixture was then adjusted to pH=7 with aq. 76 mM NaHCO$_3$ and filtered through a pad of Celite 521. The clarified reaction mixture was then acidified to ca. pH 3 with 5 mL glacial acetic acid resulting in the formation of a gummy red precipitate. The precipitate was isolated by Celite 521 filtration, subsequent rinsing of the Celite pad with methanol, filtration of the methanol solution through a 10-20 µM fritted glass filter and rotary evaporation of the filtered solution to yield 7.31 g of gummy red product. This product was converted to the sodium salt by dissolution in 70 mL 76 mM NaHCO$_3$ (0.95 eq.) and lyophilized to yield 7.30 g, 66.1% physical yield sodium salt of succinyl-N-cap form of Oligopeptide 38-Dox therapeutic agent, 84.5% pure by HPLC.

The product was identical the example above.

**Example 27**

**Immobilized Candida Antarctica "B" Lipase Hydrolysis Methyl Succinyl-N-cap form of Oligopeptide 38-Dox therapeutic agent**

30.0g *Candida Antarctica* "B" lipase (Altus Biologics) was dissolved in 300 mL water and dialyzed against 3 x 4l of 50 mM aq. NaHCO$_3$ (pH=6.4). After dialysis, the volume of the dialyzed solution was ~300 mL. 360 mL of Pharmacia NHS-Activated Sepharose 4 Fast Flow was placed in a coarse glass fritted funnel and rinsed with 5 x 450 mL ice-cold 1 mM aq. HCl. The rinsed NHS-Activated Sepharose was then combined with the dialyzed enzyme solution. The resulting suspension was stirred at ambient temperature (*ca.* 22° C) for 2.0 hours. The sepharose/enzyme conjugate was then isolated on a coarse fritted glass filter and then stirred in 1000 mL 100 mM aq. TRIS (pH=7.45) for 15 minutes. This suspension was filtered and incubated with another 1000mL 100 mM aqueous TRIS buffer (pH=7.45) at 4°C, overnight. The immobilized enzyme in the morning was filtered off and after washing with water, was placed into a 2000mL three necked, round bottomed flask. 43g methyl succinyl-N-cap form of Oligopeptide 38-Dox therapeutic agent was added and the solids were suspended in 800mL deionized water. The flask was fitted with an overhead stirrer, and a pH-stat set to keep the pH of the reaction mixture between 5.9-6.2 by controlling a syringe pump. The syringe pump was charged 0.1 M $NaHCO_3$. Progress of the reaction was followed by HPLC. After 6 days the immobilized enzyme was filtered off and the liquid phase was lyophilized. The dry solids were then suspended in ~11mL dry THF and filtered off. 42.66g, 98.34% physical yield, 93.43%(254nm), 94.43%(480nm) pure by HPLC by method B.

**Example 28**

**Synthesis of the Lactate Salt of Oligopeptide 38-Dox Therapeutic Agent [β-Ala-Leu-Ala-Leu-Dox Lactate]**

Piperidine (26 mL, 264 mmol) was added to a solution of Fmoc form of Oligopeptide 38-Dox therapeutic agent (6.00 g, 5.3 mmol) in DMF (265 mL). After stirring for 5 minutes at room temperature, the reaction mixture was placed in an ice-salt bath, and precooled (4°C) 10% lactate buffer pH 3 (600 mL) was immediately added. The aqueous solution was extracted with DCM (3x500 mL) and excess salts were removed by solid phase extraction. C18 ODS-A silica gel (120 g) was conditioned (500 mL methanol, 2x500mL water) in a glass frit and loaded with the aqueous solution of crude product lactate salt. After washing with water (2x500 mL) and drying, the filter cake was dissolved in methanol. The methanol was evaporated and the residue was dissolved in water. The resulting solution was lyophilized to give 3.54 g of lactate salt of Oligopeptide 38-Dox therapeutic agent (67% yield, HPLC purity method B: 89%).

**Example 29**

**Synthesis of Succinyl-N-Cap Form of Oligopeptide 38-Dox Therapeutic Agent [succinyl-β-Ala-Leu-Ala-Leu-Dox] Starting From Lactate Salt of Oligopeptide 38-Dox Therapeutic Agent [β-Ala-Leu-Ala-Leu-Dox lactate].**

DIEA (417 μL, 2.40 mmol) was added to a solution of Lactate salt of Oligopeptide 38-Dox therapeutic agent (1.200 g, 1,20 mmol) in DMF (35 mL). After stirring for 15 minutes at room temperature, succinic anhydride 97% (2) (0.144 g, 1.44 mmol) was added. The mixture was stirred for 2 h, and DMF was removed by rotary evaporation. The residue was dissolved in a mixture of $CHCl_3/CH_3OH$ 4/1 (6 mL), and 200 mL of a mixture of $Et_2O$/hexane 1/1 were added. After the mixture was stirred for 30 minutes, the precipitate was filtered on quantitative paper (Whatman 42), washed ($Et_2O$/hexane: 1/1) and air-dried. The filter cake was suspended in water. (150 mL), and 1M NaOH ($\pm$ 1.2 eq., 1.5 mL) was added dropwise until complete dissolution (pH=7.2). The solution was lyophilized to give 1.218 g of succinyl-N-cap form of Oligopeptide 38-Dox therapeutic agent (97% yield; HPLC purity method B: 80.2%.

**Example 30**

**Synthesis of Succinyl-N-Cap Form of Oligopeptide 38-Dox Therapeutic Agent (succinyl-β-Ala-Leu-Ala-Leu-Dox) starting with Fmoc Form of Oligopeptide 38-Dox Therapeutic Agent [Fmoc-β-Ala-Leu-Ala-Leu-Dox]**

Piperidine (2180 μL, 22.06 mmol) was added to a solution of Fmoc form of Oligopeptide 38-Dox therapeutic agent (0.50 g, 0.44 mmol) in DMF (21.5 mL). After stirring for 5 minutes at room temperature, the reaction mixture was quickly cooled to -5°C and succinic anhydride (2.25 g, 22.51 mmol) was added immediately. The cold bath was removed as soon as the color changed and the mixture was stirred at room temperature for 10 minutes. The DMF was removed by rotary evaporation and the residue was dissolved in chloroform (12.5 mL). Diethylether (360 mL) was quickly added. A precipitate immediately appeared. The precipitate was filtered on Whatman 42 paper and washed with $Et_2O$. The solid was suspended in water. (120 mL; pH=4.1) and 0.025M NaOH (20 mL, 0.53 mmol) was added dropwise until complete dissolution (pH=7.4). This solution was then lyophilized to give succinyl-N-cap form of Oligopeptide 38-Dox therapeutic agent in 89% yield and 91% HPLC purity by Method D.

The invention as claimed therefore relates to a prodrug compound, the compound comprising:

(1) a therapeutic agent capable of entering a target cell,
(2) an oligopeptide having a formula $(AA)_n-AA^4-AA^3-AA^2-AA^1$, wherein:

each AA independently represents any genetically encoded amino acid,

n is an integer from 0 to 12,
$AA^4$ represents a non-genetically-encoded amino acid,
$AA^3$ represents any amino acid,
$AA^2$ represents any amino acid, and
$AA^1$ represents any amino acid,

(3) a stabilizing group that hinders cleavage of said oligopeptide by enzymes present in whole blood, and
(4) optionally, a linker group not cleavable by trouase,

wherein the oligopeptide is directly linked to the stabilizing group at a first attachment site of the oligopeptide and $AA^1$ of the oligopeptide is directly linked to the therapeutic agent or indirectly linked through the linker group to the therapeutic agent at a second attachment site of the oligopeptide;
the compound being selectively cleaved by an enzyme associated with the target cell.

The invention as claimed further relates to a method for decreasing toxicity of a therapeutic agent wherein the therapeutic agent is intended for administration to a patient, the method comprising:

covalently forming a prodrug by linking an oligopeptide cleanable by trouase to a stabilizing group at a first attachment site of the oligopeptide and directly or indirectly
linking the therapeutic agent at a second attachment site of the oligopeptide, the prodrug being selectively cleaved by trouase, whereby the prodrug provides for decreased toxicity of the therapeutic agent when administrated to the patient.

The disclosure includes a method of making a prodrug compound agent comprising the following steps:

(1) activating an Fmoc-protected oligopeptide with an activating agent in the presence of a therapeutic agent to make a Fmoc-protected oligopeptide therapeutic agent conjugate,
(2) deprotecting the Fmoc-protected oligopeptide therapeutic agent by contacting it with a base,
(3) reacting the oligopeptide therapeutic agent with a stabilizing group,
(4) neutralizing the stabilizing group-oligopeptide therapeutic agent conjugate with a pharmaceutically acceptable salt.

The disclosure also includes making a prodrug compound comprising the following steps:

(1) activating an alkyl ester-protected-stabilizing group oligopeptide with an activating agent in the presence of a therapeutic agent to make an alkylester-protected stabilizing group oligopeptide therapeutic agent conjugate,
(2) deprotecting the alkyl ester-protected stabilizing group oligopeptide therapeutic agent, and
(3) neutralizing the stabilizing group-oligopeptide therapeutic agent with a pharmaceutically acceptable salt.

Another aspect of the disclosure is a method of making a prodrug compound comprising the following steps:

(1) activating a trityl-protected oligopeptide with an activating agent in the presence of a therapeutic agent to make a trityl-protected oligopeptide therapeutic agent conjugate,
(2) deprotecting the trityl-protected oligopeptide therapeutic agent conjugate under acidic conditions for 30-120 minutes at 0 to 25 °C,
(3) reacting the oligopeptide-therapeutic agent with a stabilizing group, and
(4) neutralizing the stabilizing group-oligopeptide-therapeutic agent with a pharmaceutically acceptable salt.

Compositions of the invention include prodrugs made by all of the methods above.

The invention now being fully described, it will be apparent to one of ordinary skill in the art that many changes and modifications can be made thereto without departing from the scope of the appended claims.

SEQUENCE LISTING

<110> Coulter Pharmaceutical, Inc.

<120> PRODRUG COMPOUNDS AND PROCESS FOR PREPARATION THEREOF

<130> COUL-007/01WO

<140> not yet available
<141> 1999-12-10

<150> 60/119,312
<151> 1999-02-08

<150> 60/111,793
<151> 1998-12-11

<160> 103

<170> PatentIn Ver. 2.1

<210> 1
<211> 7
<212> PRT
<213> Artificial Sequence

<220>
<223> Description of Artificial Sequence: Synthetic

<220>
<221> SITE
<222> (1)
<223> D-Alanine

<220>
<221> SITE
<222> (2)
<223> 2-Thienylalanine

<220>
<221> SITE
<222> (3)
<223> Beta-Alanine

<220>
<221> SITE
<222> (4)
<223> Beta-Alanine

<400> 1

```
Xaa Xaa Xaa Xaa Leu Ala Leu
1                   5
```

<210> 2
<211> 6
<212> PRT
<213> Artificial Sequence

<220>
<223> Description of Artificial Sequence: Synthetic

<220>

<221> SITE
<222> (1)
<223> 2-Thienylalanine

<220>
<221> SITE
<222> (2)
<223> Beta-Alanine

<220>
<221> SITE
<222> (3)
<223> Beta-Alanine

<400> 2

**Xaa Xaa Xaa Leu Ala Leu**
**1                      5**

<210> 3
<211> 5
<212> PRT
<213> Artificial Sequence

<220>
<223> Description of Artificial Sequence: Synthetic

<220>
<221> SITE
<222> (1)
<223> Beta-Alanine

<220>
<221> SITE
<222> (2)
<223> Beta-Alanine

<400> 3

**Xaa Xaa Leu Ala Leu**
**1                  5**

<210> 4
<211> 4
<212> PRT
<213> Artificial Sequence

<220>
<223> Description of Artificial Sequence: Synthetic

<220>
<221> SITE
<222> (1)
<223> Beta-Alanine

<400> 4

Xaa Ala Ala Ile
1

<210> 5
<211> 4
<212> PRT
<213> Artificial Sequence

<220>
<223> Description of Artificial Sequence: Synthetic

<220>
<221> SITE
<222> (1)
<223> Beta-Alanine

<400> 5

Xaa Ala Ala Leu
1

<210> 6
<211> 4
<212> PRT
<213> Artificial Sequence

<220>
<223> Description of Artificial Sequence: Synthetic

<220>
<221> SITE
<222> (1)
<223> Beta-Alanine

<400> 6

Xaa Phe Tyr Leu
1

<210> 7
<211> 4
<212> PRT
<213> Artificial Sequence

<220>
<223> Description of Artificial Sequence: Synthetic

<220>
<221> SITE
<222> (1)
<223> Beta-Alanine

<400> 7

Xaa Phe Thr Phe
1

<210> 8
<211> 4
<212> PRT
<213> Artificial Sequence

<220>
<223> Description of Artificial Sequence: Synthetic

<220>
<221> SITE
<222> (1)
<223> Beta-Alanine

<400> 8

Xaa Phe Gly Ile

1

<210> 9
<211> 4
<212> PRT
<213> Artificial Sequence

<220>
<223> Description of Artificial Sequence: Synthetic

<220>
<221> SITE
<222> (1)
<223> Beta-Alanine

<400> 9

Xaa Phe Gly Leu
1

<210> 10
<211> 4
<212> PRT
<213> Artificial Sequence

<220>
<223> Description of Artificial Sequence: Synthetic

<220>

<221> SITE
<222> (1)
<223> Beta-Alanine

<400> 10

                              Xaa Phe Phe Phe
                               1

<210> 11
<211> 4
<212> PRT
<213> Artificial Sequence

<220>
<223> Description of Artificial Sequence: Synthetic

<220>
<221> SITE
<222> (1)
<223> Beta-Alanine

<400> 11

                              Xaa Phe Phe Ile
                               1

<210> 12
<211> 4
<212> PRT
<213> Artificial Sequence

<220>
<223> Description of Artificial Sequence: Synthetic

<220>
<221> SITE
<222> (1)
<223> Beta-Alanine

<400> 12

                              Xaa Phe Phe Leu
                               1

<210> 13
<211> 4
<212> PRT
<213> Artificial Sequence

<220>
<223> Description of Artificial Sequence: Synthetic

<220>

<221> SITE
<222> (1)
<223> Beta-Alanine

<400> 13

```
                              Xaa Phe Ala Ile
                               1
```

<210> 14
<211> 4
<212> PRT
<213> Artificial Sequence

<220>
<223> Description of Artificial Sequence: Synthetic

<220>
<221> SITE
<222> (1)
<223> Beta-Alanine

<400> 14

```
                              Xaa Phe Ala Leu
                               1
```

<210> 15
<211> 4
<212> PRT
<213> Artificial Sequence

<220>
<223> Description of Artificial Sequence: Synthetic

<220>
<221> SITE
<222> (1)
<223> 2-Thienylalanine

<400> 15

```
                              Xaa Gly Ala Leu
                               1
```

<210> 16
<211> 4
<212> PRT
<213> Artificial Sequence

<220>
<223> Description of Artificial Sequence: Synthetic

<220>

<221> SITE
<222> (1)
<223> 2-Naphthylalanine

<400> 16

Xaa Gly Ala Leu

1

<210> 17
<211> 4
<212> PRT
<213> Artificial Sequence

<220>
<223> Description of Artificial Sequence: Synthetic

<220>
<221> SITE
<222> (1)
<223> Beta-Alanine

<400> 17

Xaa Leu Tyr Leu
1

<210> 18
<211> 4
<212> PRT
<213> Artificial Sequence

<220>
<223> Description of Artificial Sequence: Synthetic

<220>
<221> SITE
<222> (1)
<223> Beta-Alanine

<220>
<221> SITE
<222> (3)
<223> 2-Thienylalanine

<400> 18

Xaa Leu Xaa Leu
1

<210> 19
<211> 4
<212> PRT
<213> Artificial Sequence

<220>
<223> Description of Artificial Sequence: Synthetic

<220>
<221> SITE
<222> (1)
<223> Beta-Alanine

<400> 19

```
                        Xaa Leu Thr Phe
                         1
```

<210> 20
<211> 4
<212> PRT
<213> Artificial Sequence

<220>
<223> Description of Artificial Sequence: Synthetic

<220>
<221> SITE
<222> (1)
<223> Beta-Alanine

<400> 20

```
                        Xaa Leu Thr Ile
                         1
```

<210> 21
<211> 4
<212> PRT
<213> Artificial Sequence

<220>
<223> Description of Artificial Sequence: Synthetic

<220>
<221> SITE
<222> (1)
<223> Beta-Alanine

<400> 21

```
                        Xaa Leu Thr Leu
                         1
```

<210> 22
<211> 4
<212> PRT
<213> Artificial Sequence

<220>
<223> Description of Artificial Sequence: Synthetic

<220>
<221> SITE
<222> (1)
<223> Beta-Alanine

<400> 22

```
                    Xaa Leu Ser Leu
                     1
```

<210> 23
<211> 4
<212> PRT
<213> Artificial Sequence

<220>
<223> Description of Artificial Sequence: Synthetic

<220>
<221> SITE
<222> (1)
<223> Beta-Alanine

<220>
<221> SITE
<222> (3)
<223> 3-Pyridylalanine

<400> 23

```
                    Xaa Leu Xaa Leu
                     1
```

<210> 24
<211> 4
<212> PRT
<213> Artificial Sequence

<220>
<223> Description of Artificial Sequence: Synthetic

<220>
<221> SITE
<222> (1)
<223> Beta-Alanine

<400> 24

                                        Xaa Leu Leu Leu
                                              1


<210> 25
<211> 4
<212> PRT
<213> Artificial Sequence

<220>
<223> Description of Artificial Sequence: Synthetic

<220>
<221> SITE
<222> (1)
<223> Beta-Alanine

<400> 25


                                        Xaa Leu Gly Phe
                                              1


<210> 26
<211> 4
<212> PRT
<213> Artificial Sequence

<220>
<223> Description of Artificial Sequence: Synthetic

<220>
<221> SITE
<222> (1)
<223> Beta-Alanine

<400> 26


                                        Xaa Leu Gly Ile
                                              1


<210> 27
<211> 4
<212> PRT
<213> Artificial Sequence

<220>
<223> Description of Artificial Sequence: Synthetic

<220>
<221> SITE
<222> (1)

<223> 2-Thienylalanine

<400> 27

```
                              Xaa Leu Gly Leu
                               1
```

<210> 28
<211> 4
<212> PRT
<213> Artificial Sequence

<220>
<223> Description of Artificial Sequence: Synthetic

<220>
<221> SITE
<222> (1)
<223> Beta-Alanine

<400> 28

```
                              Xaa Leu Gly Leu
                               1
```

<210> 29
<211> 4
<212> PRT
<213> Artificial Sequence

<220>
<223> Description of Artificial Sequence: Synthetic

<220>
<221> SITE
<222> (1)
<223> Aminoisobutyric acid

<400> 29

```
                              Xaa Leu Gly Leu
                               1
```

<210> 30
<211> 4
<212> PRT
<213> Artificial Sequence

<220>
<223> Description of Artificial Sequence: Synthetic

<220>
<221> SITE

<222> (1)
<223> Beta-Alanine

<400> 30

Xaa Leu Phe Ile
1

<210> 31
<211> 4
<212> PRT
<213> Artificial Sequence

<220>
<223> Description of Artificial Sequence: Synthetic

<220>
<221> SITE
<222> (1)
<223> Beta-Alanine

<400> 31

Xaa Leu Phe Leu
1

<210> 32
<211> 4
<212> PRT
<213> Artificial Sequence

<220>
<223> Description of Artificial Sequence: Synthetic

<220>
<221> SITE
<222> (1)
<223> Beta-Alanine

<220>
<221> SITE
<222> (3)
<223> Aminoisobutyric acid

<400> 32

Xaa Leu Xaa Leu
1

<210> 33
<211> 4
<212> PRT
<213> Artificial Sequence

<220>
<223> Description of Artificial Sequence: Synthetic

<220>
<221> SITE
<222> (1)
<223> Beta-Alanine

<400> 33

Xaa Leu Ala Ala
1

<210> 34
<211> 4
<212> PRT
<213> Artificial Sequence

<220>
<223> Description of Artificial Sequence: Synthetic

<220>
<221> SITE
<222> (1)
<223> Beta-Alanine

<220>
<221> SITE
<222> (4)
<223> Beta-Alanine

<400> 34

Xaa Leu Ala Xaa
1

<210> 35
<211> 4
<212> PRT
<213> Artificial Sequence

<220>
<223> Description of Artificial Sequence: Synthetic

<220>
<221> SITE
<222> (1)
<223> Beta-Alanine

<400> 35

Xaa Leu Ala Phe
1

<210> 36
<211> 4
<212> PRT
<213> Artificial Sequence

<220>
<223> Description of Artificial Sequence: Synthetic

<220>
<221> SITE
<222> (1)
<223> Beta-Alanine

<400> 36

```
                    Xaa Leu Ala Gly
                     1
```

<210> 37
<211> 4
<212> PRT
<213> Artificial Sequence

<220>
<223> Description of Artificial Sequence: Synthetic

<220>
<221> SITE
<222> (1)
<223> Beta-Alanine

<400> 37

```
                    Xaa Leu Ala Ile
                     1
```

<210> 38
<211> 4
<212> PRT
<213> Artificial Sequence

<220>
<223> Description of Artificial Sequence: Synthetic

<220>
<221> SITE
<222> (1)
<223> Beta-Alanine

<400> 38

```
                    Xaa Leu Ala Leu
                     1
```

<210> 39
<211> 4
<212> PRT
<213> Artificial Sequence

<220>
<223> Description of Artificial Sequence: Synthetic

<220>
<221> SITE
<222> (1)
<223> Tetrahydroisoquinoline-3-carboxylic acid

<400> 39

```
                            Xaa Leu Ala Leu
                             1
```

<210> 40
<211> 4
<212> PRT
<213> Artificial Sequence

<220>
<223> Description of Artificial Sequence: Synthetic

<220>
<221> SITE
<222> (1)
<223> Thiazolidine-4-carboxylic acid

<400> 40

```
                            Xaa Leu Ala Leu
                             1
```

<210> 41
<211> 4
<212> PRT
<213> Artificial Sequence

<220>
<223> Description of Artificial Sequence: Synthetic

<220>
<221> SITE
<222> (1)
<223> 2-Thienylalanine

<400> 41

```
                              Xaa Leu Ala Leu
                                    1
```

<210> 42
<211> 4
<212> PRT
<213> Artificial Sequence

<220>
<223> Description of Artificial Sequence: Synthetic

<220>
<221> SITE
<222> (1)
<223> 2-Naphthylalanine

<400> 42

```
                              Xaa Leu Ala Leu
                                    1
```

<210> 43
<211> 4
<212> PRT
<213> Artificial Sequence

<220>
<223> Description of Artificial Sequence: Synthetic

<220>
<221> SITE
<222> (1)
<223> 3-Amino-4,4-diphenylbutyric acid

<400> 43

```
                              Xaa Leu Ala Leu
                                    1
```

<210> 44
<211> 4
<212> PRT
<213> Artificial Sequence

<220>
<223> Description of Artificial Sequence: Synthetic

<220>
<221> SITE
<222> (1)
<223> D-Leucine

<400> 44

Xaa Leu Ala Leu
1

<210> 45
<211> 4
<212> PRT
<213> Artificial Sequence

<220>
<223> Description of Artificial Sequence: Synthetic

<220>
<221> SITE
<222> (1)
<223> D-Alanine

<400> 45

Xaa Leu Ala Leu
1

<210> 46
<211> 4
<212> PRT
<213> Artificial Sequence

<220>
<223> Description of Artificial Sequence: Synthetic

<220>
<221> SITE
<222> (1)
<223> D-Methionine

<400> 46

Xaa Leu Ala Leu
1

<210> 47
<211> 4
<212> PRT
<213> Artificial Sequence

<220>
<223> Description of Artificial Sequence: Synthetic

<220>
<221> SITE
<222> (1)
<223> 3-Amino-3-phenylpropionic acid

<400> 47

Xaa Leu Ala Leu
1

<210> 48
<211> 4
<212> PRT
<213> Artificial Sequence

<220>
<223> Description of Artificial Sequence: Synthetic

<220>
<221> SITE
<222> (1)
<223> 4-(Aminomethyl)benzoic acid

<400> 48

Xaa Leu Ala Leu
1

<210> 49
<211> 4
<212> PRT
<213> Artificial Sequence

<220>
<223> Description of Artificial Sequence: Synthetic

<220>
<221> SITE
<222> (1)
<223> Beta-Alanine

<220>
<221> SITE
<222> (4)
<223> 2-Naphthylalanine

<400> 49

Xaa Leu Ala Xaa
1

<210> 50
<211> 4
<212> PRT
<213> Artificial Sequence

<220>
<223> Description of Artificial Sequence: Synthetic

<220>

<221> SITE
<222> (1)
<223> Beta-Alanine

<400> 50

Xaa Leu Ala Ser
1

<210> 51
<211> 4
<212> PRT
<213> Artificial Sequence

<220>
<223> Description of Artificial Sequence: Synthetic

<220>
<221> SITE
<222> (1)
<223> Beta-Alanine

<400> 51

Xaa Leu Ala Tyr
1

<210> 52
<211> 4
<212> PRT
<213> Artificial Sequence

<220>
<223> Description of Artificial Sequence: Synthetic

<220>
<221> SITE
<222> (1)
<223> Beta-Alanine

<400> 52

Xaa Met Tyr Phe
1

<210> 53
<211> 4
<212> PRT
<213> Artificial Sequence

<220>

<223> Description of Artificial Sequence: Synthetic

<220>
<221> SITE
<222> (1)
<223> Beta-Alanine

<400> 53

```
Xaa Met Tyr Leu
 1
```

<210> 54
<211> 4
<212> PRT
<213> Artificial Sequence

<220>
<223> Description of Artificial Sequence: Synthetic

<220>
<221> SITE
<222> (1)
<223> Beta-Alanine

<400> 54

```
Xaa Met Gly Ile
 1
```

<210> 55
<211> 4
<212> PRT
<213> Artificial Sequence

<220>
<223> Description of Artificial Sequence: Synthetic

<220>
<221> SITE
<222> (1)
<223> 2-Thienylalanine

<400> 55

```
Xaa Met Gly Leu
 1
```

<210> 56
<211> 4
<212> PRT
<213> Artificial Sequence

<220>

<223> Description of Artificial Sequence: Synthetic

<220>
<221> SITE
<222> (1)
<223> Beta-Alanine

<400> 56

```
                                    Xaa Met Phe Phe
                                     1
```

<210> 57
<211> 4
<212> PRT
<213> Artificial Sequence

<220>
<223> Description of Artificial Sequence: Synthetic

<220>
<221> SITE
<222> (1)
<223> Beta-Alanine

<400> 57

```
                                    Xaa Met Phe Ile
                                     1
```

<210> 58
<211> 4
<212> PRT
<213> Artificial Sequence

<220>
<223> Description of Artificial Sequence: Synthetic

<220>
<221> SITE
<222> (1)
<223> Tetrahydroisoquinoline-3-carboxylic acid

<400> 58

```
                                    Xaa Met Ala Leu
                                     1
```

<210> 59
<211> 4
<212> PRT
<213> Artificial Sequence

<220>
<223> Description of Artificial Sequence: Synthetic

<220>
<221> SITE
<222> (1)
<223> 2-Naphthylalanine

<400> 59

```
                              Xaa Met Ala Leu
                               1
```

<210> 60
<211> 4
<212> PRT
<213> Artificial Sequence

<220>
<223> Description of Artificial Sequence: Synthetic

<220>
<221> SITE
<222> (1)
<223> 3-Amino-4,4-diphenylbutyric acid

<400> 60

```
                              Xaa Met Ala Leu
                               1
```

<210> 61
<211> 4
<212> PRT
<213> Artificial Sequence

<220>
<223> Description of Artificial Sequence: Synthetic

<220>
<221> SITE
<222> (1)
<223> Beta-Alanine

<400> 61

```
                              Xaa Met Ala Leu
                               1
```

<210> 62
<211> 4
<212> PRT
<213> Artificial Sequence

<220>
<223> Description of Artificial Sequence: Synthetic

<220>
<221> SITE
<222> (1)
<223> 3-Amino-3-phenylpropionic acid

<400> 62

$$\text{Xaa Met Ala Leu}$$
$$1$$

<210> 63
<211> 4
<212> PRT
<213> Artificial Sequence

<220>
<223> Description of Artificial Sequence: Synthetic

<220>
<221> SITE
<222> (1)
<223> Beta-Alanine

<220>
<221> SITE
<222> (2)
<223> Norleucine

<400> 63

$$\text{Xaa Xaa Tyr Ile}$$
$$1$$

<210> 64
<211> 4
<212> PRT
<213> Artificial Sequence

<220>
<223> Description of Artificial Sequence: Synthetic

<220>
<221> SITE
<222> (1)
<223> Beta-Alanine

<220>
<221> SITE
<222> (2)
<223> Norleucine

<400> 64

Xaa Xaa Tyr Leu
1

<210> 65
<211> 4
<212> PRT
<213> Artificial Sequence

<220>
<223> Description of Artificial Sequence: Synthetic

<220>
<221> SITE
<222> (1)
<223> Beta-Alanine

<220>
<221> SITE
<222> (2)
<223> Norleucine

<400> 65

Xaa Xaa Thr Ile
1

<210> 66
<211> 4
<212> PRT
<213> Artificial Sequence

<220>
<223> Description of Artificial Sequence: Synthetic

<220>
<221> SITE
<222> (1)
<223> Beta-Alanine

<220>
<221> SITE
<222> (2)
<223> Norleucine

<400> 66

Xaa Xaa Thr Leu
1

<210> 67
<211> 4

<212> PRT
<213> Artificial Sequence

<220>
<223> Description of Artificial Sequence: Synthetic

<220>
<221> SITE
<222> (1)
<223> Beta-Alanine

<220>
<221> SITE
<222> (2)
<223> Norleucine

<400> 67

```
                              Xaa Xaa Gly Phe
                               1
```

<210> 68
<211> 4
<212> PRT
<213> Artificial Sequence

<220>
<223> Description of Artificial Sequence: Synthetic

<220>
<221> SITE
<222> (1)
<223> Beta-Alanine

<220>
<221> SITE
<222> (2)
<223> Norleucine

<400> 68

```
                              Xaa Xaa Gly Ile
                               1
```

<210> 69
<211> 4
<212> PRT
<213> Artificial Sequence

<220>
<223> Description of Artificial Sequence: Synthetic

<220>
<221> SITE

<222> (1)
<223> Beta-Alanine

<220>
<221> SITE
<222> (2)
<223> Norleucine

<400> 69

**Xaa Xaa Gly Leu**
**1**

<210> 70
<211> 4
<212> PRT
<213> Artificial Sequence

<220>
<223> Description of Artificial Sequence: Synthetic

<220>
<221> SITE
<222> (1)
<223> Beta-Alanine

<220>
<221> SITE
<222> (2)
<223> Norleucine

<400> 70

**Xaa Xaa Phe Ile**
**1**

<210> 71
<211> 4
<212> PRT
<213> Artificial Sequence

<220>
<223> Description of Artificial Sequence: Synthetic

<220>
<221> SITE
<222> (1)
<223> Beta-Alanine

<220>
<221> SITE
<222> (2)
<223> Norleucine

<400> 71

Xaa Xaa Ala Ile
1

<210> 72
<211> 4
<212> PRT
<213> Artificial Sequence

<220>
<223> Description of Artificial Sequence: Synthetic

<220>
<221> SITE
<222> (1)
<223> Beta-Alanine

<220>
<221> SITE
<222> (2)
<223> Norleucine

<400> 72

Xaa Xaa Ala Leu
1

<210> 73
<211> 3
<212> PRT
<213> Artificial Sequence

<220>
<223> Description of Artificial Sequence: Synthetic

<220>
<221> SITE
<222> (1)
<223> Beta-Alanine

<220>
<221> SITE
<222> (2)
<223> Norleucine

<400> 73

Xaa Xaa Ala Phe
1

<210> 74

<211> 4
<212> PRT
<213> Artificial Sequence

<220>
<223> Description of Artificial Sequence: Synthetic

<220>
<221> SITE
<222> (1)
<223> Beta-Alanine

<220>
<221> SITE
<222> (2)
<223> Norvaline

<400> 74

```
                              Xaa Xaa Ala Leu
                               1
```

<210> 75
<211> 4
<212> PRT
<213> Artificial Sequence

<220>
<223> Description of Artificial Sequence: Synthetic

<220>
<221> SITE
<222> (1)
<223> Beta-Alanine

<400> 75

```
                              Xaa Phe Tyr Ile
                               1
```

<210> 76
<211> 4
<212> PRT
<213> Artificial Sequence

<220>
<223> Description of Artificial Sequence: Synthetic

<220>
<221> SITE
<222> (1)
<223> 2-Thienylalanine

<400> 76

```
                        Xaa Pro Gly Leu
                          1
```

<210> 77
<211> 4
<212> PRT
<213> Artificial Sequence

<220>
<223> Description of Artificial Sequence: Synthetic

<220>
<221> SITE
<222> (1)
<223> 2-Thienylalanine

<400> 77

```
                                Xaa Pro Ala Leu
                                  1
```

<210> 78
<211> 4
<212> PRT
<213> Artificial Sequence

<220>
<223> Description of Artificial Sequence: Synthetic

<220>
<221> SITE
<222> (1)
<223> 2-Naphthylalanine

<400> 78

```
                                Xaa Pro Ala Leu
                                  1
```

<210> 79
<211> 4
<212> PRT
<213> Artificial Sequence

<220>
<223> Description of Artificial Sequence: Synthetic

<220>
<221> SITE
<222> (1)
<223> Beta-Alanine

<400> 79

<div align="center">Xaa Pro Ala Leu</div>

<div align="center">1</div>

<210> 80
<211> 4
<212> PRT
<213> Artificial Sequence

<220>
<223> Description of Artificial Sequence: Synthetic

<220>
<221> SITE
<222> (1)
<223> Beta-Alanine

<220>
<221> SITE
<222> (2)
<223> Phe(C1)

<400> 80

<div align="center">Xaa Xaa Ala Leu</div>

<div align="center">1</div>

<210> 81
<211> 4
<212> PRT
<213> Artificial Sequence

<220>
<223> Description of Artificial Sequence: Synthetic

<220>
<221> SITE
<222> (1)
<223> Beta-Alanine

<220>
<221> SITE
<222> (2)
<223> Phe(N02)

<400> 81

<div align="center">Xaa Xaa Ala Ile</div>

<div align="center">1</div>

<210> 82

<211> 4
<212> PRT
<213> Artificial Sequence

<220>
<223> Description of Artificial Sequence: Synthetic

<220>
<221> SITE
<222> (1)
<223> Beta-Alanine

<220>
<221> SITE
<222> (2)
<223> Phe(N02)

<400> 82

```
                              Xaa Xaa Ala Leu
                               1
```

<210> 83
<211> 4
<212> PRT
<213> Artificial Sequence

<220>
<223> Description of Artificial Sequence: Synthetic

<220>
<221> SITE
<222> (1)
<223> Beta-Alanine

<220>
<221> SITE
<222> (2)
<223> Phenylglycine

<400> 83

```
                              Xaa Xaa Ala Leu
                               1
```

<210> 84
<211> 4
<212> PRT
<213> Artificial Sequence

<220>
<223> Description of Artificial Sequence: Synthetic

<220>
<221> SITE

<222> (1)
<223> Beta-Alanine

<220>
<221> SITE
<222> (2)
<223> 3-Pyridylalanine

<400> 84

```
                                    Xaa Xaa Ala Leu
                                         1
```

<210> 85
<211> 4
<212> PRT
<213> Artificial Sequence

<220>
<223> Description of Artificial Sequence: Synthetic

<220>
<221> SITE
<222> (1)
<223> Tetrahydroisoquinoline-3-carboxylic acid

<400> 85

```
                                    Xaa Thr Gly Leu
                                         1
```

<210> 86
<211> 4
<212> PRT
<213> Artificial Sequence

<220>
<223> Description of Artificial Sequence: Synthetic

<220>
<221> SITE
<222> (1)
<223> Beta-Alanine

<220>
<221> SITE
<222> (2)
<223> 2-Thienylalanine

<400> 86

```
Xaa Xaa Gly Ile
1
```

<210> 87
<211> 4
<212> PRT
<213> Artificial Sequence

<220>
<223> Description of Artificial Sequence: Synthetic

<220>
<221> SITE
<222> (1)
<223> Beta-Alanine

<220>
<221> SITE
<222> (2)
<223> 2-Thienylalanine

<400> 87

```
Xaa Xaa Ala Leu
1
```

<210> 88
<211> 4
<212> PRT
<213> Artificial Sequence

<220>
<223> Description of Artificial Sequence: Synthetic

<220>
<221> SITE
<222> (1)
<223> Beta-Alanine

<220>
<221> SITE
<222> (2)
<223> Tetrahydroisoquinoline-3-carboxylic acid

<400> 88

```
Xaa Xaa Ala Ile
1
```

<210> 89
<211> 4
<212> PRT
<213> Artificial Sequence

```
<220>
<223> Description of Artificial Sequence: Synthetic

<220>
<221> SITE
<222> (1)
<223> Beta-Alanine

<220>
<221> SITE
<222> (2)
<223> Tetrahydroisoquinoline

<400> 89
```

Xaa Xaa Ala Leu
1

```
<210> 90
<211> 4
<212> PRT
<213> Artificial Sequence

<220>
<223> Description of Artificial Sequence: Synthetic

<220>
<221> SITE
<222> (1)
<223> Beta-Alanine

<400> 90
```

Xaa Val Ala Leu
1

```
<210> 91
<211> 4
<212> PRT
<213> Artificial Sequence

<220>
<223> Description of Artificial Sequence: Synthetic

<220>
<221> SITE
<222> (1)
<223> Beta-Alanine

<400> 91
```

```
                                    Xaa Trp Ala Leu
                                         1
```

<210> 92
<211> 4
<212> PRT
<213> Artificial Sequence

<220>
<223> Description of Artificial Sequence: Synthetic

<220>
<221> SITE
<222> (1)
<223> Beta-Alanine

<400> 92

```
                                    Xaa Tyr Tyr Phe
                                         1
```

<210> 93
<211> 4
<212> PRT
<213> Artificial Sequence

<220>
<223> Description of Artificial Sequence: Synthetic

<220>
<221> SITE
<222> (1)
<223> Beta-Alanine

<400> 93

```
                                    Xaa Tyr Tyr Ile
                                         1
```

<210> 94
<211> 4
<212> PRT
<213> Artificial Sequence

<220>
<223> Description of Artificial Sequence: Synthetic

<220>

<221> SITE
<222> (1)
<223> Beta-Alanine

<400> 94

Xaa Tyr Tyr Leu
1

<210> 95
<211> 4
<212> PRT
<213> Artificial Sequence

<220>
<223> Description of Artificial Sequence: Synthetic

<220>
<221> SITE
<222> (1)
<223> Beta-Alanine

<400> 95

Xaa Tyr Thr Leu
1

<210> 96
<211> 4
<212> PRT
<213> Artificial Sequence

<220>
<223> Description of Artificial Sequence: Synthetic

<220>
<221> SITE
<222> (1)
<223> Beta-Alanine

<400> 96

Xaa Tyr Phe Leu
1

<210> 97
<211> 4
<212> PRT
<213> Artificial Sequence

<220>
<223> Description of Artificial Sequence: Synthetic

<220>
<221> SITE
<222> (1)

<223> Beta-Alanine

<400> 97

```
                              Xaa Tyr Gly Ile
                                1
```

<210> 98
<211> 4
<212> PRT
<213> Artificial Sequence

<220>
<223> Description of Artificial Sequence: Synthetic

<220>
<221> SITE
<222> (1)
<223> 2-Thienylalanine

<400> 98

```
                              Xaa Tyr Gly Leu
                                1
```

<210> 99
<211> 4
<212> PRT
<213> Artificial Sequence

<220>
<223> Description of Artificial Sequence: Synthetic

<220>
<221> SITE
<222> (1)
<223> Beta-Alanine

<400> 99

```
                              Xaa Tyr Gly Leu
                                1
```

<210> 100
<211> 4
<212> PRT
<213> Artificial Sequence

<220>
<223> Description of Artificial Sequence: Synthetic

<220>
<221> SITE

<222> (1)
<223> Beta-Alanine

<400> 100

                                    Xaa Tyr Phe Ile
                                         1


<210> 101
<211> 4
<212> PRT
<213> Artificial Sequence

<220>
<223> Description of Artificial Sequence: Synthetic

<220>
<221> SITE
<222> (1)
<223> Beta-Alanine

<400> 101

                                    Xaa Tyr Ala Ile
                                         1


<210> 102
<211> 4
<212> PRT
<213> Artificial Sequence

<220>
<223> Description of Artificial Sequence: Synthetic

<220>
<221> SIMILAR
<222> (1)
<223> 2-Thienylalanine

<400> 102

                                    Xaa Tyr Ala Leu
                                         1


<210> 103
<211> 4
<212> PRT
<213> Artificial Sequence

<220>
<223> Description of Artificial Sequence: Synthetic

<220>
<221> SITE
<222> (1)
<223> Beta-Alanine

<400> 103

```
Xaa Tyr Ala Leu
      1
```

## Claims

1. A compound comprising:

   (1) a therapeutic agent capable of entering a target cell,
   (2) an oligopeptide having a formula $(AA)_n$-$AA^4$-$AA^3$-$AA^2$-$AA^1$, wherein:

   each AA independently represents any genetically encoded amino acid,
   n is an integer from 0 to 12,
   $AA^4$ represents a non-genetically-encoded amino acid,
   $AA^3$ represents any amino acid,
   $AA^2$ represents any amino acid, and
   $AA^1$ represents any amino acid, wherein the oligopeptide is selected from: D-AlaThiβAlaβAlaLeuAlaLeu (SEQ ID NO: 1), ThiβAlaβAlaLeuAlaLeu (SEQ ID NO: 2), β AlaThiβAlaβAlaLeuAlaLeu (SEQ ID NO: 3), β3AlaAlaAlaIle (SEQ ID NO: 4), βAlaAlaAlaLeu (SEQ ID NO: 5), βAlaPheTyrLeu (SEQ ID NO: 6), βAlaPheThrPhe (SEQ ID NO: 7), β AlaPheGlyIle (SEQ ID NO: 8), βAlaPheGlyLeu (SEQ ID NO.: 9), βAlaPhePhePhe (SEQ ID NO: 10), βAlaPhePheIle (SEQ ID NO: 11), βAlaPhePheLeu (SEQ ID NO: 12), β AlaPheAlaIle (SEQ ID NO: 13), βAlaPheAlaLeu (SEQ ID NO: 14), ThiGlyAlaLeu (SEQ ID NO: 15), NalGlyAlaLeu (SEQ ID NO: 16), βAlaLeuTyrLeu (SEQ ID NO: 17), p AlaLeuThiLeu (SEQ ID NO: 18), βAlaLeuThrPhe (SEQ ID NO: 19), βAlaLeuThrIle (SEQ ID NO: 20), βAlaLeuThrLeu (SEQ ID NO: 21), βAlaLeuSerLeu (SEQ ID NO: 22), β AlaLeuPyrLeu (SEQ ID NO: 23), βAlaLeuLeuLeu (SEQ ID NO: 24), βAlaLeuGlyPhe (SEQ ID NO: 25), βAlaLeuGlyIle (SEQ ID NO: 26), ThiLeuGlyLeu (SEQ ID NO: 27), β AlaLeuGlyLeu (SEQ ID NO: 28), AibLeuGlyLeu (SEQ ID NO: 29), βAlaLeuPheIle (SEQ ID NO: 30), βAlaLeuPheLeu (SEQ ID NO: 31), βAlaLeuAibLeu ((SEQ ID NO: 32), β AlaLeuAlaAla (SEQ ID NO: 33), βAlaLeuAlaβAla (SEQ ID NO: 34), βAlaLeuAlaPhe (SEQ ID NO: 35), βAlaLeuAlaGly (SEQ ID NO: 36), βAlaLeuAlaIle (SEQ ID NO: 37), β AlaLeuAlaLeu (SEQ ID NO: 38), TicLeuAlaLeu (SEQ ID NO: 39), ThzLeuAlaLeu (SEQ ID NO: 40), ThiLeuAlaLeu (SEQ ID NO: 41), NalLeuAlaLeu (SEQ ID NO: 42), NAALeuAlaLeu (SEQ ID NO: 43), D-LeuLeuAlaLeu (SEQ ID NO: 44), D-AlaLeuAlaLeu (SEQ ID NO: 45), D-MetLeuAlaLeu (SEQ ID NO: 46), APPLeuAlaLeu (SEQ ID NO: 47), AmbLeuAlaLeu (SEQ ID NO: 48), βAlaLeuAlaNal (SEQ ID NO: 49), βAlaLeuAla (Ser (SEQ ID NO: 50), βAlaLeuAlaTyr (SEQ ID NO: 51), βAlaMetTyrPhe (SEQ ID NO: 52), β AlaMetTyrLeu (SEQ ID NO: 53), pAlaMetGlyIle (SEQ ID NO: 54), ThiMetGlyLeu (SEQ ID NO: 55), βAlaMetPhePhe (SEQ ID NO: 56), βAlaMetPheIle (SEQ ID NO: 57), TicMetAlaLeu (SEQ ID NO: 58), NalMetAlaLeu (SEQ ID NO: 59), NAAMetAlaLeu (SEQ ID NO: 60), βAlaMetAlaLeu (SEQ ID NO: 61), APPMetAlaLeu (SEQ ID NO: 62), β AlaNleTyrIle (SEQ ID NO: 63), βAlaNleTyrLeu (SEQ ID NO: 64), βAlaNleThrIle (SEQ ID NO: 65), βAlaNleThrLeu (SEQ ID NO: 66), βAlaNleGlyPhe (SEQ ID NO: 67), p AlaNleGlyIle (SEQ ID NO: 68), βAlaNleGlyLeu (SEQ ID NO: 69), βaNlePheIle (SEQ ID NO: 70), βAlaNleAlaIle (SEQ ID NO: 71), βAlaNleAlaLeu (SEQ ID NO: 72), β AlaNleAlaPhe (SEQ ID NO: 73), βAlaNvaAlaLeu (SEQ ID NO: 74), βAlaPheTyrIle (SEQ ID NO: 75), ThiProGlyLeu (SEQ ID NO: 76), ThiProAlaLeu (SEQ ID NO: 77), NalProAlaLeu (SEQ ID NO: 78), βAlaProAlaLeu (SEQ ID NO: 79), βAlaPhe(Cl,AlaLeu (SEQ ID NO: 80), βAlaPho(NO$_2$),AlaIle (SEQ ID NO: 81), βAlaPhe(NO$_2$),AlaLeu (SEQ ID NO: 82), βAlaPhgAlaLeu (SEQ ID NO: 83), βAlaPyrAlaLeu (SEQ ID NO: 84), TicThrGlyLeu (SEQ ID NO: 85), βAlaThiGlyIle (SEQ ID NO: 86), βAlaThiAlaLeu (SEQ ID NO: 87), βAlaTicAlaIle (SEQ ID NO: 88), βAlaTicAlaLeu (SEQ ID NO: 89), p AlaValAlaLeu (SEQ ID NO: 90), βAlaTrpAlaLeu (SEQ ID NO: 91), βAlaTyrTyrPhe (SEQ ID NO: 92), βAlaTyrTyrIle (SEQ ID NO: 93), βAlaTyrTyrLeu (SEQ ID NO: 94), β AlaTyrThrLeu (SEQ ID NO: 95), βAlaTyrPheLeu (SEQ ID NO: 96), βAlaTyrGlyIle (SEQ ID NO: 97), ThiTyrGlyLeu (SEQ ID NO: 98), βAlaTyrGlyLeu (SEQ ID NO: 99), β AlaTyrPhelle (SEQ ID NO: 100), βAlaTyrAlalle (SEQ ID NO: 101), ThiTyrAlaLeu (SEQ ID NO: 102), and βAlaTyrAlaLeu (SEQ ID NO: 103)

(3) optionally, a linker group, and

(4) a negatively-charged stabilizing group that hinders cleavage of said oligopeptide by enzymes present in whole blood,

wherein the oligopeptide is directly linked to the stabilizing group at the amino terminus of the oligopeptide and $AA^1$ of the oligopeptide is directly linked to the therapeutic agent or indirectly linked through the linker group to the therapeutic agent at a second attachment site of the oligopeptide, and wherein the compound is selectively cleaved by an enzyme associated with the target cell, wherein the stabilizing group is selected from: Succinic acid, Diglycolic acid, Maleic acid, Pyroglutamic acid, and Glutaric acid.

2. The compound of claim 1 wherein n is an integer from 0 to 8.

3. The compound of claim 1 wherein the target cell is a tumor or inflammatory cell.

4. The compound of claim 1 wherein $AA^1$ of the oligopeptide is selected from Phenylalanine, Isoleucine, Alanine, Glycine, Tyrosine, 2-Naphthylalanine, Serine, and β -Alanine.

5. The compound of claim 1 wherein $AA^2$ of the oligopeptide is selected from Leucine, Tyrosine, Alanine, Glycine, Serine, 3-Pyridylalanine, 2-Thienylalanine, Aminoisobutyric Acid, Threonine, and Phenylalanine.

6. The compound of claim 1 wherein $AA^3$ of the oligopeptide is selected from Leucine, Tyrosine, Phenylalanine, *p*-Cl-Phenylalanine, p-Nitrophenylalanine, Valine, Norleucine, Norvaline, Phenylglycine, Tryptophan, Tetrahydroisoquinoline-3-carboxylic acid, 3-Pyridylalanine, Alanine, Glycine, Thienylalanine, Methionine, Valine, and Proline.

7. The compound of claim 1 wherein $AA^4$ is selected from β-Alanine, Thiazolidine-4-carboxylic acid, 2-Thienylalanine, 2-Naphthylalanine, D-Alanine, D-Leucine, D-Methionine, D-Phenylalanine, 3-Amino-3-phenylpropionic acid, , γ-Aminobutyric acid, , 3-amino-4,4-diphenylbutyric acid, Tetrahydroisoquinoline-3-carboxylic acid, 4-Aminomethylbenzoic acid, and Aminoisobutyric acid.

8. The compound of claim 1 wherein the stabilizing group reduces interaction between the compound and endothelial cells that line blood vessels when administered to the patient.

9. The compound of claim 1 wherein the therapeutic agent is selected from Alkylating Agents, Antiproliferative agents, Tubulin Binding agents, Vinca Alkaloids, Enediynes, Podophyllotoxins or Podophyllotoxin derivatives, the Pteridine family of drugs, Taxanes, Anthracyclines, Dolastatins, Topoiosomerase inhibitors, and cis-Platinums.

10. The compound of claim 1 wherein the therapeutic agent is selected from Doxorubicin, Daunorubicin, Vinblastine, Vincristine, Calicheamicin, Etoposide, Etoposide phosphate, CC-1065, Duocarmycin, KW-2189, Methotrexate, Methopterin, Aminopterin, Dichloromethotrexate, Docetaxel, Paclitaxel, Combretastatin, Combretastatin $A_4$ Phos-phate, Dotastatin 10, Dolastatin 11, Dolastatin 15, Topotecan, Camptothecan, Mitomycin C, Porfiromycin, 5-Fluorouracil, 6-Mercaptopurine, Fludarabine, Tamoxifen, Cytosine arabinoside, Adenosine arabinoside, Colchicine, Carboplatin, Mitomycin C, Bleomycin, Melphalan, and derivatives and analogs thereof.

11. The compound of claim 1 wherein the therapeutic agent has an intracellular active site.

12. The compound of claim 1 wherein $AA^1$ of the oligopeptide is directly linked to the therapeutic agent.

13. The compound of claim 1 wherein $AA^1$ of the oligopeptide sequence is indirectly linked to the therapeutic agent at the second attachment site of the oligopeptide via a linker group, the linker group selected from amino caproic acid, hydrazide group, an ester group, an ether group, and a sulphydryl group.

14. A pharmaceutical composition comprising

(1) a compound according to any preceding claim, and
(2) a pharmaceutically acceptable carrier.

**15.** An *in vitro* method for decreasing toxicity of a therapeutic agent wherein the therapeutic agent is intended for administration to a patient, the method comprising:

covalently forming a prodrug by linking an oligopeptide, having a formula $(AA)_n$-$AA^4$-$AA^3$-$AA^2$-$AA^1$, wherein:

each AA independently represents any genetically encoded amino acid,
n is an integer from 0 to 12,
$AA^4$ represents a non-genetically-encoded amino acid,
and each of $AA^1$, $AA^2$, and $AA^3$ represents any amino acid,

wherein the oligopeptide is selected from: D-AlaThiβAlaβAlaLeuAlaLeu (SEQ ID NO: 1), ThiβAlaβAlaLeuAlaLeu (SEQ ID NO: 2), βAlapAtaLeuAlaLeu (SEQ ID NO: 3), p AlaAlaAlalle (SEQ ID NO: 4), βAlaAlaAlaLeu (SEQ ID NO: 5), βAlaPheTyrLeu (SEQ ID NO: 6), βAlaPheThrPhe (SEQ ID NO: 7), βAlaPheGlylle (SEQ ID NO: 8), β AlaPheGlyLeu (SEQ ID NO: 9), βAlaPhePhePhe (SEQ ID NO: 10), βAlaPhePhelle (SEQ ID NO: 11), βAlaPhePheLeu (SEQ ID NO: 12), βAlaPheAlalle (SEQ ID NO: 13), β AlaPheAlaLeu (SEQ ID NO: 14), ThiGlyAlaLeu (SEQ ID NO: 15), NalGlyAlaLeu (SEQ ID NO: 16), βAlaLeuTyrLeu (SEQ ID NO: 17), βAlaLeuThiLeu (SEQ ID NO: 18), β AlaLeuThrPhe (SEQ ID NO: 19), βAlaLeuThrlle (SEQ ID NO: 20), βAlaLeuThrLeu (SEQ ID NO: 21), βAlaLeuSerLeu (SEQ ID NO: 22), βAlaLeuPyrLeu (SEQ ID NO: 23), βAlaLeuLeuLeu (SEQ ID NO: 24), βAlaLeuGlyPhe (SEQ ID NO: 25), βAlaLeuGlylle (SEQ ID NO: 26), ThiLeuGlyLeu (SEQ ID NO: 27), βAlaLeuGlyLeu (SEQ ID NO: 28), AibLeuGlyLeu (SEQ ID NO: 29), βAlaLeuPhelle (SEQ ID NO: 30), βAlaLeuPheLeu (SEQ ID NO: 31), βAlaLeuAibLeu (SEQ ID NO: 32), βAlaLeuAlaAla (SEQ ID NO: 33), β AlaLeuAlaβla (SEQ ID NO: 34), βAlaLeuAlaPhe (SEQ ID NO: 35), βAlaLeuAlaGly (SEQ ID NO: 36), βAlaLeuAlalle (SEQ ID NO: 37), βAlaLeuAlaLeu (SEQ ID NO: 38), TicLeuAlaLeu (SEQ ID NO: 39), ThzLeuAlaLeu (SEQ ID NO: 40), ThiLeuAlaLeu (SEQ ID NO: 41), NalLeuAlaLeu (SEQ ID NO: 42), NAALeuAlaLeu (SEQ ID NO: 43), D-LeuLeuAlaLeu (SEQ ID NO: 44), D-AlaLeuAlaLeu (SEQ ID NO: 45), D-MetLeuAlaLeu (SEQ ID NO: 46), APPLeuAlaLeu (SEQ ID NO: 47), AmbLeuAlaLeu (SEQ ID NO: 48), βAlaLeuAlaNal (SEQ ID NO: 49), βAlaLeuAla (Ser (SEQ ID NO: 50), βAlaLeuAlaTyr (SEQ ID NO: 51), βAlaMetTyrPhe (SEQ ID NO: 52), βAlaMetTyrLeu (SEQ ID NO: 53), βAlaMetGlylle (SEQ ID NO: 54), ThiMetGlyLeu (SEQ ID NO: 55), βAlaMetPhePhe (SEQ ID NO: 56), βAlaMetPhelle (SEQ ID NO: 57), TicMetAlaLeu (SEQ ID NO: 58), NalMetAlaLeu (SEQ ID NO: 59), NAAMetAlaLeu (SEQ ID NO: 60), βAlaMetAlaLeu (SEQ ID NO: 61), APPMetAlaLeu (SEQ ID NO: 62), βAlaNleTyrlle (SEQ ID NO: 63), β AlaNleTyrLeu (SEQ ID NO: 64), βAlaNleThrlle (SEQ ID NO: 65), βAlaNleThrLeu (SEQ ID NO: 66), βAlaNleGlyPhe (SEQ ID NO: 67), βAlaNleGlylle (SEQ ID NO: 68), β AlaNleGlyLeu (SEQ ID NO: 69), βAlaNlePhelle (SEQ ID NO: 70), βAlaNleAlalle (SEQ ID NO: 71), βAlaNleAlaLeu (SEQ ID NO: 72), βAlaNleAlaPhe (SEQ ID NO: 73), β AlaNvaAlaLeu (SEQ ID NO: 74), βAlaPheTyrlle (SEQ ID NO: 75), ThiProGlyLeu (SEQ ID NO: 76), ThiProAlaLeu (SEQ ID NO: 77), NalProAlaLeu (SEQ ID NO: 78), β AlaProAlaLeu (SEQ ID NO: 79), βAlaPhe(Cl),AlaLeu (SEQ ID NO: 80), β AlaPhe(NO$_2$),Alalle (SEQ ID NO: 81), βAlaPhe(NO$_2$),AlaLeu (SEQ ID NO: 82), β AlaPhgAlaLeu (SEQ ID NO: 83), βAlaPyrAlaLeu (SEQ ID NO: 84), TicThrGlyLeu (SEQ ID NO: 85), βAlaThiGlylle (SEQ ID NO: 86), βAlaThiAlaLeu (SEQ ID NO: 87), β AlaTicAlalle (SEQ ID NO: 88), βAlaTicAlaLeu (SEQ ID NO: 89), βAlaValAlaLeu (SEQ ID NO: 90), βAlaTrpAlaLeu (SEQ ID NO: 91), βAlaTyrTyrPhe (SEQ ID NO: 92), β AlaTyrTyrlle (SEQ ID NO: 93), β3AlaTyrTyrLeu (SEQ ID NO: 94), βAlaTyrThrLeu (SEQ ID NO: 95), βAlaTyrPheLeu (SEQ ID NO: 96), βAlaTyrGlylle (SEQ ID NO: 97), ThiTyrGlyLeu (SEQ ID NO: 98), βAlaTyrGlyLeu (SEQ ID NO: 99), βAlaTyrPhelle (SEQ ID NO: 100), βAlaTyrAlalle (SEQ ID NO: 101), ThiTyrAlaLeu (SEQ ID NO: 102), and β AlaTyrAlaLeu (SEQ ID NO: 103),
to a negatively-charged stabilizing group at a first attachment site of the oligopeptide and directly or indirectly linking the therapeutic agent at a second attachment site of the oligopeptide,
wherein the stabilizing group is selected from: Succinic acid, Diglycolic acid, Maleic acid, Pyroglutamic acid, and Glutaric acid,
whereby the prodrug provides for decreased toxicity of the therapeutic agent when administered to the patient.

**16.** The method of claim 15 wherein the prodrug allows for administration of an increased dosage of the therapeutic agent to the patient relative to the dosage of the therapeutic agent without a prodrug linkage,

**17.** A prodrug formed by the method of 15.

**Patentansprüche**

**1.** Verbindung, umfassend:

(1) einen therapeutischen Wirkstoff, der in der Lage ist, in eine Zielzelle einzudringen,

(2) ein Oligopeptid mit einer Formel $(AA)_n\text{-}AA^4\text{-}AA^3\text{-}AA^2\text{-}AA^1$, wobei:

jedes AA unabhängig voneinander eine beliebige genetisch codierte Aminosäure repräsentiert,

n eine Ganzzahl von 0 bis 12 ist,

$AA^4$ eine nicht genetisch codierte Aminosäure repräsentiert,

$AA^3$ eine beliebige Aminosäure repräsentiert,

$AA^2$ eine beliebige Aminosäure repräsentiert, und

$AA^1$ eine beliebige Aminosäure repräsentiert, wobei die Auswahl des Oligopeptids erfolgt aus: D-AlaThiβAlaβAlaLeuAlaLeu (SEQ-ID-NR: 1), ThiβAlaβAlaLeuAlaLeu (SEQ-ID-NR: 2), βAlaβAlaLeuAlaLeu (SEQ-ID-NR: 3), βAlaAlaAlaIle (SEQ-ID-NR: 4), βAlaAlaAlaLeu (SEQ-ID-NR: 5), βAlaPheTyLeu (SEQ-ID-NR: 6), βAlaPheThrPhe (SEQ-ID-NR: 7), βAlaPheGlyIle (SEQ-ID-NR: 8), βAlaPheGlyLeu (SEQ-ID-NR: 9), βAla-PhePhePhe (SEQ-ID-NR: 10), βAlaPhePheIle (SEQ-ID-NR: 11), βAlaPhePheLeu (SEQ-ID-NR: 12), βAla-PheAlaIle (SEQ-ID-NR: 13), βAlaPheAlaLeu (SEQ-ID-NR: 14), ThiGlyAlaLeu (SEQ-ID-NR: 15), NalGlyAlaLeu (SEQ-ID-NR: 16), βAlaLculyrLeu (SEQ-ID-NR: 17), βAlaLeuThiLeu (SEQ-ID-NR: 18), βAlaLeuThr-Phe (SEQ-ID-NR: 19), βAlaLeuThrIle (SEQ-ID-NR: 20), βAlaLeuThiLeu (SEQ-ID-NR: 21), βAlaLeuSerLeu (SEQ-ID-NR: 22), βAlaLenPyrLeu (SEQ-ID-NR: 23), βAlaLeuLeuLeu (SEQ-ID-NR: 24), βAlaLeuGlyPhe (SEQ-ID-NR: 25), βAlaLeuGlyIle (SEQ-ID-NR: 26), ThiLeuGlyLeu (SEQ-ID-NR: 27), βAlaLeuGlyLeu (SEQ-ID-NR: 28), AibLeuGlyLeu (SEQ-ID-NR: 29), βAlaLeuPheIle (SEQ-ID-NR: 30), βAlaLeuPheLeu (SEQ-ID-NR: 31), βAlaLeuAibLeu (SEQ-ID-NR: 32), βAlaLeuAlaAla (SEQ-ID-NR: 33), βAlaLeuAlaβAla (SEQ-ID-NR: 34), βAlaLeuAlaPhe (SEQ-ID-NR: 35), βAlaLeuAlaGly (SEQ-ID-NR: 36), βAlaLeuAlaIle (SEQ-ID-NR: 37), βAlaLeuAlaLeu (SEQ-ID-NR: 38), TicLeuAlaLeu (SEQ-ID-NR: 39), ThzLeuAlaLeu (SEQ-ID-NR: 40), ThiLeuAlaLeu (SEQ-ID-NR: 41), NalLeuAlaLeu (SEQ-ID-NR: 42), NAALeuAlaLeu (SEQ-ID-NR: 43), D-LeuLeuAlaLeu (SEQ-ID-NR: 44), D-AlaLeuAlaLeu (SEQ-ID-NR: 45), D-MetLeuAlaLeu (SEQ-ID-NR: 46), APPLeuAlaLeu (SEQ-ID-NR: 47), AmbLeuAlaLeu (SEQ-ID-NR: 48), βAlaLeuAlaNal (SEQ-ID-NR: 49), βAlaLeuAla (Ser (SEQ-ID-NR: 50), βAlaLeuAlaTyr (SEQ-ID-NR: 51), βAlaMetTyrPhe (SEQ-ID-NR: 52), βAlaMetTyrLeu (SEQ-ID-NR: 53), βAlaMetGlyIle (SEQ-ID-NR: 54), ThiMetGlyLeu (SEQ-ID-NR: 55), βAlaMet-PhePhe (SEQ-ID-NR: 56), βAlaMetPheIle (SEQ-ID-NR: 57), TicMetAlaLeu (SEQ-ID-NR: 58), NalMetAlaLeu (SEQ-ID-NR: 69), NAAMetAlaLeu (SEQ-ID-NR: 60), βAlaMetAlaLeu (SEQ-ID-NR: 61), APPMetAlaLeu (SEQ-ID-NR: 62), βAlaNleTyrIle (SEQ-ID-NR: 63), βAlaNleTyrLeu (SEQ-ID-NR: 64), βAlaNleThrIle (SEQ-ID-NR: 65), βAlaNleThrLeu (SEQ-ID-NR: 66), βAlaNleGlyPhe (SEQ-ID-NR: 67), βAlaNleGlyIle (SEQ-ID-NR: 68), βAlaNleGlyLeu (SEQ-ID-NR: 69), βAlaNlePheIle (SEQ-ID-NR: 70), βAlaNleAlaIle (SEQ-ID-NR: 71), βAlaNleAlaLeu (SEQ-ID-NR: 72), βAlaNleAlaPhe (SEQ-ID-NR: 73), βAlaNvaAlaLeu (SEQ-ID-NR: 74), βAlaPheIle (SEQ-ID-NR: 75), ThiProGlyLeu (SEQ-ID-NR: 76), ThiProAlaLeu (SEQ-ID-NR: 77), NalProAlaLeu (SEQ-ID-NR: 78), βAlaProAlaLeu (SEQ-ID-NR: 79), βAlaPhe(Cl),AlaLeu (SEQ-ID-NR: 80), βAlaPhe(NO$_2$),AlaIle (SEQ-ID-NR: 81), βAlaPhe(NO$_2$),AlaLeu (SEQ-ID-NR: 82), βAlaPhgAlaLeu (SEQ-ID-NR: 83), βAlaPyAlaLeu (SEQ-ID-NR: 84), TioThrGlyLeu (SEQ-ID-NR: 85), βAlaThiGlyIle (SEQ-ID-NR: 86), βAla-ThiAlaLeu (SEQ-ID-NR: 87), βAlaTieAlaIle (SEQ-ID-NR: 88), βAlaTicAlaLeu (SEQ-ID-NR: 89), βAlaValAlaLeu (SEQ-ID-NR: 90), βAlaTrpAlaLeu (SEQ-ID-NR: 91), βAlaTyrTyrPhe (SEQ-ID-NR: 92), βAlaTyrTyrIle (SEQ-ID-NR: 93), βAlaTyrTyrLeu (SEQ-ID-NR: 94), βAlaTyrThrLeu (SEQ-ID-NR: 95), βAlaTyrPheLeu (SEQ-ID-NR: 96), βAlaTyrGlyIle (SEQ-ID-NR: 97), ThiTyrGlyLeu (SEQ-ID-NR: 98), βAlaTyrGlyLeu (SEQ-ID-NR: 99), βAlaTyrPheIle (SEQ-ID-NR: 100), βAlaTyrAlaIle (SEQ-ID-NR: 101), ThiTyrAlaLeu (SEQ-ID-NR: 102) und βAlaTyrAlaLeu (SEQ-ID-NR: 103)

(3) optional, eine Linkergruppe, und

(4) eine negativ geladene Stabilisierungsgruppe, welche die Spaltung des Oligopeptids durch in Vollblut vorhandene Enzyme hemmt,

wobei das Oligopeptid am Amino-Terminus des Oligopeptids direkt mit der Stabilisierungsgruppe gekoppelt ist und $AA^1$ des Oligopeptids an einer zweiten Anheftungsstelle des Oligopeptids direkt mit dem therapeutischen Wirkstoff gekoppelt ist oder indirekt über die Linkergruppe mit dem therapeutischen Wirkstoff gekoppelt ist, und wobei die Verbindung selektiv durch ein Enzym gespalten wird, welches mit der Zielzelle assoziiert ist, wobei die Auswahl der Stabilisierungsgruppe erfolgt aus: Bernsteinsäure, Diglycolsäure, Maleinsäure, Pyroglutaminsäure und Glutarsäure.

**2.** Verbindung gemäß Anspruch 1, wobei n eine Ganzzahl von 0 bis 8 ist

**3.** Verbindung gemäß Anspruch 1, wobei die Zielzelle eine Tumor- oder Entzündungszelle ist

4. Verbindung gemäß Anspruch 1, wobei die Auswahl des AA$^1$ des Oligopeptids erfolgt aus: Phenylalanin, Isoleucin, Alanin, Glycin, Tyrosin, 2-Naphthylalanin, Serin und β-Alanin.

5. Verbindung gemäß Anspruch 1, wobei die Auswahl des AA$^2$ des Oligopeptids erfolgt aus: Leucin, Tyrosin, Alanin, Glycin, Serin, 3-Pyridylalanin, 2-Thienylalanin, Aminoisobuttersäure, Threonin und Phenylalanin.

6. Verbindung gemäß Anspruch 1, wobei die Auswahl des AA$^3$ des Oligopeptids erfolgt aus: Leucin, Tyrosin, Phenylalanin, p-Cl-Phenylalanin, p-Nitrophenylalanin, Valin, Norleucin, Norvalin, Phenylglycin, Tryptophan, Tetrahydroisochinolin-3-carbonsäure, 3-Pyridylalanin, Alanin, Glycin, Thienylalanin, Methionin, Valin und Prolin.

7. Verbindung gemäß Anspruch 1, wobei die Auswahl des AA$^4$ erfolgt aus: β-Alanin, Thiazolidin-4-carbonsäure, 2-Thienylalanin, 2-Naphthylalanin, D-Alanin, D-Leucin, D-Methionin, D-Phenylalanin, 3-Amino-3-phenylpropionsäure, γ-Aminobuttersäure, 3-Amino-4,4-diphenylbuttersäure, Tetrahydroisochinolin-3-carbonsäure, 4-Aminomethylbenzoesäure und Aminoisobuttersäme.

8. Verbindung gemäß Anspruch 1, wobei die Stabilisierungsgruppe bei Verabreichung an den Patienten die Interaktion zwischen der Verbindung und Enclothelzellen reduziert, welche die Blutgefäße auskleiden.

9. Verbindung gemäß Anspruch 1, wobei die Auswahl des therapeutischen Wirkstoffs erfolgt aus: Alkylierungsmitteln, antiproliferativen Mitteln, Tubulin bindenden Mitteln, Vincaalkaloiden, Enediynen, Podophyllotoxinen oder Podophyllotoxinderivativen, aus der Pteridin-Arzneimittelfamilie, aus Taxanen, Anthracyclinen, Dolastatinen, Topoiosomerase-Inhibitoren und cis-Platinen

10. Verbindung gemäß Anspruch 1, wobei die Auswahl des therapeutischen Wirkstoffs erfolgt aus: Doxorubicin, Daunorubicin, Vinblastin, Vincristin, Calicheamicin, Etoposid, Etoposidphosphat, CC-1065, Duocaimycin, KW-2189, Methotrexat, Methopterin, Aminopterin, Dichlormethotrexat, Docetaxel, Paclitaxel, Combretastatin, Combretastatin-A$_4$-phosphat, Dolastatin 10, Dolastatin 11, Dolastatin 15, Topotecan, Camptothecan, Mitomycin C, Porfiromycin, 5-Fluorouracil, 6-Mercaptopurin, Fludarabin, Tamoxifen, Cytosinarabinosid, Adenosinarabinosid, Colchicin, Carboplatin, Mitomycin C, Bleomycin, Melphalan sowie aus Derivaten und Analogen von diesen.

11. Verbindung gemäß Anspruch 1, wobei der therapeutische Wirkstoff eine intrazelluläre Wirkstelle hat

12. Verbindung gemäß Anspruch 1, wobei AA$^1$ des Oligopeptids direkt mit dem therapeutischen Wirkstoff gekoppelt ist

13. Verbindung gemäß Anspruch 1, wobei AA$^1$ der Oligopeptidsequenz an der zweiten Anheftungsstelle des Oligopeptids über eine Linkergruppe indirekt mit dem therapeutischen Wirkstoff verknüpft ist, wobei die Auswahl der Linkergruppe erfolgt aus: Aminocapronsäure, Hydrazidgruppe, einer Estergruppe, einer Ethergruppe und einer Sulfhydrylgruppe.

14. Pharmazeutische Zusammensetzung, umfassend:

> (1) eine Verbindung gemäß einem der vorherigen Ansprüche, und
> (2) einen pharmazeutisch akzeptablen Träger.

15. *In vitro*-Verfahren zum Verringern der Toxizität eines therapeutischen Wirkstoffs, wobei der therapeutische Wirkstoff zur Verabreichung an einen Patienten bestimmt ist, das Verfahren umfässend:

> das kovalente Bilden eines Prodrugs durch Verknüpfung eines Oligopeptids mit einer Formel (AA)$_n$-AA$^4$-AA$^3$-AA$^2$-AA$^1$, wobei:

>> jedes AA unabhängig voneinander eine beliebige genetisch codierte Aminosäure repräsentiert,
>> n eine Ganzzahl von 0 bis 12 ist,
>> AA$^4$ eine nicht genetisch codierte Aminosäure repräsentiert,
>> und jedes von AA$^1$, AA$^2$ und AA$^3$ eine beliebige Aminosäure repräsentiert,

> wobei die Auswahl des Oligopeptids erfolgt aus: D-AlaThiβAlaβAlaLeuAlaLeu (SEQ-ID-NR: 1), ThiβAlaβAlaLeuAlaLeu (SEQ-ID-NR: 2), βAlaβAlaLeuAlaLeu (SEQ-ID-NR: 3), βAlaAlaAlaIle (SEQ-ID-NR: 4), βAlaAlaAlaLeu (SEQ-ID-NR: 5), βAlaPheTyrLeu (SEQ-ID-NR: 6), βAlaPheThrPhe (SEQ-ID-NR: 7), βAlaPheGlyIle (SEQ-ID-NR: 8), βAlaPheGlyLeu (SEQ-ID-NR: 9), βAlaPhePhePhe (SEQ-ID-NR: 10), βAlaPhePheIle (SEQ-ID-NR: 11), βAlaPhePheLeu (SEQ-ID-NR: 12), βAlaPheAlaIle (SEQ-ID-NR: 13), βAlaPheAlaLeu (SEQ-ID-NR: 14), ThiGly-AlaLeu (SEQ-ID-NR: 15), NalGlyAlaLeu (SEQ-ID-NR: 16), βAlaLeuTyrLeu (SEQ-ID-NR: 17), βAlaLeuThiLeu (SEQ-ID-NR: 18), AlaLeuThrPhe (SEQ-ID-NR: 19), βAlaLeuThrIle (SEQ-ID-NR: 20), βAlaLeuThrLeu (SEQ-ID-NR: 21), βAIaLeuSerLeu (SEQ-ID-NR: 22), βAlaLeuPyrLeu (SEQ-ID-NR: 23), βAlaLeuLeuLeu (SEQ-ID-NR: 24), βAlaLeuGlyPhe (SEQ-ID-NR: 25), βAlaLeuGlyIle (SEQ-ID-NR: 26), ThiLeuGlyLeu (SEQ-ID-NR: 27), βAlaLeuGlyLeu (SEQ-ID-NR: 28), AibLeuGlyLeu (SEQ-ID-NR: 29), βAlaLeuPheIle (SEQ-ID-NR: 30), βAlaLeu-PheLeu (SEQ-ID-NR: 31), βAlaLeuAibLeu (SEQ-ID-NR: 32), βAlaLeuAlaAla (SEQ-ID-NR: 33), βAlaLeuAlaβAla (SEQ-ID-NR: 34), βAlaLeuAlaPhe (SEQ-ID-NR: 35), βAlaLeuAlaGly (SEQ-ID-NR: 36), βAlaLeuAlaIle (SEQ-ID-NR: 37), βAlaLeuAlaLeu (SEQ-ID-NR: 38), TicLeuAlaLeu (SEQ-ID-NR: 39), ThzLeuAlaLeu (SEQ-ID-NR: 40), ThiLeuAlaLeu (SEQ-ID-NR: 41), NalLeuAlaLeu (SEQ-ID-NR: 42), NAALeuAlaLeu (SEQ-ID-NR: 43), D-LeuLeuAlaLeu (SEQ-ID-NR: 44), D-AlaLeuAlaLeu (SEQ-ID-NR: 45), D-MetLeuAlaLeu (SEQ-ID-NR: 46), APPLeuAlaLeu (SEQ-ID-NR: 47), AmbLeuAlaLeu (SEQ-ID-NR: 48), βAlaLeuAlaNal (SEQ-ID-NR: 49), βAlaLeuAla (Ser (SEQ-ID-NR: 50), βAlaLeuAlaTyr (SEQ-ID-NR: 51), βAlaMetTyrPhe (SEQ-ID-NR: 52), βAlaMetTyrLeu (SEQ-ID-NR: 53), βAlaMetGlyIle (SEQ-ID-NR: 54), ThiMetGlyLeu (SEQ-ID-NR: 55), βAlaMetPhePhe (SEQ-ID-NR: 56), βAlaMetPheIle (SEQ-ID-NR: 57), TicMetAlaLeu (SEQ-ID-NR: 58), NalMetAlaLeu (SEQ-ID-NR: 59), NAAMetAlaLeu (SEQ-ID-NR: 60), βAlaMetAlaLeu (SEQ-ID-NR: 61), APPMetAlaLeu (SEQ-ID-NR: 62), βAlaNleTyrIle (SEQ-ID-NR: 63), βAlaNleTyrLeu (SEQ-ID-NR: 64), βAlaNleThrIle (SEQ-ID-NR: 65), βAlaNleThrLeu (SEQ-ID-NR: 66), βAlaNleGlyPhe (SEQ-ID-NR: 67), βAlaNleGlyIle (SEQ-ID-NR: 68), βAlaNleGlyLeu (SEQ-ID-NR: 69), βAlaNlePheIle (SEQ-ID-NR: 70), βAlaNleAlaIle (SEQ-ID-NR: 71), βAlaNleAlaLeu (SEQ-ID-NR: 72), βAlaNleAlaPhe (SEQ-ID-NR: 73), βAlaNvaAlaLeu (SEQ-ID-NR: 74), βAlaPheIyrIle (SEQ-ID-NR: 75), ThiPro-GlyLeu (SEQ-ID-NR: 76), ThiProAlaLeu (SEQ-ID-NR: 77), NalProAlaLeu (SEQ-ID-NR: 78), βAlaProAlaLeu (SEQ-ID-NR: 79), βAlaPhe(Cl),AlaLeu (SEQ-ID-NR: 80), βAlaPhe(NO$_2$),AlaIle (SEQ-ID-NR: 81), βAlaPhe (NO$_2$),AlaLeu (SEQ-ID-NR: 82), βAlaPhgAlaLeu (SEQ-ID-NR: 83), βAlaPyrAlaLeu (SEQ-ID-NR: 84), TicThr-GlyLeu (SEQ-ID-NR: 85), βAlaThiGlyIle (SEQ-ID-NR: 86), βAlaThiAlaLeu (SEQ-ID-NR: 87), βAlaTicAlaIle (SEQ-ID-NR: 88), βAlaTicAlaLeu (SEQ-ID-NR: 89), βAlaValAlaLeu (SEQ-ID-NR: 90), βAlaTrpAlaLeu (SEQ-ID-NR: 91), βAlaTyrTyrPhe (SEQ-ID-NR: 92), βAlaTyrTyrIle (SEQ-ID-NR: 93), βAlaTyrIyrLeu (SEQ-ID-NR: 94), βyAlaTyrThrLeu (SEQ-ID-NR: 95), βAlaTyrPheLeu (SEQ-ID-NR: 96), βAlaTyrGlyIle (SEQ-ID-NR: 97), ThiTy1.GlyLeu (SEQ-ID-NR: 98), βAlaTyrGlyLeu (SEQ-ID-NR: 99), βAlaTyPheIle (SEQ-ID-NR: 100), βAlaTyrAlaIle (SEQ-ID-NR: 101), ThiTyrAlaLeu (SEQ-ID-NR: 102) und βAlaTyrAlaLeu (SEQ-ID-NR: 103),

mit einer negativ geladenen Stabilisierungsgruppe an einer ersten Anheftungsstelle des Oligopeptids und das direkte oder indirekte Verknüpfen des therapeutischen Wirkstoffs an einer zweiten Anheftungsstelle des Oligopeptids,

wobei die Auswahl der Stabilisierungsgruppe erfolgt aus: Bernsteinsäure, Diglycolsäure, Maleinsäure, Pyroglutaminsäure und Glutarsäure,

wodurch das Prodrug für die verringerte Toxizität des therapeutischen Wirkstoffs bei Verabreichung an den Patienten sorgt.

**16.** Verfahren gemäß Anspruch 15, wobei das Prodrug die Verabreichung einer erhöhten Dosis des therapeutischen Wirkstoffs an den Patienten im Vergleich zur Dosierung des therapeutischen Wirkstoffs ohne eine Prodrug-Verknüpfung ermöglicht

**17.** Prodrug, das durch das Verfahren gemäß Anspruch 15 gebildet wird.

## Revendications

**1.** Un composé comportant :

(1) un agent thérapeutique capable d'entrer dans une cellule cible,
(2) un oligopeptide ayant une formule $(AA)_n$-$AA^4$-$AA^3$-$AA^2$-$AA^1$, où :

chaque AA représente indépendamment un acide aminé génétiquement codé quelconque,
n est un entier de 0 à 12,
$AA^4$ représente un acide aminé non génétiquement codé,
$AA^3$ représente un acide aminé quelconque,
$AA^2$ représente un acide aminé quelconque, et
$AA^1$ représente un acide aminé quelconque, où l'oligopeptide est sélectionné parmi : D-AlaThiβAlaβAla-

LeuAlaLeu (SEQ ID NO: 1), ThiβAlaβAlaLeuAlaLeu (SEQ ID NO: 2), βAlaβAlaLeuAlaLeu (SEQ ID NO: 3), βAlaAlaAlaIle (SEQ ID NO: 4), βAlaAlaAlaLeu (SEQ ID NO: 5), βAlaPheTyrLeu (SEQ ID NO: 6), βAla-PheThrPhe (SEQ ID NO: 7), βAlaPheGlyIle (SEQ ID NO: 8), βAlaPheGlyLeu (SEQ ID NO: 9), βAlaPhe-PhePhe (SEQ ID NO: 10), βAlaPhePheIle (SEQ ID NO: 11), βAlaPhePheLeu (SEQ ID NO: 12), βAlaPheAlaIle (SEQ ID NO: 13), βAlaPheAlaLeu (SEQ ID NO: 14), ThiGlyAlaLeu (SEQ ID NO: 15), NalGlyAlaLeu (SEQ ID NO: 16), βAlaLeuTyrLeu (SEQ ID NO: 17), βAlaLeuThiLeu (SEQ ID NO: 18), βAlaLeuThrPhe (SEQ ID NO: 19), βAlaLeuThrIle (SEQ ID NO: 20), βAlaLeuThrLeu (SEQ ID NO: 21), βAlaLeuSerLeu (SEQ ID NO: 22), βAlaLeuPyrLeu (SEQ ID NO: 23), βAlaLeuLeuLeu (SEQ ID NO: 24), βAlaLeuGlyPhe (SEQ ID NO: 25), βAlaLeuGlyIle (SEQ ID NO: 26), ThiLeuGlyLeu (SEQ ID NO: 27), βAlaLeuGlyLeu (SEQ ID NO: 28), AibLeuGlyLeu (SEQ ID NO: 29), βAlaLeuPheIle (SEQ ID NO: 30), βAlaLeuPheLeu (SEQ ID NO: 31), βAlaLeuAibLeu (SEQ ID NO: 32), βAlaLeuAlaAla (SEQ ID NO: 33), βAlaLeuAlaβAla (SEQ ID NO: 34), βAlaLeuAlaPhe (SEQ ID NO: 35), βAlaLeuAlaGly (SEQ ID NO: 36), βAlaLeuAlaIle (SEQ ID NO: 37), βAlaLeuAlaLeu (SEQ ID NO: 38), TicLeuAlaLeu (SEQ ID NO: 39), ThzLeuAlaLeu (SEQ ID NO: 40), ThiLeuAlaLeu (SEQ ID NO: 41), NalLeuAlaLeu (SEQ ID NO: 42), NAALeuAlaLeu (SEQ ID NO: 43), D-LeuLeuAlaLeu (SEQ ID NO: 44), D-AlaLeuAlaLeu (SEQ ID NO: 45), D-MetLeuAlaLeu (SEQ ID NO: 46), APPLeuAlaLeu (SEQ ID NO: 47), AmbLeuAlaLeu (SEQ ID NO: 48), βAlaLeuAlaNal (SEQ ID NO: 49), βAlaLeuAla (Ser (SEQ ID NO: 50), βAlaLeuAlaTyr (SEQ ID NO: 51), βAlaMetTyrPhe (SEQ ID NO: 52), βAlaMetTyrLeu (SEQ ID NO: 53), βAlaMetGlyIle (SEQ ID NO: 54), ThiMetGlyLeu (SEQ ID NO: 55), βAlaMetPhePhe (SEQ ID NO: 56), βAlaMetPheIle (SEQ ID NO: 57), TicMetAlaLeu (SEQ ID NO: 58), NalMetAlaLeu (SEQ ID NO: 59), NAAMetAlaLeu (SEQ ID NO: 60), βAlaMetAlaLeu (SEQ ID NO: 61), APPMetAlaLeu (SEQ ID NO: 62), βAlaNleTyrIle (SEQ ID NO: 63), βAlaNleTyrLeu (SEQ ID NO: 64), βAlaNleThrIle (SEQ ID NO: 65), βAlaNleThrLeu (SEQ ID NO; 66), βAlaNleGlyPhe (SEQ ID NO: 67), βAlaNleGlyIle (SEQ ID NO: 68), βAlaNleGlyLeu (SEQ ID NO: 69), βAlaNlePheIle (SEQ ID NO: 70), βAlaNleAlaIle (SEQ ID NO:71), βAlaNleAlaLeu (SEQ ID NO: 72), βAlaNleAlaPhe (SEQ ID NO: 73), βAlaNvaAlaLeu (SEQ ID NO; 74), βAlaPheTyrIle (SEQ ID NO: 75), ThiProGlyLeu (SEQ ID NO: 76), ThiProAlaLeu (SEQ ID NO: 77), NalProAlaLeu (SEQ ID NO: 78), βAlaProAlaLeu (SEQ ID NO: 79), βAlaPhe(Cl),AlaLeu (SEQ ID NO: 80), βAlaPhe(NO$_2$),AlaIle (SEQ ID NO: 81), βAlaPhe(NO$_2$),AlaLeu (SEQ ID NO: 82), βAlaPhgAlaLeu (SEQ ID NO: 83), βAlaPyrAlaLeu (SEQ ID NO: 84), TicThrGlyLeu (SEQ ID NO: 85), βAlaThlGlyIle (SEQ ID NO: 86), βAlaIhiAlaLeu (SEQ ID NO: 87), βAlaTicAlaIle (SEQ ID NO: 88), βAlaTicAlaLeu (SEQ ID NO: 89), βAlaValAlaLeu (SEQ ID NO: 90), βAla-TrpAlaLeu (SEQ ID NO: 91), βAlalyrTyrPhe (SEQ ID NO; 92), βAlaTyrTyrIle (SEQ ID NO: 93), βAlaTyrTyrLeu (SEQ ID NO: 94), βAlaTyrThrLeu (SEQ ID NO: 95), βAlaTyrPheLeu (SEQ ID NO: 96), βAlaTyrGlyIle (SEQ ID NO: 97), ThiTyrGlyLeu (SEQ ID NO: 98), βAlaTyrGlyLeu (SEQ ID NO: 99), βAlaTyrPheIle (SEQ ID NO: 100), βAlaTyrAlaIle (SEQ ID NO: 101), ThiTyrAlaLeu (SEQ ID NO: 102), et βAlaTyrAlaLeu (SEQ ID NO: 103)

(3) de façon optionnelle, un groupe lieur, et
(4) un groupe stabilisant chargé négativement qui empêche une division dudit oligopeptide par les enzymes présentes dans le sang total,

où l'oligopeptide est directement lié au groupe stabilisant au niveau de l'aminé terminal de l'oligopeptide et AA$^1$ de l'oligopeptide est directement lié à l'agent thérapeutique ou indirectement lié par le biais du groupe lieur à l'agent thérapeutique au niveau d'un deuxième site d'attachement à l'oligopeptide, et où le composé est divisé sélectivement par une enzyme associée à la cellule cible, où le groupe stabilisant est sélectionné parmi : acide succinique, acide diglycolique, acide maléique, acide pyroglutamique, et acide glutarique.

2. Le composé de la revendication 1 où n est un entier de 0 à 8.

3. Le composé de la revendication 1 où la cellule cible est une tumeur ou une cellule inflammatoire.

4. Le composé de la revendication 1 où AA$^1$ de l'oligopeptide est sélectionné parmi phénylalanine, isoleucine, alanine, glycine, tyrosine, 2-naphtylalanine, sérine, et β-alanine

5. Le composé de la revendication 1 où AA$^2$ de l'oligopeptide est sélectionné parmi leucine, tyrosine, alanine, glycine, sérine, 3-pyridylalanine, 2-thiénylalanine, acide amino-isobutyrique, thréonine, et phénylalanine.

6. Le composé de la revendication 1 où AA$^3$ de l'oligopeptide est sélectionné parmi leucine, tyrosine, phénylalanine, p-Cl-phénylalanine, p-nitrophénylalanine, valine, norleucine, norvaline, phénylglycine, tryptophane, acide tétrahydro-isoquinoléine-3-carboxylique, 3-pyridylalanine, alanine, glycine, thiénylalanine, méthionine, valine, et proline.

**7.** Le composé de la revendication 1 où AA$^4$ est sélectionné parmi β-alanine, acide thiazolidine-4-caiboxylique, 2-thiénylalanine, 2-naphtylalanine, D-alanine, D-leucine, D-méthionine, D-phénylalanine, acide 3-amino-3-phénylpropionique, acide γ-aminobutyrique, acide 3-amino-4,4-diphénylbutyrique, acide tétrahydro-isoquinoléine-3-carboxylique, acide 4-aminométhylbenzoique, and acide amino-isobutyrique

**8.** Le composé de la revendication 1 où le groupe stabilisant réduit l'intéraction entre le composé et les cellules endothéliales qui bordent les vaisseaux sanguins quand il est administré au patient

**9.** Le composé de la revendication 1 où l'agent thérapeutique est sélectionné parmi des agents alcoylants, des agents antiprolifératifs, des agents de liaison de la tubuline, des alcaloïdes de la pervenche, des podophyllotoxines ou dérivés des podophyllotoxines, la famille de médicaments ptéridine, des taxanes, des anthracyclines, des dolastatines, des inhibiteurs de topoisomérase$^{(1)}$, et des cisplatines.

**10.** Le composé de la revendication 1 où l'agent thérapeutique est sélectionné parmi doxorubicin, daunorubicine, vinblastine, vincristine, calicheamicine, étoposide, phosphate d'étoposide, CC-1065, duocarmycine, KW-2189, méthotrexate, méthoptérine, aminoptérine, dichlorométhotrexate, docetaxel, paclitaxel, combretastatine, phosphate A$_4$ de combretastatine, dolastatine 10, dolastatine 11, dolastatine 15, topotécan, camptothecan, mitomycine C, porfiromycine, 5-fluorouracile, 6-mercaptopurine, fludarabine, tamoxifen, cytosine arabinoside, adénosine arabinoside, colchicine, carboplatine, mitomycine C$^{(2)}$, bléomycine, melphalan, et dérivés et analogues de ces derniers

**11.** Le composé de la revendication 1 où l'agent thérapeutique a un site actif intracellulaire.

**12.** Le composé de la revendication 1 où AA$^1$ de l'oligopeptide est directement lié à l'agent thérapeutique

**13.** Le composé de la revendication 1 où AA$^1$ de la séquence oligopeptide est indirectement lié à l'agent thérapeutique au niveau du deuxième site d'attachement de l'oligopeptide par l'intermédiaire d'un groupe lieur, le groupe lieur étant sélectionné parmi acide aminocaproïque, groupe hydrazide, un groupe ester, un groupe éther, et un groupe sulfrydryle

**14.** Une composition pharmaceutique comportant

(1) un composé selon n'importe quelle revendication précédente, et
(2) un véhicule de qualité pharmaceutique.

**15.** Un procédé in vitro pour réduite la toxicité d'un agent thérapeutique où l'agent thérapeutique est destiné à être administré à un patient, le procédé comportant :

former de façon covalente un promédicament en liant un oligopeptide, ayant une formule (AA)$_n$-A-A$^4$-AA$^3$-AA$^2$-AA$^1$, où :

chaque AA représente indépendamment un acide aminé génétiquement codé quelconque,
n est un entier de 0 à 12,
AA$^4$ représente un acide aminé non génétiquement codé,
et chacun de AA$^1$, AA$^2$, et AA$^3$ représente un acide aminé quelconque,
où l'oligopeptide est sélectionné parmi : D-AlaThiβAlaβAlaLeuAlaLeu (SEQ ID NO: 1), ThiβAlaβAlaLeuAlaLeu (SEQ ID NO: 2), βAlaβAlaLeuAlaLeu (SEQ ID NO: 3), βAlaAlaAlaIle (SEQ ID NO: 4), βAlaAlaAlaLeu (SEQ ID NO: 5), βAlaPheTyrLeu (SEQ ID NO: 6), βAlaPheThrPhe (SEQ ID NO: 7), βAlaPheGlyIle (SEQ ID NO: 8), βAlaPheGlyLeu (SEQ ID NO: 9), βAlaPhePhePhe (SEQ ID NO: 10), βAlaPhePheIle (SEQ ID NO: 11), βAlaPhePheLeu (SEQ ID NO: 12), βAlaPheAlaIle (SEQ ID NO: 13), βAlaPheAlaLeu (SEQ ID NO: 14), IhiGlyAlaLeu (SEQ ID NO: 15), NalGlyAlaLeu (SEQ ID NO: 16), βAlaLeuTyrLeu (SEQ ID NO: 17), βAlaLeuThiLeu (SEQ ID NO: 18), βAlaLeuThrPhe (SEQ ID NO: 19), βAlaLeuThrIle (SEQ ID NO: 20), βAlaLeuThrLeu (SEQ ID NO: 21), βAlaLeuSerLeu (SEQ ID NO: 22), βAlaLeuPyrLeu (SEQ ID NO: 23), βAlaLeuLeuLeu (SEQ ID NO: 24), βAlaLeuGlyPhe (SEQ ID NO: 25), βAlaLeuGlyIle (SEQ ID NO: 26), ThiLeuGlyLeu (SEQ ID NO: 27), βAlaLeuGlyLeu (SEQ ID NO: 28), AibLeuGlyLeu (SEQ ID NO: 29), βAlaLeuPheIle (SEQ ID NO: 30), βAlaLeuPheLeu (SEQ ID NO: 31), βAlaLeuAibLeu (SEQ ID NO: 32), βAlaLeuAlaAla (SEQ ID NO: 33), βAlaLeuAlaβAla (SEQ ID NO: 34), βAlaLeuAlaPhe (SEQ ID NO: 35), βAlaLeuAlaGly (SEQ ID NO: 36), βAlaLeuAlaIle (SEQ ID NO: 37), βAlaLeuAlaLeu (SEQ ID NO: 38), TicLeuAlaLeu (SEQ ID NO: 39), ThzLeuAlaLeu (SEQ ID NO: 40), ThiLeuAlaLeu (SEQ ID NO: 41), NalLeuAlaLeu (SEQ ID NO: 42), NAALeuAlaLeu (SEQ ID NO: 43), D-LeuLeuAlaLeu (SEQ ID NO: 44), D-AlaLeuAlaLeu (SEQ ID NO: 45), D-MetLeuAlaLeu (SEQ ID NO: 46), APPLeuAlaLeu (SEQ ID NO: 47), AmbLeuAlaLeu (SEQ ID NO: 48), βAlaLeuAlaNal (SEQ ID NO: 49), βAlaLeuAla (Ser (SEQ ID NO: 50), βAlaLeuAlaTyr (SEQ ID NO: 51), βAlaMetTyrPhe (SEQ ID NO: 52), βAlaMetTyrLeu (SEQ ID NO: 53), βAlaMetGlyIle (SEQ ID NO: 54), ThiMetGlyLeu (SEQ ID NO: 55), βAlaMetPhePhe (SEQ ID NO: 56), βAlaMetPheIle (SEQ ID NO: 57), TicMetAlaLeu (SEQ ID NO: 58), NalMetAlaLeu (SEQ ID NO: 59), NAAMetAlaLeu (SEQ ID NO: 60), βAlaMetAlaLeu (SEQ ID NO: 61), APPMetAlaLeu (SEQ ID NO: 62), βAlaNleTyrIle (SEQ ID NO: 63), βAlaNleTyrLeu (SEQ ID NO: 64), βAlaNleThrIle (SEQ ID NO: 65), βAlaNleThrLeu (SEQ ID NO; 66), βAlaNleGlyPhe (SEQ ID NO: 67), βAlaNleGlyIle (SEQ ID NO: 68), βAlaNleGlyLeu (SEQ ID NO: 69), βAlaNlePheIle (SEQ ID NO: 70), βAlaNleAlaIle (SEQ ID NO: 71), βAlaNleAlaLeu (SEQ ID NO: 72), βAlaNleAlaPhe (SEQ ID NO: 73), βAlaNvaAlaLeu (SEQ ID NO; 74), βAlaPheTyrIle (SEQ ID NO: 75), ThiProGlyLeu (SEQ ID NO: 76), ThiProAlaLeu (SEQ ID NO: 77), NalProAlaLeu (SEQ ID NO: 78), βAlaProAlaLeu (SEQ ID NO: 79), βAlaPhe(Cl),AlaLeu (SEQ ID NO: 80), βAlaPhe(NO$_2$),AlaIle (SEQ ID NO: 81), βAlaPhe(NO$_2$),AlaLeu (SEQ ID NO: 82), βAlaPhgAlaLeu (SEQ ID NO: 83), βAlaPyrAlaLeu (SEQ ID NO: 84), TicThrGlyLeu (SEQ ID NO: 85), βAlaThlGlyIle (SEQ ID NO: 86), βAlaThiAlaLeu (SEQ ID NO: 87), βAlaTicAlaIle (SEQ ID NO: 88), βAlaTicAlaLeu (SEQ ID NO: 89), βAlaValAlaLeu (SEQ ID NO: 90), βAlaTrpAlaLeu (SEQ ID NO: 91), βAlaTyrTyrPhe (SEQ ID NO; 92), βAlaTyrTyrIle (SEQ ID NO: 93), βAlaTyrTyrLeu (SEQ ID NO: 94), βAla-TyrThrLeu (SEQ ID NO: 95), βAlaTyrPheLeu (SEQ ID NO: 96), βAlaTyrGlyIle (SEQ ID NO: 97), ThiTyr-GlyLeu (SEQ ID NO: 98), βAlaTyrGlyLeu (SEQ ID NO: 99), βAlaTyrPheIle (SEQ ID NO: 100), βAlaTyrAlaIle (SEQ ID NO: 101), ThiTyrAlaLeu (SEQ ID NO: 102), et βAlaTyrAlaLeu (SEQ ID NO: 103).
à un groupe stabilisant chargé négativement au niveau d'un premier site d'attachement de l'oligopeptide et liant directement ou indirectement l'agent thérapeutique au niveau d'un deuxième site d'attachement de l'oligopeptide,
où le groupe stabilisant est sélectionné parmi: acide succinique, acide diglycolique, acide maléique, acide pyroglutamique, et acide glutarique,
grâce à quoi le promédicament assure une toxicité réduite de l'agent thérapeutique quand il est administré au patient.

**16.** Le procédé de la revendication 15 où le médicament permet une administration d'un dosage accru de l'agent thérapeutique au patient par rapport au dosage de l'agent thérapeutique sans une liaison par promédicament.

**17.** Un promédicament formé par le procédé de 15

| Symbol | Name | Structure |
|--------|------|-----------|
| Aca | 6-Aminocaproic Acid | $H_2N$ _____ COOH |
| Aib | Aminoisobutyric Acid | $H_2N$ —COOH, $H_3C$ $CH_3$ |
| Amb | 4-(Aminomethyl)benzoic Acid | $H_2N$ — benzene — COOH |
| APP | 3-Amino-3-phenylpropionic Acid | phenyl, $NH_2$, O, OH |
| Dg | Diglycolic Acid | HO — O — O — OH |

**FIG. 1A**

| Symbol | Name | Structure |
|--------|------|-----------|
| Gl | Glutaric Acid | |
| Mal | Maleic Acid | |
| NAA | 3-Amino-4,4-diphenylbutyric Acid | |
| Nal | 2-Naphthylalanine | |
| Naph | 1,8-Naphthalene dicarboxylic Acid | |

FIG. 1B

| Symbol | Name | Structure |
|--------|------|-----------|
| Phg | Phenylglycine | |
| PEG | Polyethylene Glycol$_{5000}$ Hemisuccinyl Ester | |
| Pyg | Pyroglutamic Acid | |
| Pyr | 3-Pyridylalanine | |
| Suc | Succinic Acid | |

FIG. 1C

| Symbol | Name | Structure |
|---|---|---|
| Thi | 2-Thienylalanine | |
| Thz | 3-Thioproline<br>or<br>Thiazolidine-4-carboxylic Acid | |
| Tic | Tetrahydroisoquinoline-3-carboxylic Acid | |

**FIG. 1D**

FIG. 2

**FIG. 3**

**FIG. 4**

**FIG. 5**

**FIG.6**

Coupling Agent/Solvent/Temperature

R = Trityl or substituted Trityl, or
other suitable amino protecting group

**FIG. 7**

83

**FIG. 8**

84

**FIG. 9**

85

| No: | (AA⁷) P5 | (AA⁶) P4 | (AA⁵) P3 | (AA⁴) P2 | (AA³) P1 | (AA²) P1′ | (AA¹) P2′ | SEQ ID NO: |
|---|---|---|---|---|---|---|---|---|
| 1 | D-Ala | Thi | βAla | βAla | Leu | Ala | Leu | SEQ ID NO: 1 |
| 2 | Ø | Thi | βAla | βAla | Leu | Ala | Leu | SEQ ID NO: 2 |
| 3 | Ø | Ø | βAla | βAla | Leu | Ala | Leu | SEQ ID NO: 3 |
| 4 | Ø | Ø | Ø | βAla | Ala | Ala | Ile | SEQ ID NO: 4 |
| 5 | Ø | Ø | Ø | βAla | Ala | Ala | Leu | SEQ ID NO: 5 |
| 6 | Ø | Ø | Ø | βAla | Phe | Tyr | Leu | SEQ ID NO: 6 |
| 7 | Ø | Ø | Ø | βAla | Phe | Thr | Phe | SEQ ID NO: 7 |
| 8 | Ø | Ø | Ø | βAla | Phe | Gly | Ile | SEQ ID NO: 8 |
| 9 | Ø | Ø | Ø | βAla | Phe | Gly | Leu | SEQ ID NO: 9 |
| 10 | Ø | Ø | Ø | βAla | Phe | Phe | Phe | SEQ ID NO: 10 |
| 11 | Ø | Ø | Ø | βAla | Phe | Phe | Ile | SEQ ID NO: 11 |
| 12 | Ø | Ø | Ø | βAla | Phe | Phe | Leu | SEQ ID NO: 12 |
| 13 | Ø | Ø | Ø | βAla | Phe | Ala | Ile | SEQ ID NO: 13 |
| 14 | Ø | Ø | Ø | βAla | Phe | Ala | Leu | SEQ ID NO: 14 |
| 15 | Ø | Ø | Ø | Thi | Gly | Ala | Leu | SEQ ID NO: 15 |
| 16 | Ø | Ø | Ø | Nal | Gly | Ala | Leu | SEQ ID NO: 16 |
| 17 | Ø | Ø | Ø | βAla | Leu | Tyr | Leu | SEQ ID NO: 17 |
| 18 | Ø | Ø | Ø | βAla | Leu | Thi | Leu | SEQ ID NO: 18 |
| 19 | Ø | Ø | Ø | βAla | Leu | Thr | Phe | SEQ ID NO: 19 |
| 20 | Ø | Ø | Ø | βAla | Leu | Thr | Ile | SEQ ID NO: 20 |
| 21 | Ø | Ø | Ø | βAla | Leu | Thr | Leu | SEQ ID NO: 21 |
| 22 | Ø | Ø | Ø | βAla | Leu | Ser | Leu | SEQ ID NO: 22 |
| 23 | Ø | Ø | Ø | βAla | Leu | Pyr | Leu | SEQ ID NO: 23 |
| 24 | Ø | Ø | Ø | βAla | Leu | Leu | Leu | SEQ ID NO: 24 |
| 25 | Ø | Ø | Ø | βAla | Leu | Gly | Phe | SEQ ID NO: 25 |
| 26 | Ø | Ø | Ø | βAla | Leu | Gly | Ile | SEQ ID NO: 26 |
| 27 | Ø | Ø | Ø | Thi | Leu | Gly | Leu | SEQ ID NO: 27 |
| 28 | Ø | Ø | Ø | βAla | Leu | Gly | Leu | SEQ ID NO: 28 |
| 29 | Ø | Ø | Ø | Aib | Leu | Gly | Leu | SEQ ID NO: 29 |
| 30 | Ø | Ø | Ø | βAla | Leu | Phe | Ile | SEQ ID NO: 30 |
| 31 | Ø | Ø | Ø | βAla | Leu | Phe | Leu | SEQ ID NO: 31 |
| 32 | Ø | Ø | Ø | βAla | Leu | Aib | Leu | SEQ ID NO: 32 |
| 33 | Ø | Ø | Ø | βAla | Leu | Ala | Ala | SEQ ID NO: 33 |
| 34 | Ø | Ø | Ø | βAla | Leu | Ala | βAla | SEQ ID NO: 34 |
| 35 | Ø | Ø | Ø | βAla | Leu | Ala | Phe | SEQ ID NO: 35 |
| 36 | Ø | Ø | Ø | βAla | Leu | Ala | Gly | SEQ ID NO: 36 |
| 37 | Ø | Ø | Ø | βAla | Leu | Ala | Ile | SEQ ID NO: 37 |
| 38 | Ø | Ø | Ø | βAla | Leu | Ala | Leu | SEQ ID NO: 38 |
| 39 | Ø | Ø | Ø | Tic | Leu | Ala | Leu | SEQ ID NO: 39 |
| 40 | Ø | Ø | Ø | Thz | Leu | Ala | Leu | SEQ ID NO: 40 |

**FIG. 10A**

| No: | (AA⁷) P5 | (AA⁶) P4 | (AA⁵) P3 | (AA⁴) P2 | (AA³) P1 | (AA²) P1' | (AA¹) P2' | SEQ ID NO: |
|-----|----|----|----|------|--------|------|------|------------|
| 41 | Ø | Ø | Ø | Thi | Leu | Ala | Leu | SEQ ID NO: 41 |
| 42 | Ø | Ø | Ø | Nal | Leu | Ala | Leu | SEQ ID NO: 42 |
| 43 | Ø | Ø | Ø | NAA | Leu | Ala | Leu | SEQ ID NO: 43 |
| 44 | Ø | Ø | Ø | D-Leu | Leu | Ala | Leu | SEQ ID NO: 44 |
| 45 | Ø | Ø | Ø | D-Ala | Leu | Ala | Leu | SEQ ID NO: 45 |
| 46 | Ø | Ø | Ø | D-Met | Leu | Ala | Leu | SEQ ID NO: 46 |
| 47 | Ø | Ø | Ø | APP | Leu | Ala | Leu | SEQ ID NO: 47 |
| 48 | Ø | Ø | Ø | Amb | Leu | Ala | Leu | SEQ ID NO: 48 |
| 49 | Ø | Ø | Ø | βAla | Leu | Ala | Nal | SEQ ID NO: 49 |
| 50 | Ø | Ø | Ø | βAla | Leu | Ala | Ser | SEQ ID NO: 50 |
| 51 | Ø | Ø | Ø | βAla | Leu | Ala | Tyr | SEQ ID NO: 51 |
| 52 | Ø | Ø | Ø | βAla | Met | Tyr | Phe | SEQ ID NO: 52 |
| 53 | Ø | Ø | Ø | βAla | Met | Tyr | Leu | SEQ ID NO: 53 |
| 54 | Ø | Ø | Ø | βAla | Met | Gly | Ile | SEQ ID NO: 54 |
| 55 | Ø | Ø | Ø | Thi | Met | Gly | Leu | SEQ ID NO: 55 |
| 56 | Ø | Ø | Ø | βAla | Met | Phe | Phe | SEQ ID NO: 56 |
| 57 | Ø | Ø | Ø | βAla | Met | Phe | Ile | SEQ ID NO: 57 |
| 58 | Ø | Ø | Ø | Tic | Met | Ala | Leu | SEQ ID NO: 58 |
| 59 | Ø | Ø | Ø | Nal | Met | Ala | Leu | SEQ ID NO: 59 |
| 60 | Ø | Ø | Ø | NAA | Met | Ala | Leu | SEQ ID NO: 60 |
| 61 | Ø | Ø | Ø | βAla | Met | Ala | Leu | SEQ ID NO: 61 |
| 62 | Ø | Ø | Ø | APP | Met | Ala | Leu | SEQ ID NO: 62 |
| 63 | Ø | Ø | Ø | βAla | Nle | Tyr | Ile | SEQ ID NO: 63 |
| 64 | Ø | Ø | Ø | βAla | Nle | Tyr | Leu | SEQ ID NO: 64 |
| 65 | Ø | Ø | Ø | βAla | Nle | Thr | Ile | SEQ ID NO: 65 |
| 66 | Ø | Ø | Ø | βAla | Nle | Thr | Leu | SEQ ID NO: 66 |
| 67 | Ø | Ø | Ø | βAla | Nle | Gly | Phe | SEQ ID NO: 67 |
| 68 | Ø | Ø | Ø | βAla | Nle | Gly | Ile | SEQ ID NO: 68 |
| 69 | Ø | Ø | Ø | βAla | Nle | Gly | Leu | SEQ ID NO: 69 |
| 70 | Ø | Ø | Ø | βAla | Nle | Phe | Ile | SEQ ID NO: 70 |
| 71 | Ø | Ø | Ø | βAla | Nle | Ala | Ile | SEQ ID NO: 71 |
| 72 | Ø | Ø | Ø | βAla | Nle | Ala | Leu | SEQ ID NO: 72 |
| 73 | Ø | Ø | Ø | βAla | Nle | Ala | Phe | SEQ ID NO: 73 |
| 74 | Ø | Ø | Ø | βAla | Nva | Ala | Leu | SEQ ID NO: 74 |
| 75 | Ø | Ø | Ø | βAla | Phe | Tyr | Ile | SEQ ID NO: 75 |
| 76 | Ø | Ø | Ø | Thi | Pro | Gly | Leu | SEQ ID NO: 76 |
| 77 | Ø | Ø | Ø | Thi | Pro | Ala | Leu | SEQ ID NO: 77 |
| 78 | Ø | Ø | Ø | Nal | Pro | Ala | Leu | SEQ ID NO: 78 |
| 79 | Ø | Ø | Ø | βAla | Pro | Ala | Leu | SEQ ID NO: 79 |
| 80 | Ø | Ø | Ø | βAla | Phe(Cl) | Ala | Leu | SEQ ID NO: 80 |

**FIG. 10B**

| No: | (AA⁷) P5 | (AA⁶) P4 | (AA⁵) P3 | (AA⁴) P2 | (AA³) P1 | (AA²) P1′ | (AA¹) P2′ | SEQ ID NO: |
|---|---|---|---|---|---|---|---|---|
| 81 | Ø | Ø | Ø | βAla | Phe(NO₂) | Ala | Ile | SEQ ID NO: 81 |
| 82 | Ø | Ø | Ø | βAla | Phe(NO₂) | Ala | Leu | SEQ ID NO: 82 |
| 83 | Ø | Ø | Ø | βAla | Phg | Ala | Leu | SEQ ID NO: 83 |
| 84 | Ø | Ø | Ø | βAla | Pyr | Ala | Leu | SEQ ID NO: 84 |
| 85 | Ø | Ø | Ø | Tic | Thr | Gly | Leu | SEQ ID NO: 85 |
| 86 | Ø | Ø | Ø | βAla | Thi | Gly | Ile | SEQ ID NO: 86 |
| 87 | Ø | Ø | Ø | βAla | Thi | Ala | Leu | SEQ ID NO: 87 |
| 88 | Ø | Ø | Ø | βAla | Tic | Ala | Ile | SEQ ID NO: 88 |
| 89 | Ø | Ø | Ø | βAla | Tic | Ala | Leu | SEQ ID NO: 89 |
| 90 | Ø | Ø | Ø | βAla | Val | Ala | Leu | SEQ ID NO: 90 |
| 91 | Ø | Ø | Ø | βAla | Trp | Ala | Leu | SEQ ID NO: 91 |
| 92 | Ø | Ø | Ø | βAla | Tyr | Tyr | Phe | SEQ ID NO: 92 |
| 93 | Ø | Ø | Ø | βAla | Tyr | Tyr | Ile | SEQ ID NO: 93 |
| 94 | Ø | Ø | Ø | βAla | Tyr | Tyr | Leu | SEQ ID NO: 94 |
| 95 | Ø | Ø | Ø | βAla | Tyr | Thr | Leu | SEQ ID NO: 95 |
| 96 | Ø | Ø | Ø | βAla | Tyr | Phe | Leu | SEQ ID NO: 96 |
| 97 | Ø | Ø | Ø | βAla | Tyr | Gly | Ile | SEQ ID NO: 97 |
| 98 | Ø | Ø | Ø | Thi | Tyr | Gly | Leu | SEQ ID NO: 98 |
| 99 | Ø | Ø | Ø | βAla | Tyr | Gly | Leu | SEQ ID NO: 99 |
| 100 | Ø | Ø | Ø | βAla | Tyr | Phe | Ile | SEQ ID NO: 100 |
| 101 | Ø | Ø | Ø | βAla | Tyr | Ala | Ile | SEQ ID NO: 101 |
| 102 | Ø | Ø | Ø | Thi | Tyr | Ala | Leu | SEQ ID NO: 102 |
| 103 | Ø | Ø | Ø | βAla | Tyr | Ala | Leu | SEQ ID NO: 103 |

**FIG. 10C**

MDS

**FIG. 11**

**FIG. 12**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9605863 A
- US 5962216 A

- WO 60119312 A

**Non-patent literature cited in the description**

- **Bruce A. Chabner ; Jerry M. Collins.** Cancer Chemotherapy. 1990
- **Merrifield.** *J. A. Chem. Soc.,* 1963, vol. 88, 2149
- **Bodanszky ; Bodanszky.** The Practice of Peptide Synthesis. Springer-Verlag, 1994, 7-161
- **Stewart.** Solid Phase Peptide Synthesis. Pierce Chemical, 1984
- **Bodanszky, M.** The Practice of Peptide Synthesis. Springer Verlag, 1984, vol. 185
- **Bodanszky, M.** Principles of Peptide Synthesis. Springer Verlag, 1984, vol. 159
- **Matzanke, B. F. et al.** *Eur. J. Biochem.,* 1992, vol. 207, 747-55
- **Chaires, J. B. et al.** *Biochemistry,* 1982, vol. 21, 3927-32
- **Hayakawa, E. et al.** *Chem. Pharm. Bull.,* 1991, vol. 39, 1282-6
- **Casimir, J. R.** *Tet. Lett.,* 1995, vol. 36 (19), 3409
- **Genet, J-P et al.** *Tet. Lett.,* 1994, vol. 50, 497
- **Bricout, H.** *Tet. Lett.,* 1998, vol. 54, 1073
- **Genet, J-P.** *Synlett,* 1993, vol. 680
- **Waldmann, H.** *Bioorg. Med. Chem.,* 1998, vol. 7, 749
- **Shaphiro, G. ; Buechler, D.** *Tet. Lett.,* 1994, vol. 35, 5421
- **Wang, S. S.** *J. Am. Chem. Soc.,* 1973, vol. 95, 1328

- **Zhang, C. ; Mjaili, A. M. M.** *Tet. Lett.,* 1996, vol. 37, 5457
- **Rink, H.** *Tet, Lett.,* 1987, vol. 28, 3787
- **Chen, C.** *J. Am. Chem. Soc.,* 1994, vol. 116, 2661
- **Bartos, K.** *Peptides, Proc. 22nd European Peptide Symposium,* 1992
- ESCOM, Leiden. 1993, 281
- **Menozzi et al.** Self-association of doxorubicin and related compounds in aqueous solutions. *J. Pharmaceut. Sci.,* 1984, vol. 73 (6), 766-770
- **Confalonieri, C. et al.** The use of new laser particle sizer and shape analyser to detect and evaluate gelatinous microparticles suspended in reconstituted anthracycline infusion solutions. *J. Pharmaceut. Biomed. Anal.,* 1991, vol. 9 (1), 1-8
- **DeLucia III, A. et al.** Efficacy and toxicity of differently charged polycationic protamine-like peptides for heparin anticoagulation reversal. *J. Vasc. Surg.,* 1993, vol. 18, 49-60
- **Ekrami, H. M. ; Shen, W. C.** Carbamylation decreases the cytotoxicity but not the drug-carrier properties of polylysines. *J. Drug Targ.,* 1995, vol. 2, 469-475
- **Wakefield, T. W. et al.** Heparin-mediated reductions of the toxic effects of protamine sulfate on rabbit myocardium. *J. Vasc. Surg.,* 1992, vol. 16, 47-53